# EUROPEAN PATENT SPECIFICATION

(11) **EP 1 765 065 B1**
(45) Date of publication and mention of the grant of the patent: **27.06.2012**
(21) Application number: 05751794.8
(22) Date of filing: 23.05.2005
(51) Int. Cl.: A01K 67/00, G01N 33/50

(54) **METHODS AND COMPOSITIONS RELATED TO DELIVERY OF CHEMICAL COMPOUNDS TO INVERTEBRATE EMBRYOS**
VERFAHREN UND ZUSAMMENSETZUNGEN FÜR DIE ABGABE VON CHEMIKALIEN AN EMBRYONEN VON WIRBELLOSEN
PROCEDES ET COMPOSITIONS RELATIVES A L'ADMINISTRATION DE COMPOSES CHIMIQUES A DES EMBRYONS D'INVERTEBRES

(30) Priority: 21.05.2004 US 573194 P
(43) Date of publication of application: 28.03.2007
(73) Proprietor: University of Utah Research Foundation, Salt Lake City, UT 84108 (US)
(72) Inventor: SCHMID, Aloisia, T., Salt Lake City, UT 84108 (US)
(74) Representative: von Kreisler Selting Werner
(86) International application number: PCT/US2005/017900
(87) International publication number: WO 2005/112978

(56) References cited:
- WO-A1-00/37938
- US-A1- 2002 009 751
- US-A1- 2002 025 297
- MYERS S P ET AL: "CHARACTERIZATION OF INTRACELLULAR ICE FORMATION IN DROSOPHILA-MELANOGASTER EMBRYOS" CRYOBIOLOGY, vol. 26, no. 5, 1989, pages 472-484, XP002578331
- LYNCH D V ET AL: "A TWO-STEP METHOD FOR PERMEABILIZATION OF DROSOPHILA EGGS" CRYOBIOLOGY, vol. 26, no. 5, 1989, pages 445-452, XP002578332
- TRAUT H: "THE SOLVENT DIMETHYLSULFOXIDE DOES NOT INDUCE ANEUPLOIDY IN OOCYTES OF DROSOPHILA-MELANOGASTER" ENVIRONMENTAL MUTAGENESIS, vol. 5, no. 3, 1983, pages 273-278, XP002578333
- DATABASE BIOSIS [Online] LYNCH ET AL.: 'A two-step method for premeabilization of drosophila eggs', XP008138491 Retrieved from STN Database accession no. PREV199089010137 & CRYOBIOLOGY vol. 26, no. 5, 1989, pages 445 - 452
- BONINI N M ET AL: "Human neurodegenerative disease modeling using Drosophila.", ANNUAL REVIEW OF NEUROSCIENCE, vol. 26, 2003, pages 627-656,

## Description

### I. BACKGROUND

1. In the pharmaceutical industry, chemical compound screening has been performed using cell-based assays in high throughput screening approaches. Template compounds are identified, optimized via medicinal chemistry and then introduced to whole animal systems for evaluation of toxicity. Judging the "success" of the new paradigm of drug discovery on the basis of published data has been difficult (Drews, 2000). High throughput screening has resulted in a large number of "hits," However, very few leads and development compounds, if any, can be credited to the new drug discovery paradigm. (Jurgens, 1999). The major reason these hits have not proved useful for lead development is that the lead that is identified in the cell-based drug screening approach is very often later proved toxic in whole animals (Drews, 2000). Therefore, what is needed in the art are compositions and methods for delivering chemical compounds to invertebrate embryos, with the ability to do so in a high throughput screening assay.

US 2002/0009751 discloses a screening assay using drosophila embryos (optionally transgenic), wherein the compounds are administered diluted in DMSO. Myers et al: CRYOBIOLOGY (1989) vol 26, pages 472-484 studies the effects of cryoprotective agents like DMSO on intracellular ice formation of dechorionized Drosophila embryos.

### II. SUMMARY

2. Disclosed are methods and compositions related to the genetic modification of invertebrate embryos, as well as the delivery of chemical compounds to invertebrate embryos.

### III. BRIEF DESCRIPTION OF THE DRAWINGS

3. The accompanying drawings, which are incorporated in and constitute a part of this specification, illustrate several embodiments and together with the description illustrate the disclosed compositions and methods.

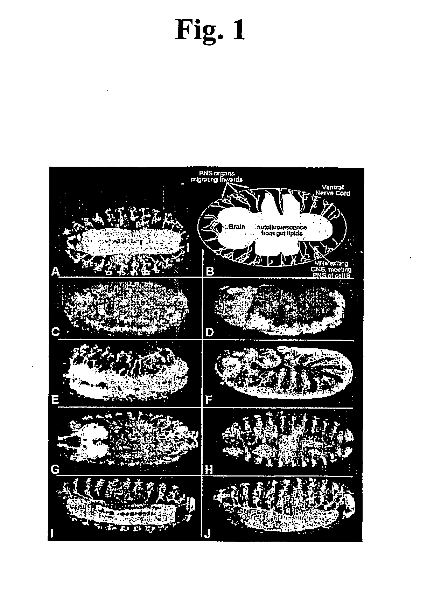

4. **Figure 1** shows the effects of gamma secretase inhibitors on Drosophila development. In all cases, anterior is to the left. All panels are confocal projections of whole mount embryos stained with rhodaminated antibodies directed against HRP, recognizing an antigen on the surface of all insect neurons. Panel A shows a horizontal view of wild type embryo demonstrating an intact central nervous system (CNS) with well

organized peripheral nervous system, consisting of motoneuronal projections and sensory inputs. Panel B shows a diagram of the structures seen in wild type embryos. Panel C shows embryos exposed to 10 *µ*M concentrations of gamma secretase inhibitors generate severe developmental defects, and never complete gastrulation. Panel D shows at 1 *µ*M concentrations, the same drugs allow early cell populations to form but stall development prior to neurogenesis. Panel E shows 100 nM concentrations of gamma secretase inhibitors generate classic Notch neurogenic phenotypes. Panel F shows 10 nM concentrations generate slightly less severe neurogenic defects. Panels I (10 fM) and J (1 fM) show femtomolar concentrations of gamma secretase inhibitors generate defects only in peripheral nervous system pathfinding, a fmding consistent with mildest hypomorphic alleles. Panels G and H show another gamma secretase inhibitor generated more severe defects in ventral nerve cord condensation.

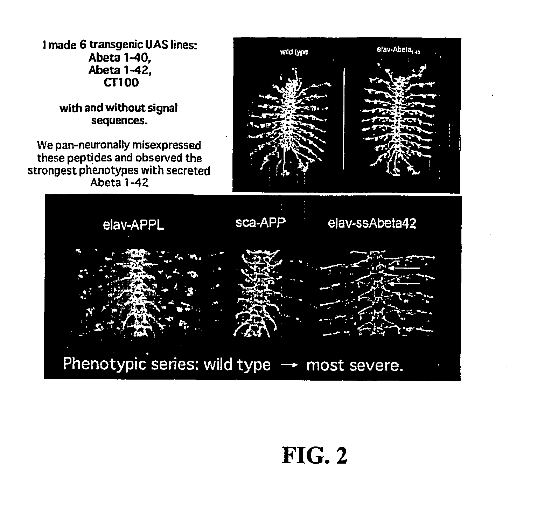

5. **Figure 2** shows allelic series of the phenotypes associated with the misexpression of human APP fragments in the developing cells of the embryonic central nervous system. Pan-neuronal drivers were used to misexpress the amyloidgenic peptides associated with AD. The most severe phenotypes were consistently observed using ₛₛAbeta₁₋₄₂.

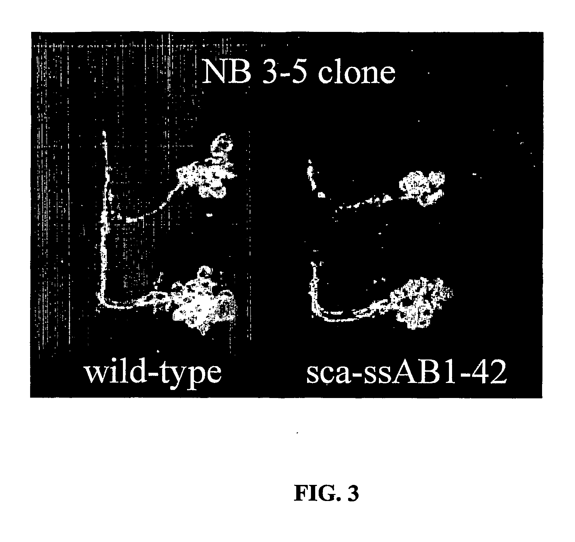

6. **Figure 3** shows that when DiI labeling experiments were performed to examine the single cell defects associated with the misexpression of Abeta peptides pan-neuronally, it was observed that the population of neurons most strongly affected were longitudinal projection neurons. These neurons consistently failed to extend beyond segment boundaries in this gain of function background. When staining was done with fascicular antibodies, clear breaks could be visualized at segment boundaries that typically extended for 25% of the Anteroposterior length of the hemisegment.

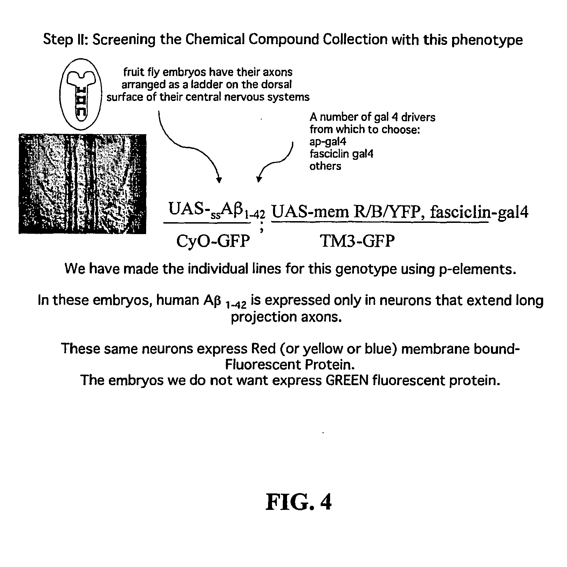

7. **Figure 4** shows a summary of an example of an Alzheimer's model (Example 2). This is an example of a phenotype on which a compound screen can be based.

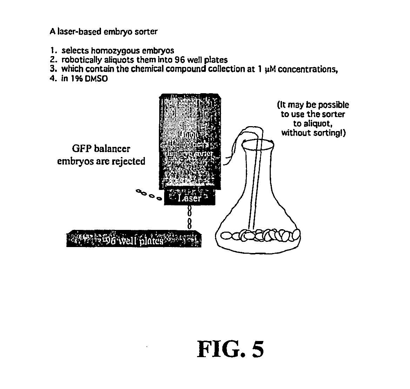

8. **Figure 5** shows a laser-based embryo sorter. The sorter uses a laser-based technology to detect the presence of GFP-tagged balancer chromosomes, eliminates those embryos from further study, but aliquots the embryos without such balancer chromosomes into each well of a 96 well plate, robotically. Each 96 well plate can be filled with 10 embryos per well, within 15 minutes.

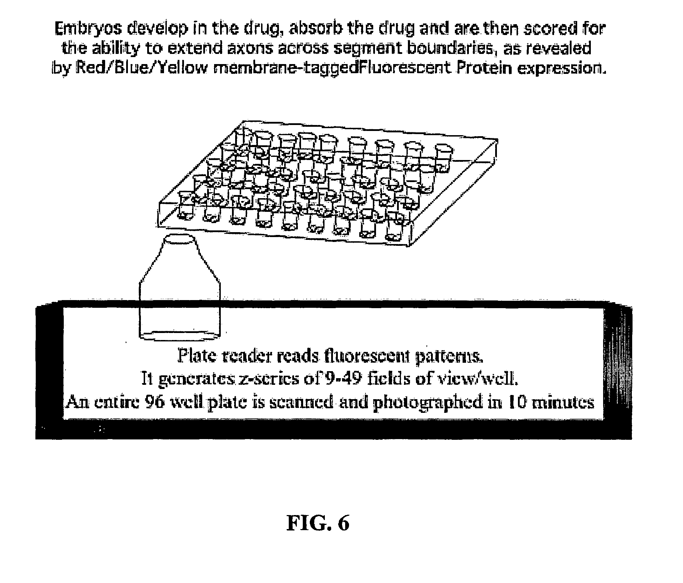

9. **Figure 6** shows a plate reader. Embryos develop in the drug, absorb the drug, and are then scored for the ability to extend axons across segment boundaries, as revealed by Red/Blue/Yellow membrane-tagged Fluorescent Protein expression patterns. An entire 96 well plate can be scanned and photographed in 10 minutes.

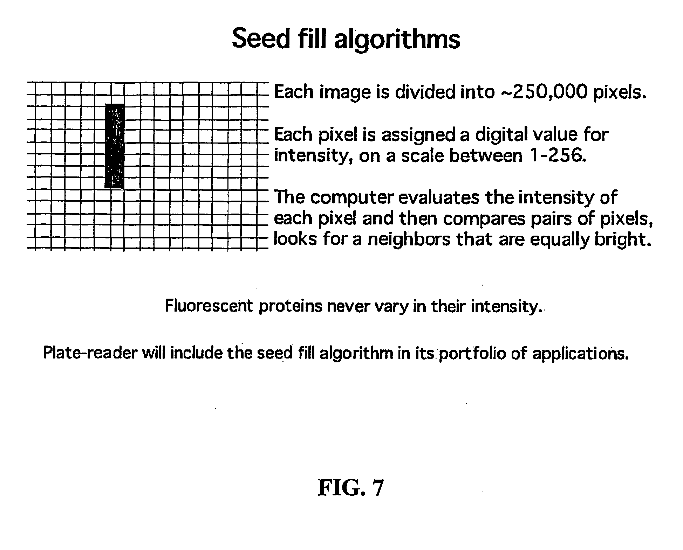

10. **Figure 7** shows a seed fill algorithm. Each image is divided into approximately 250,000 pixels, and each pixel is assigned a digital value for intensity, on a scale between 1-256. The computer evaluates the intensity of each pixel and then compares pairs of pixels and looks for neighbors that are equally bright. Fluorescent proteins never vary in their intensity, and plate readers can be used with seed fill algorithms. Essentially, the algorithm evaluates contiguous lines of pixels. So if membrane tagged fluorescent axons (pixels) are only 100 pixels long in the untreated controls, then a drug that ameliorates the phenotype can be expected to improve this number by what would be a statistically significant amount.

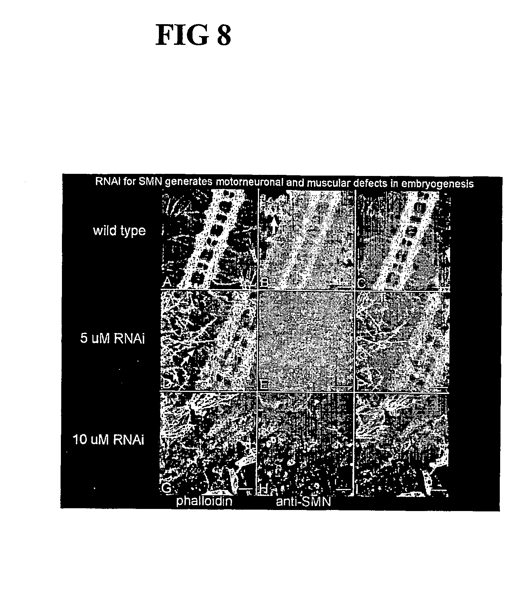

11. **Figure 8** shows RNAi for SMN generates motorneuronal and muscular defects in embryogenesis. Wild type stage 16 embryos of Drosophila melanogaster were filleted and stained with antibodies directed against a strongly conserved epitope of human SMN1; these were counterstained with A488-conjugated phalloidin (panels A-C) and photographed on a Biorad Radiance 2000 confocal microscope. The SMN1 antibodies appear to recognize an epitope in Drosophila that is specific to neurons, their axons (asterisk,panel B) and muscles (arrow, panel B). RNAi experiments at 5 uM concentrations (panels D-F) revealed that the antibody staining is specific, as its cellular specificity disappears and is replaced by relatively low level non-specific background staining (panel E). Motoneuronal projections, visible as regular processes exiting from the CNS (panel A) are absent in RNAi backgrounds (Compare panels A and D). Furthermore, muscles continue to extend myopodia in RNAi backgrounds (arrow, Panel D), apparently because their extension has not been suppressed by successful motoneuronal innervation, which would normally be intact by stage 16. At 10 uM concentrations (panels G-I), little CNS cytoarchitecture remains and SMN staining is non-specific and confined to overlying fat cells. The number of segments in which recognizeable muscles are formed is less than 40%; these panels demonstrate that muscles can form, but continue to exhibit abnormal morphology, including the prolonged extension of myopodia. In all cases, anterior is down. Panels A-C and G-I are photographed at 60X. Panels D-F were photographed at 84X (indicated by size bars). Single cell labelings are in progress to determine whether or not motorneurons do indeed fail to form, or extend axons.

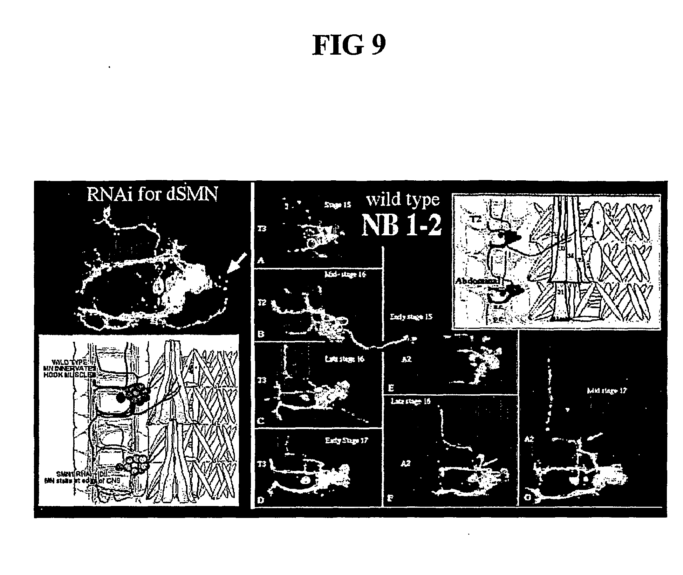

12. **Figure 9** shows that at the single cell level, motoraxons fail to exit the CNS. On the left are DiI lineages that were created in backgrounds in which gene function had been removed by RNAi. (See figure X for method) The embryonic CNS contains 34 stem cells (neuroblasts, NB) that generate invariant lineages. NB 1-2 in thoracic segments generates the DC motorneurons which are the most robust motorneurons of the CNS and which innervate the mouth hook muscles. Larvae are able to retract their heads to the insides of their bodies as a defensive mechanism, when touched or threatened, by a relfelx mediated by the DC motorneuronal innervation mouth hook muscles. In the figure provided, the wild type lineages (including thoracic lineages with robust DC motomeurons) are depicted in the wild type panels on the right, whereas RNAi mutant clones are presented on the left. Even these very large calibre motorneurons are not able to exit the CNS when SMN function is removed. All of the lineages of the ventral nerve cord were examined, and the same failure to extend axons to target fields were found in every lineage examined at high doses. A dose responsiveness to these phenotypes was also found; lower doses of dsRNA allowed some motorneurons to escape this axonal failure. At 5 uM concentrations, virtually all motorneurons were affected identically, as shown in this figure.

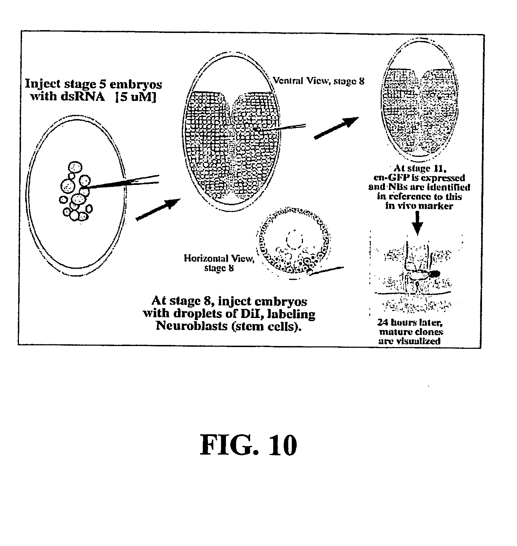

13. **Figure 10** shows RNAi followed by lineage tracing. Embryos were injected with double-stranded RNA at presyncytial blastoderm stages. They were then allowed to develop to stage 8 at 16 degrees C. Then they were injected with 1 micron droplets of DiI suspended in vegetable oil. Single neurectodermal cells take up this dye and are able to transfer this dye to other cells only by lineal transfer during cytokinesis. In this way the entire lineage was visualized, in loss of function backgrounds. The RNAi can be used to inactive one or more genes simultaneously.

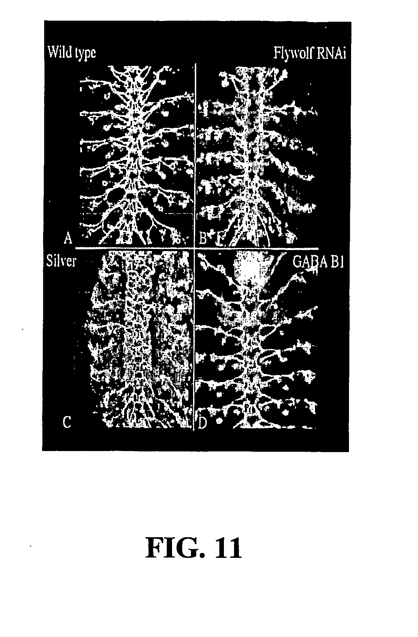

14. **Figure 11** shows filleted Drosophila embryos were fixed and stained with Monoclonal antibody 22C10, staining a known subset of neurons in the embryonic CNS. Panel A: wild type, Panel B: RNAi for Flywolf, C: Silver, a Drosophila ortholog of Carboxypeptidase E, and D: GABA B1, the Drosophila ortholog of the AGAB receptor. Carboxypeptidase E and GABA B1 are putative interactors with Wolfram. Drosophila alleles were tested for similarity of phenotype, and even though the Flywolf RNAi experiments demonstrate cell specific losses, defects observed with carboxypeptidase and GABA B1 orthologs did not generate phenotypes that appeared to place them in the Flywolf pathway. Thus, Flywolf reveals a new genetic pathway related to depression and hearing defects.

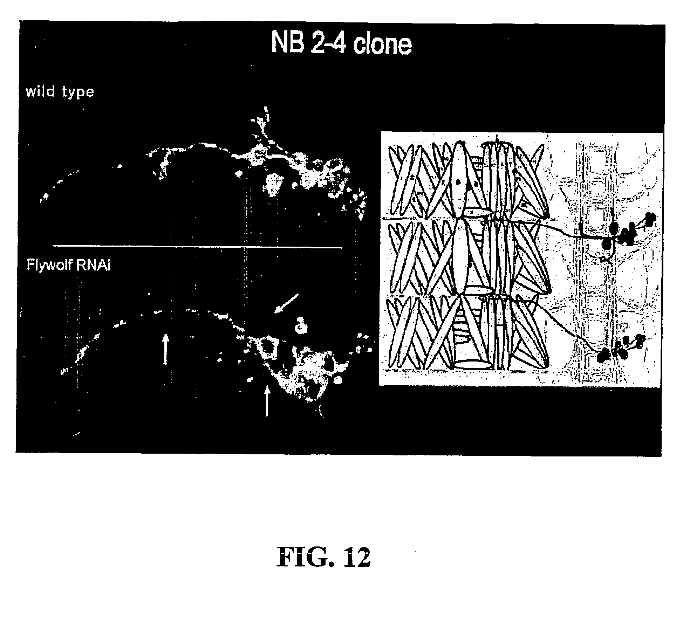

15. **Figure 12** shows embryos were injected with dsRNA. They were allowed to develop to stage 8 and were then injected with single droplets of DiI suspended in Wesson Vegetable oil. In wild type embryos, NB 2-4 generates a single contralaterally projecting motorneuron, and a small cluster of local interneurons. In Flywolf RNAi backgrounds, the motomeuron is relatively unperturbed, but the local intemeurons fail to extend axons, both ipsilaterally and contralaterally. This phenoptye is conserved with those reported in human Wolfram Syndrome patients.

### IV. DETAILED DESCRIPTION

16.

The invention is defined by the following embodiments.
1. A method of making an invertebrate animal embryo useful for screening compounds comprising:
   a) introducing a nucleic acid into the invertebrate embryo forming a transgenic invertebrate embryo;
   b) dechorionating the transgenic invertebrate embryo;
      wherein the invertebrate embryo is transgenic for a human gene; wherein the expression of the human gene by the embryo is an invertebrate model for a disease; and
   c) exposing the embryo to DMSO.
2. A method of screening a candidate compound for its effect on a disease comprising:
   a) dechorionating a transgenic invertebrate animal embryo; wherein the invertebrate embryo is transgenic for a human gene; wherein the expression of the human gene by the embryo is an invertebrate model for the disease;
   b) administering the compound to the invertebrate transgenic animal embryo; and
   c) assaying the effect of the compound on the embryo.
3. A method of screening a compound for its effect on an invertebrate embryo comprising:
   a) dechorionating the embryo,
   b) incubating the dechorionated embryo with the compound and DMSO, and
   c) assaying the effect of the compound on the embryo.
4. The method of embodiments 1 or 2, wherein the effective amount of DMSO is 0.1 % to
   10%, preferably is 1% to 5%.
5. The method of any one of embodiments 1 to 4, wherein the invertebrate is from the genus Drosophila.
6. The method of embodiments 1 or 2, wherein the disease is a neurodegenerative disease.

### A. Definitions

17. As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

18. Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10" as well as "greater than or equal to 10" is also disclosed. It is also understood that the throughout the application, data is provided in a number of different formats, and that this data, represents endpoints and starting points, and ranges for any combination of the data points. For example, if a particular data point "10" and a particular data point 15 are disclosed, it is understood that greater than, greater than or equal to, less than, less than or equal to, and equal to 10 and 15 are considered disclosed as well as between 10 and 15.

19. In this specification and in the claims which follow, reference will be made to a number of terms which shall be defined to have the following meanings:

20. "Optional" or "optionally" means that the subsequently described event or circumstance may or may not occur, and that the description includes instances where said event or circumstance occurs and instances where it does not.

21. "Primers" are a subset of probes which are capable of supporting some type of enzymatic manipulation and which can hybridize with a target nucleic acid such that the enzymatic manipulation can occur. A primer can be made from any combination of nucleotides or nucleotide derivatives or analogs available in the art which do not interfere with the enzymatic manipulation.

22. "Probes" are molecules capable of interacting with a target nucleic acid, typically in a sequence specific manner, for example through hybridization. The hybridization of nucleic acids is well understood in the art and discussed herein. Typically a probe can be made from any combination of nucleotides or nucleotide derivatives or analogs available in the art.

23. The term "transgene" is used herein to describe genetic material which is artificially inserted into the genome of an invertebrate cell. The transgene encodes a product that, when expressed in embryos, gives rise to a specific phenotype. Generally, the transgene encodes a transcription factor or mimetic thereof having the desired result.

24. The terms "alleviating" or "ameliorating" denotes a lessening of an effect of a condition or disorder, such as a detrimental affect, in the animal or situation having the effect, such as the invertebrate embryo. This lessening of the effect can occur phenotypically or genotypically.

25. The term "therapeutically effective" means that the amount of a composition used is of sufficient quantity to ameliorate at least one effect, such as the cause, of a disease.

26. Throughout this application, various publications are referenced. The disclosures of these publications in their entireties are hereby incorporated by reference into this application in order to more fully describe the state of the art to which this pertains. The references disclosed are also individually and specifically incorporated by reference herein for the material contained in them that is discussed in the sentence in which the reference is relied upon.

### B. Methods and Compositions

27. Disclosed are methods and compositions for drug screening in insects, such as Diptera, such as Drosophila melangastor, and hymenoptera, such as the common honey bee. These methods and compositions allow for high-throughput screening of compounds for characteristics such as toxicity and effectiveness, and in certain embodiments, the screening in the insects can correlate with target disease states, in for example, humans, which is aided by correlative data. Disclosed herein, are methods and compositions that are associated with human neurodegenerative diseases, wherein models in the fly are predictive of phenotypes of these diseases. Furthermore, these predictive states in certain embodiments, are identifiable at the embryo stage of the insect. Diseases such as Parkinson's and Alzheimer's are disclosed. These methods and compositions are related to the understanding disclosed herein that embryos of insects, such as those in Diptera and Hymenoptera, can be treated in certain ways, to allow for the entry of molecules into the embryo, without destroying the embryo or destroying the development potential of the embryo. This allows for the screening methods disclosed herein. Furthermore, the insects, can be genetically manipulated, such as the Diptera and Hymenoptera, so that these screens can be performed on particular genetic backgrounds. For example, the insects can be genetically manipulated such that the insects harbor a human transgene, such that when the gene is expressed when a phenotype arises in the insect that is predictive of a particular disease state in humans, such as a neurodegenerative state, such as Parkinson's or Alzheimer's. In association with this, disclosed are particular transgenes that are shown herein to have a predictive phenotype in insects for the disclosed human disease states.

28. Since, in certain embodiments, high-throughput screening is desired, also disclosed herein are machines and algorithms to facilitate the sorting of insect embryos, the categorizing of insect embryos, and the analyzing of the embryos, for example.

29. In part, provided below is a discussion of general drug screening and high-throughput drug screening. Then a discussion of the developmental pathway of insects, such as Diptera, along with important developmental steps related to the embryo stage. Also provided is a discussion of the various parts of the disclosed compositions, such as the altered insect embryos, and the various permutations and alterations on the screening methods disclosed herein. In addition, there is a discussion of a number of the important aspects of, for example, molecular biology techniques and structural aspects of biological macromolecules, such as proteins. Molecules that can be used for screening are also discussed.

### 1. Drug screening

30. Compound screening is typically performed using cell-based assays. Template compounds are identified, optimized via medicinal chemistry and then introduced to whole animal systems for evaluation of toxicity. Previously, drug discovery was performed in whole animal models, usually rodents. When whole animal studies became prohibitively expensive or too time consuming, rodent models were replaced with cell-based assays so that high throughput screening (HTS) approaches could be developed. High-throughput screening can be any screen that allows for the analysis of at least 2000, or more compounds in a day. In certain high-throughput screening methods, more than one compound, such as 10, 20, 30, 40, 50, 60, 70, 80, 90, 100, 150, 200, 250, 300, 350, 400, 450, 500, 600, 700, 800, 900, 1000, 2000, 3000, 4000, 5000, or more compounds are screened simultaneously, meaning analyzed in the same set of experimental manipulations.

### 2. Parkinson's Disease

31. Parkinson's Disease (PD) is the second most common neurodegenerative disease in aging populations. It affects dopaminergic neurons specifically and is characterized by the presence of Lewy bodies and Lewy neurites, the major component of which is α-synuclein. The disease is further characterized by severe behavioral deficits including dyskinesia and tremor. Hereditary forms of Parkinson's disease have been linked to mutations in proteasomal proteins (e.g., parkin, an E3 ubiquitin ligase) and to mutations in α-synuclein, such as the A53T α-synuclein mutation, and the A30P α-synuclein mutation.

### 3. Alzheimer's disease

32. Alzheimer's Disease is even more common than Parkinson's Disease, affecting an estimated 35% of Americans over the age of 75, and 50% of Americans over the age of 80. It is correlated with the deposition of amyloid plaques containing cleaved products of the Amyloid Precursor Protein (APP); these cleavage products (Abeta₁₋₄₀ and Abeta₁₋₄₂) are the result of cleavage in APP's extracellular domain by beta-secretase (BACE) and in the transmembrane domain by gamma-secretase.

### 4. Flies as models of human disease

33. The introduction of human amyloidgenic peptides into the fly have been observed to produce reliable models of human neurodegenerative diseases (Feany and Bender, 2000; Pendelton et al, 2002; Bonini 2001). The fly model of Parkinson's Disease relies on the expression of human mutant α-synuclein in the fly's own dopaminergic neurons. These Drosophila neurons, expressing human mutant α-synuclein, are observed to die in an age-related manner, whereas non-dopaminergic neurons are not affected. α-synuclein-positive fibrils are observed to form Lewy-body-like structures in the brains of these flies, something that has never been observed in any other animal model of Parkinson's Disease. Flies expressing human mutant α-synuclein also experience behavioral deficits that mimic those observed in PD patients, namely dyskinesia and tremor.

34. When treated with the same drugs used to treat human PD patients, flies respond with the same dose-responsiveness and in the same efficacy order observed in humans. In other words, flies respond to drugs in the same dose ranges used for humans (normalized for body weight), and respond well to drugs that are effective in humans, and show less amelioration of their phenotypes when exposed to drugs that are not particularly effective in human patients (Pendleton et al, 2002). When modifier screens were performed using this model, Hsp70 proteins were identified as being able to suppress (albeit incompletely) the age-related neurodegeneration of human mutant α-synuclein in dopaminergic neurons when simultaneously expressed in the same cells. When human PD brains were probed for the expression of these Hsp70 proteins, they were indeed identified as colocalizing with mutant α-synuclein in the Lewy bodies of these brains, revealing for the first time that the fly could be used to predict the pathology of the human (Auluck et al, 2002).

35. Thus, the Drosophila model of PD generates a convincing replica of human age-related dopaminergic neurodegeneration that responds to compounds in a way that suggests the underlying cell biology is identical in both systems; genes/proteins identified as modifying these pathologies in the fly have been identified in identical contexts in humans. However, drug studies using age-related neurodegeneration as a read-out are exceptionally slow, laborious and costly, limiting the number of compounds that can be screened in large adult flies (Reifegerste et al. WO 03/028446, 2003).

36. The value of Drosophila as a screening system for evaluating the biological activities of chemicals has been well-documented (Schulz, et al., 1955). Previously, small numbers of chemical substances were administered to larvae or flies by feeding, and the adult flies were then analyzed for survival and for phenotypic alteration. Alternatively, flies were injected with compounds and then the adult flies were monitored. These methods, however, did not permit high-throughput screening, nor permit the directed search for small molecular weight compounds that interfere with a specific morphogenetic pathway related to a human disease condition.

37. What is needed in the art is the ability to introduce chemical compounds into invertebrate embryos, which improves the accuracy and speed of both template identification and the evaluation of toxicity, as well as analysis of the effectiveness of compounds. In addition, these systems, if appropriately modified will allow for the identification of drugs which can treat a wide variety of diseases, such as neurodegenerative diseases. Such a system would allow for high throughput screening approaches in genetic model systems, using minute concentrations of compounds.

### 5. Drosophila genetics

38. The use of invertebrate model organism genetics and related technologies can greatly facilitate the elucidation of biological pathways (Scangos, 1997). Drosophila melanogaster is the premier model system of genetics. It has a number of advantages that make it ideally suited to compound screening: 1) Drosophila are fast-growing, 2) Dropsophila generate large populations of offspring, and 3) Drosophila cost very little to maintain. Furthermore, because embryonic development requires only 24 hours at room temperature, the growth and maturity of new populations occurs very rapidly, and the phenotypic analysis can proceed. Drosophila have within them, and can accept, transposable elements allowing for the easy introduction of foreign DNA (including human) into its genome. Drosophila also have fluorescently tagged balancer chromosomes allowing for the easy maintenance of lab stocks, the detection of genotype in the embryo and the sorting of heterozygotes from homozygotes. The invariant central nervous system (CNS) lineages are known (Schmid et al, 1999), making single cell phenotypic analysis of CNS defects relatively easy. Furthermore, a developing embryo is an organism with complex organ systems, and almost the entire genome is expressed during embryonic development, making toxicity observed in an embryo reflective of toxicity in an adult, as disclosed herein.

39. The expression of human disease genes or their homologs within developing Drosophila larva models as shown herein can have distinct effects on Drosophila. The effects of these genes in human cells can be elucidated or mimicked in Drosophila, and subsequently the phenotypes which are modified by either the mutations within these interacting genes, or by compounds which block the function of the corresponding gene product, can be produced. These gene products are prime candidates as targets for small compounds which interfere with their function.

### 6. Screening assays

40. Disclosed herein are high throughput assays with a readout system comprised of invertebrate embryos which are genetically-sensitized for a specific disease pathway, such as a human disease pathway.

41. Disclosed herein are methods for conducting a wide variety of biological assays using invertebrate embryos, such as insects, such as Endopterygota, such as Diptera, such as Drosophila melangastor, or Hymenoptera, such as bees. These biological assays can be conducted in a number of ways, including, but not limited to, high throughput screens or screens containing one, two, or more embryos. Large numbers of compositions can be screened simultaneously, or compositions can be screened one at a time (individually). Biological assays can also be used to detect interaction between compositions, as well as to analyze the function of molecules, such as small molecular weight compounds. Large numbers of compounds can be screened for biological/therapeutic activity in a rapid, quantitative and highly efficacious manner. Such assays can be conducted on chemical compounds or any molecule of biological interest, included but not limited to drug candidates, such as those found in combinatorial libraries, siRNAs, and antibodies. High-throughput screening of collections of chemically-synthesized molecules and of natural products can be carried out using the disclosed methods. Furthermore, these methods can be performed on embryos that have been specially engineered to display a desired phenotype. For example, the phenotype can be a phenotype that correlates with a known human or other non-fly animal condition, which has an orthologous condition or response which is correlative within the Drosophila. In this fashion, models for human diseases can be made, within the drosophila, and specifically, a phenotype that is identifiable within the embryo of the Drosophila can be utilized. Such phenotypes are disclosed herein.

42. Also provided are methods for profiling multiple biological responses of drug candidates on invertebrate embryos. The potent and specific biological activities of many low molecular weight molecules make these molecules attractive starting points for therapeutic drug discovery (Hirschmann, et al., 1991). Also disclosed are methods of identifying pharmacological agents for the treatment of disease. For example, by working in the disclosed systems, toxicology and efficacy testing can be performed simultaneously, eliminating the need for doing, for example preclinical data, to arrive at a candidate therapeutic compound, only to have that compound be untractable in a therapeutic setting because of a later identified toxicity problem associated with the compound.

43. In certain embodiments, the methods involve administering a compound to an insect embryo. Typically, the insect embryo has been manipulated. For example, in certain embodiments, the insect embryo first has a hard shell, external to the vitelline membrane, which is removed or degraded using any means. Then, for example, the vitelline membrane is compromised, for example, with any material, such as DMSO, so that materials can enter into the embryo. Thus, disclosed are methods that make and/or utilize embryos that have been treated in this way.

### a) Development and embryos

44. Any invertebrate animal embryo can be used with the methods disclosed herein. Invertebrate organisms include, but are not limited to, arthropods; particularly insects species such as Drosophila (such as the fruit fly *D. melanogaster*), Apis (such as the honeybee A. *mellifera*), and Leptotarsa; acarids; crustacean; mollusks; worms such as Caenorhabditis elegans; coelomates and pseudocoelomates. Of particular use is the insect model organism, Drosophila melanogaster.

45. Drosophila development takes approximately ten days, from the fertilization of an egg by a sperm cell to the eclosion, or hatching, of a mature adult. There are four major stages of development in that ten day period of time, and each of these is separated by a clear, punctuated event.

46. Embryonic development occurs within the first 24 hours following fertilization, and encompasses 17 stages, as seen in Table 1 (http://www.flybase.org; http://sdb.bio.purdue.edu/fly/aimain/1aahome.htm). Embryonic development ends with hatching from the egg shell, with a fully functioning larvae. The hatching event is a test of development, as most genetic defects render an embryo incapable of moving on to larval development.

47. There are two major developmental strategies that have evolved through time: hemimetabolous (partial metamorphic changes) and holometabolous. Hemimetabolous reproduction is the type in which post-hatching developmental stages are primarily enlargement stages and do not require major structural rearrangement or maturation. Fruit flies are holometabolous organisms, meaning embryonic development occurs over a much shorter timespan. The eggs are smaller, invested with far fewer calories, requiring the developing embryo to hatch earlier, but also demanding much less of it in terms of its behavioral repertoire. Fly larvae find and store calories from external sources, as these calories have not been maternally provided.

48. There are three larval stages in Drosophila. These are referred to as larval instars. Each transition in larval development requires a molting, a shedding of the exoskeleton and the formation of a new larger skin (exoskeleton). Each instar is therefore defined from one molting to the next. The first instar spans the period from hatching from the egg, to its first instar molting, and this requires approximately 24-26 hours at room temperature. The second instar goes from the end of the first instar to the start of the third instar, at the second molting and this requires approximately 36 hours. The final instar stages require about 48 hours and is the major feeding stage of Drosophila development, the larvae having grown from approximately 0.5 mm in length to 4.5 mm in length, and increasing in body mass approximately 50-fold and with approximately 80% of the growth occurring during third instar larval stage.

49. White pupae are the earliest stages of pupae development. During larval and pupal development, the tissues fated for perdurance in the adult become polytene, expressing thousands of copies of each gene, in order to facilitate rapid transcription and growth. The only tissues which will endure to adulthood that do not become polytene are the cells of the central nervous system. The larval polytene tissues are referred to as imaginal discs and histoblast nests. During pupal development, the tissues of the larvae that are not CNS, imaginal discs or histoblast nests, liquefy, and are then remodeled into new configurations. All of pupal development requires 4-5 days. The first 65% of pupal development is white pupal stages, in which these remodeling events occur, and the final pupal stages are referred to as pigmented pupal stages, and are melanization stages, where cuticle is formed and pigmented, where final eye development and photoreceptor maturation occurs (as visualized by eye pigment deposition) and where wing, leg, and antennal structures are melanized. Eclosion is the final hatching from the pupal case (the chrysalis) and occurs almost exactly ten days from fertilization.

50. The present methods can be used with an embryo in any of the various stages of development described above. In one embodiment, the embryos are collected from a cage containing many flies, such as thousands or millions of flies. In one embodiment, these cages comprise plastic cylinders approximately one foot in diameter. One end is covered with a mesh circle, allowing free air circulation and at the other end are mesh sleeves allowing easy access to the interior of the cage. The sleeves allow collection plates to be inserted and easily retrieved. Large petri dishes with yeast as a food source are inserted, allowed to remain for approximately 30 minutes and are then removed. Optimally functioning cages can produce plates with millions of embyos within in a 30 minute time span. An automated embryo collector (such as these cages described above) can be used. Embryos can be collected at various times. The embryos can be collected, for example, every 5, 10, 15, 20, 30, 40, 50 or 60 minutes. Embryos can be collected within 30-60 minutes of egg laying so as to allow drugs to penetrate embryos from the earliest stages.

51. The chorion, or the "shell" on the embryos, can be removed after the embryo is collected. The chorion is a soft waxy shell external to the vitelline membrane of the egg. The chorion contains a number of subparts, such as the endochorion, exochorion, and inner chorionic layer. The various layers of the chorion can be used as assays for follicular cell defects, the follicle cells being the ones that secrete the chorion. Drosophila embryos are referred to as having a chorionic membrane, which is completely removed in 100% bleach. When dechorionating an embryo, one is degrading the chorion layer. There are many known methods of dechorionation, including exposure to bleach or sticky tape. When exposed to bleach, the embryo can, for example, be soaked in a 10%, 20%, 30%, 40%, 50%, 60%, 70%, 80%, 90%, or 100% solution. The embryos can be soaked for 1, 2, 3, 4, 5, 6, 7, 8, 9, 10, 15, 30, 45, or 60 or more minutes. Alternatively, the embryos can be dechorionated by brushing the embryos onto a piece of tape, such as double-stick tape, and then removing them, leaving the chorion behind. Tungsten needles can also be used. After dechorionation, the embryo can be treated to facilitate the uptake of a composition. For example, the embryo can be subjected to electroporation, or exposed to a substance that increases the permeability of the embryo, such as an alkaline solution such as soap, tritonX-100 or Tween20, or, preferably, DMSO.

52. In one embodiment, the embryo can be exposed to the alkaline solution, such as DMSO, at a concentration of less than or equal to 0.1%, 0.2%, 0.3%, 0.4%, 0.5%, 0.6%, 0.7%, 0.8%, 0.9%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10%, 11%, 12%, 13%, 14%, or 15% or greater. Preferably, the embryo is exposed to 1.0%-5.0% DMSO.

The embryos can be exposed to the alkaline solution, such as DMSO, at any temperature, for example 10°C, 12°C, 14°C, 16°C, 18°C, 20°C, 22°C, 24°C, 25°C, 26°C, 27°C, 28°C, 30°C, 32°C, 34°C, or 36°C. The warmer the temperature the greater the effect of the DMSO on the embryo. The length of time the embryos are exposed to DMSO can vary, as well. For example, the embryos can be exposed 12, 18, 24, 30, 36, 42, or 48 hours or more, and can be exposed before, during, or after exposure to the compositions of interest. Also, the longer the time the embryos are exposed the greater the effect of the DMSO. For example, a typical protocol can include exposing the embryo to DMSO for 36 hours at 18°C. Another protocol can include exposing the embryo to DMSO for 24 hours at 25°C.

53. The embryos can be exposed from the earliest stages of embryonic development. This allows for maximum uptake and exposure to the compositions of interest. For example, if embryos are collected and sorted before embryonic stage 5, they are still presyncytial blastoderms. This allows them to have compositions for screening available to them at the points at which they segregate CNS tissues, for example. Table 1 shows the 17 classic stages of embryonic development

**TABLE 1: Drosophila Embryo Stages of Development**

| **stage number** | **minutes after fertilization** | **developmental activity** |
|---|---|---|
| 1 | 0-15 | Pronuclear fusion |
| 2 | 15-70 | Preblastoderm (mitotic cycles 1-9) - early cell division - start of cleavage |
| 3 | 70-90 | Pole bud formation - nuclear division 9 |
| 4 | 90-130 | Syncytial blastoderm (mitotic cycles 10-13) - end of cleavage divisions |
| 5 | 130-180 | Cellularization of the blastoderm |
| 6 | 180-195 | Gastrulation to form mesoderm and endoderm - pole cells included in posterior midgut primordium |
| 7 | 195-200 | Germ band elongation - lengthening of the ventral epidermis |
| 8 | 200-230 | Rapid germ band elongation - start of first postblastoderm mitosis - ends with mesodermal parasegmentation |
| 9 | 230-260 | Slow germ band elongation - segmentation of neuroblasts - end of first and start of second postblastoderm mitosis - cephalic furrow formation |
| 10 | 260-320 | Gnathal and clypeolabral lobe formation (head features) - stomodeal invagination - end of second and start of third postblastoderm mitosis |
| 11 | 320-440 | Epidermal parasegmentation evident - tracheal pits invaginate - mesectodermal cell ingress - end of third postblastoderm mitosis - end of neuroblast formation |
| 12 | 440-580 | Germ band retraction - optic lobe invagination - ventral closure - segment formation - fusion of anterior and posterior midgut |
| 13 | 560-620 | End of germ band retraction - CNS and PNS differentation |
| 14 | 620-680 | Dorsal closure of midgut and epidermis - head involution begins |
| 15 | 680-800 | End of dorsal closure - head involution - discs invaginate - cuticle deposition begins - dorsal epidermal segmentation |
| 16 | 800-900 | Advanced denticles visible - Shortening of the ventral nerve cord |
| 17 | Lasts until hatching | The tracheal tree fills with air - Retraction of the ventral cord continues |
| Hatch | 21-22 hours | Hatch to first instar larva |

### b) Genetic manipulation of insects and insertion of transgenes

54. In certain embodiments, it is desired that the insect, such as the Drosophila, have been genetically manipulated prior to being used in one of the screening methods. For example, often, the insect will have one or more transgenes inserted into its genome so that a particular genetic product can be produced. Typically genetic manipulation of insects involves constructs having three components, the transferring element, understood as the vector component, a promoter region, which can also include an enhancer, and the sequence to be expressed, such as a sequence which encodes for a protein, or for example, a siRNA.

55. The genetic modification may be produced by a naturally-occurring, non-wildtype allele of a specific gene which is isolated from a genetic mutagenesis screening assay well-known to those individuals skilled within the art. Alternatively, the genetic modification may be produced by genetic manipulation using genetic recombination/molecular biological techniques known to those skilled within the art. The preferred genetic modification is a non-wild-type allele which, when present in the heterozygous state, results in an altered phenotype that is dose dependent. As utilized herein, the term "dose dependent" is designated as meaning that the genetically-sensitized Drosophila strain exhibits an observably different phenotype for each specific genetic state when it possesses either none, one or two copies of the modified allele of the gene of interest. Loss of function phenotypes are also included. For example, in SMN (Spinal motorneuronal atrophy), the human gene exists as an almost perfect duplication, known as SMN1 and SMN2 (SEQ ID NOs:9-14). In patients homozygous for loss of SMN1, duplications of SMN2 can rescue the phenotype, but only partially. This syndrome can be duplicated using Drosophila versions of these genes. The SMN2 gene is part of a 500 kb inverted duplication on chromosome 5q13. This duplicated region contains at least four genes and repetitive elements which make it prone to rearrangements and deletions. The repetitiveness and complexity of the sequence have also caused difficulty in determining the organization of this genomic region. The telomeric and centromeric copies of this gene are nearly identical and encode the same protein. While mutations in the telomeric copy are associated with spinal muscular atrophy, mutations in this gene, the centromeric copy, do not lead to disease. This gene may be a modifier of disease caused by mutation in the telomeric copy. The critical sequence difference between the two genes is a single nucleotide in exon 7 which is thought to be an exon splice enhancer. It is thought that gene conversion events may involve the two genes, leading to varying copy numbers of each gene. The full length protein encoded by this gene localizes to both the cytoplasm and the nucleus. Within the nucleus, the protein localizes to subnuclear bodies called gems which are found near coiled bodies containing high concentrations of small ribonucleoproteins (snRNPs). This protein forms heteromeric complexes with proteins such as SIP1 and GEMIN4, and also interacts with several proteins known to be involved in the biogenesis of snRNPs, such as hnRNP U protein and the small nucleolar RNA binding protein. Four transcript variants are produced by this gene: centromeric Isoform Variants: Isoform a: Transcript Variant: This variant (a) lacks exon 7, which leads to a premature termination codon. This variant is thought to be the predominant transcript produced by this copy of the gene. Apparently, no Drosophila ortholog has been identified, but vertebrate SMN2 has been shown to be regulated by transformer (a drosophila sex determination gene) and can regulate the splicing of double sex (another gene in that sex regulation pathway). The theory is that SMN2 arose as a perfect duplication of SMN1 during one of the metameric duplications, but that a single base change resulted in an alternative splice site, resulting in these various isoforms. This would account for why Drosophila orthologs do not exist (there are two metameric duplications separating the invertebrates from vertebrates, so the formation of SMN2 and its complicated alternative splice sites would not yet have happened in the fly.) Other "loss of function" phenotypes are included as well.

### 7. Transferring element

56. Transgenic flies can be prepared using any convenient protocol that provides for stable integration of the transgene into the fly genome in a manner sufficient to provide for the requisite spatial and temporal expression of the transgene, i.e. in embryonic neuroblasts. A number of different strategies can be employed to obtain the integration of the transgene with the requisite expression pattern. Generally, methods of producing the subject transgenic flies involve stable integration of the transgene into the fly genome. Stable integration is achieved by first introducing the transgene into a cell or cells of the fly, e.g. a fly embryo. The transgene is generally present on a suitable vector, such as a plasmid. Transgene introduction may be accomplished using any convenient protocol, where suitable protocols include: electroporation, microinjection, vesicle delivery, e.g. liposome delivery vehicles, and the like. Following introduction of the transgene into the cell(s), the transgene is stably integrated into the genome of the cell. Stable integration may be either site specific or random, but is generally random.

57. Where integration is random, the transgene is typically integrated with the use of transposase. In such embodiments, the transgene is introduced into the cell(s) within a vector that includes the requisite P element, terminal 31 base pair inverted repeats. Where the cell into which the transgene is to be integrated does not comprise an endogenous transposase, a vector encoding a transposase is also introduced into the cell, e.g. a helper plasmid comprising a transposase gene, such as pTURBO (Steller & Pirrotta, 1986). Methods of random integration of transgenes into the genome of a target Drosophila melanogaster cell(s) are disclosed in U.S. Pat. No. 4,670,388, the disclosure of which is herein incorporated by reference.

58. In those embodiments in which the transgene is stably integrated in a random fashion into the fly genome, means are also provided for selectively expressing the transgene at the appropriate time during development of the fly. In other words, means are provided for obtaining targeted expression of the transgene. To obtain the desired targeted expression of the randomly integrated transgene, integration of particular promoter upstream of the transgene, as a single unit in the P element vector may be employed. Alternatively, a transactivator that mediates expression of the transgene may be employed. Of particular interest is the GAL4 system as described herein.

59. As mentioned above, an example of a transposable element is the P element (Rubin and Spradling 1982, Spradling and Rubin 1982). The gene of interest is placed between P element ends, usually within a plasmid, and injected into pre-blastoderm embryos in the presence of transposase. This P element, with the gene as cargo, then transposes from the plasmid to a random chromosomal site. P-elements are small transposons with terminal 31-bp inverted repeats, and the element generates 8-bp direct repeats of target DNA sequences upon insertion. The complete element is 2907 bp and is autonomous because it encodes a functional transposase. Incomplete P elements have lost the transposition ability because the transposase has been mutated. But if a complete (autonomous) element exists in the same cell as an incomplete (non- autonomous) element, then the incomplete element can transpose because of the presence of the transposase in the cell (Ashburner 1989, Spradling 1986).

60. Examples of other transposable elements include piggyBAC and Mariner. The piggyBAC element is 2.4 kb in length and terminates in 13 bp perfect inverted repeats, with additional internal 19 bp inverted repeats located asymmetrically with respect to the ends (Cary et al. 1989). The initial sequence analysis of the piggyBAC element revealed a potential RNA polymerase II promoter sequence configuration, typical Kozak translational start signal, and two apparently overlapping long open reading frames. Mariner belongs to a superfamily of DNA-based transposons that includes the *C*. *elegans* Tc1 element. It is small (1.3 kb), encoding one protein (the transposase) flanked by 28 bp inverted repeats (Medhora, M.M., Maruyama, K. and Hartl, D.L. (1991) Genetics 128:311.)

### (1) Promoter element

61. In certain embodiments, the expression of the transgene is targeted to occur in a non-adult stage of the animal. Typically, the transgene is stably integrated into the genome of the animal in a manner such that its expression is directed both spatially and temporally to the desired cell type and the correct developmental stage, i.e. to expression in embryonic neuroblasts, but the transgenes can also be expressed constitutively as disclosed herein. Specifically, the subject transgene is stably integrated into the genome of the animal under the control of a promoter that provides for expression. The transgene may be under the control of any convenient promoter that provides for this requisite spatial and temporal expression pattern, where the promoter can be endogenous or exogenous. For example, a suitable promoter is the promoter located in the Drosophila melanogaster genome at position 86E1-3.

62. Another suitable promoter of the Drosophila origin includes the Drosophila metallothionein promoter (Lastowski-Perry, 1985). This inducible promoter directs high-level transcription of the gene in the presence of metals, e.g., CuSO₄. Use of the Drosophila metallothionein promoter results in the expression system of the invention retaining full regulation even at very high copy number. This is in direct contrast to the use of the mammalian metallothionein promoter in mammalian cells in which the regulatory effect of the metal is diminished as copy number increases. In the Drosophila expression system, this retained inducibility effect increases expression of the gene product in the Drosophila cell at high copy number. Examples of expression systems useful for this method include, but are not limited to, the gene switch protocol and the Haig Keshishian RU486 method. The gene switch system, also known as the Gal80 system, works as follows: a temperature sensitive allele of Gal80 is combined with the usual gal4-UAS constructs: a gal4 driver, a tissue-specific reporter and a Gal80 cassette to repress Gal4 and stop its ectopic gene expression. It takes approximately 3-6 hours to get peak expression and then 15 hours to turn peak expression off , so if toxicity were a problem with any of these constructs, this can be a useful misexpression system. The Haig Keshishian system uses RU486, which is also referred to as a gene switch, and is similar to a Gal4 system developed using estrogen receptors, allowing steroid hormones to act as the activating switch. This system can be thought of as a modified Gal4 system, dependent on the presence of mifepristone (RU486). Transgenic lines express this modified Gal4 protein, which remains inactive until bound to mifepristone; it can then bind to UAS sequences as would a normal Gal4 molecule and initiate transcription. (Osterwalder et al (2001) PNAS 98(22): 12596-601.) 63. The Drosophila actin 5C gene promoter (B. J. Bond et al, 1986) is also a desirable promoter sequence. The actin 5C promoter is a constitutive promoter and does not require addition of metal. Therefore, it is better-suited for use in a large scale production system, like a perfusion system, than is the Drosophila metallothionein promoter. An additional advantage is that the absence of a high concentration of copper in the media maintains the cells in a healthier state for longer periods of time.

64. Examples of other known Drosophila promoters include, e.g., the inducible heatshock (Hsp70) and COPIA LTR promoters. The SV40 early promoter gives lower levels of expression than the Drosophila metallothionein promoter.

65. The transgene may be integrated into the fly genome in a manner that provides for direct or indirect expression activation by the promoter, i.e. in a manner that provides for either *cis* or *trans* activation of gene expression by the promoter. In other words, expression of the transgene may be mediated directly by the promoter, or through one or more transactivating agents. Where the transgene is under direct control of the promoter, i.e. the promoter regulates expression of the transgene in a *cis* fashion, the transgene is stably integrated into the genome of the fly at a site sufficiently proximal to the promoter and in frame with the promoter such that *cis* regulation by the promoter occurs.

66. In other embodiments where expression of the transgene is indirectly mediated by the endogenous promoter, the promoter controls expression of the transgene through one or more transactivating agents, usually one transactivating agent, i.e. an agent whose expression is directly controlled by the promoter and which binds to the region of the transgene in a manner sufficient to turn on expression of the transgene. Any convenient transactivator may be employed. The GAL4 transactivator system is an example of such a system.

67. The GAL4 encoding sequence can be stably integrated into the genome of the animal in a manner such that it is operatively linked to the endogenous promoter that provides expression in the appropriate location. The GAL4 system consists of the yeast transcriptional activator GAL4 and its target the upstream activating sequence (UAS) located within the P-element. Initially, GAL4 and UAS are in separate lines. The UAS is mobilized to generate new UAS insertion lines which remain silent until a source of GAL4 is made available. Under the control of a promoter, the expression of GAL4 is directed in a particular pattern. Specialized promoters can be used to drive expression of GAL4 in tissue and cell specific manners. The GAL4 containing line is then crossed to the UAS containing line. The UAS in the presence of GAL4 directs the expression of any genes adjacent to its insertion site. When the insertion site is located upstream from the coding region over-or ectopic expression occurs.

68. Flies of line 31-1 (also referred to as 1822), as disclosed in Brand & Perrimon, 1993 express GAL4 in this manner, and are known to those of skill in the art. The transgene is stably integrated into a different location of the genome, generally a random location in the genome, where the transgene is operatively linked to an upstream activator sequence, i.e. UAS sequence, to which GAL4 binds and turns on expression of the transgene. Transgenic flies having a UAS: GAL4 transactivation system are known to those of skill in the art and are described in Phelps & Brand, 1998.

69. In one embodiment, the subject transgenic flies are produced by: (1) generating two separate lines of transgenic flies: (a) a first line that expresses GAL4; and (b) a second line in which the transgene is stably integrated into the cell genome and is fused to a UAS domain; (2) crossing the two lines; and (3) screening the progeny for the desired phenotype, i.e. adult onset neurodegeneration. Each of the above steps are well known to those of skill in the art.

### (2) Transgenes

### (a) Neurodegenerative Transgenes

70. Neurodegenerative diseases or disorders according to the disclosed methods comprise Alzheimer's disease, Parkinson's disease, Huntington's disease, amyotrophic lateral sclerosis, Pick's disease, fronto-temporal dementia, progressive nuclear palsy, corticobasal degeneration, cerebro-vascular dementia, multiple system atrophy, argyrophilic grain dementia and other tauopathies, and mild-cognitive impairment. Further conditions involving neurodegenerative processes are, for instance, ischemic stroke, age-related macular degeneration, narcolepsy, motor neuron diseases, prion diseases, traumatic nerve injury and repair, and multiple sclerosis. There are, in addition, a number of other diseases that can be modeled effectively in the fly via loss of function genetics, and that could be used in this screen: SMA (spinal motorneuronal atrophy) AD-HSP (autosomal dominant hereditary spastic paraplegia), dHMNII (distal hereditary motor neuropathy type II, schizophrenia (strophin1 and dysbindin), depression (wolframin), ADOA (autosomal dominant optic atrophy, caused by mutations in dynamin), GDNF for Parkinson's disease (glial cell line derived neurotrophic factor).

71. Many neurodegenerative diseases have been linked to amyloidgenic peptides, and are therefore known as amyloidgenic diseases. Most of the amyloidgenic peptides cause diseases in the elderly and seem related to long term deposition of insoluble fibrils that lead to cellular toxicity by as yet unknown mechanisms. Examples of amyloidgenic diseases include, but are not limited to, Alzheimer's Disease, Parkinson's Disease, Huntington's Disease, massive bleeding strokes (CAA), as well as some forms of Diabetes, polycystic kidney disease, and cardiac amyloidosis.

72. Alzheimer's disease is related to the Amyloid precursor protein and its related peptides. Parkinson's Disease is related to human mutant alpha-synuclein. Huntington's Disease is related to huntingtin. Amyetropic lateral Sclerosis is related to Superoxide Dismutase 1 (SOD1). Progressive nuclear palsy is related to alpha-syncuclein and microtubule associated tau protein. Corticobasal degeneration is related to tau and alpha-synuclein. Age-related macular degeneration is modeled by d-best (drosophila ortholog of bestrophin, implicated in juvenile onset macular degeneration). Motorneuron diseases are related to SMN 1, 2. Depression is related to wolframin. Schizophrenia is related to dysbindin.

73. Amyloidgenic diseases are generally characterized as being diverse, causing disease only in gain of function contexts (i.e., loss of gene function does not lead to amyloidgenic disease), and having causative agents that lack homology to one another. These causative agents are known as amyloidgenic peptides and are characterized by the presence of extensive amounts of beta sheet structure, staining by Congo Red and other Beta sheet intercalators, toxic conformational states associated with the deposition of fibrillar material, and fibrils that develop from pre-fibrillar soluble states.

74. A large number of peptides are now recognized as being amyloidgenic. these include prions (SEQ ID NO: 1), Amyloid A (AA) (SEQ ID NO: 2), Amyloid Precursor Protein (APP) and its derivatives, IAPP (diabetes), TTR, Huntington, alpha-synuclein, polyglutamine expansion repeat proteins, such as SCA1 and Fragile X protein.

75. The protein encoded by SEQ ID NO: 1 is a membrane glycosylphosphatidylinositol-anchored glycoprotein that tends to aggregate into rod-like structures. The encoded protein contains a highly unstable region of five tandem octapeptide repeats. This gene is found on chromosome 20, approximately 20 kbp upstream of a gene which encodes a biochemically and structurally similar protein to the one encoded by this gene. Mutations in the repeat region as well as elsewhere in this gene have been associated with Creutzfeldt-Jakob disease, fatal familial insomnia, Gerstmann-Straussler disease, Huntington disease-like 1, and kuru. Two transcript variants encoding the same protein have been found for this gene. This sequence can be used to generate invertebrate embryo phenotypes which are useful with the methods disclosed herein.

76. The protein encoded by SEQ ID NO: 2 is Amyloid A, which is associated with Cerebral Amyloid Angiopathy. Cerebral Amyloid Angiopathy (CAA) is recognized as a major cause of strokes in the elderly. The term CAA refers to precipitates of Amyloid A protein (an amyloidgenic peptide) in blood vessels that allow blood to leak out and cause hemorrhagic (bleeding) strokes. This sequence can be used to generate invertebrate embryo phenotypes which are useful with the methods disclosed herein.

77. Other sequences that can be used to generate fly phenotypes which are useful with the methods disclosed herein include, but are not limited to, those related to the Amyloid Precursor Protein (APP) and fragments and derivatives thereof. Examples of those fragments and derivatives are known in the art and include APPL, CT100, β-CTF, α-CTF, γ-CTF, Abeta₁₋₄₀, Abeta₁₋₄₂, Abetass₁₋₄₀, and Abetass₁₋₄₂. All sequences referred to herein can be found in GenBank, which sequences are herein incorporated by reference in their entirety. The extracellular portion of APP is cleaved by either α-secretase or β-secretase. This appears to be in response to a signaling event. γ-secretase is then cleaved within the membrane by a process known as regulated intramembraneous proteolyis (RIP), thereby generating the apparently toxic Abeta fragment. There is slippage in the γ-secretase cleavage site. Cleavage of the 100 amino acid C-terminal fragment (CT100) can occur at residues 40, 42, and 57. The sequences of these amyloigenic peptides are as follows
SEQ ID NO: 3: DAEFRHDSGYEVHHQKLVFFAEDVGSNKGAIIGLMV (Abeta₁₋₄₀)

### SEQ ID NO: 4: DAEFRHDSGYEVHHQKLVFFAEDVGSNKGAIIGLMVIA (Abeta₁₋₄₂)

### SEQ ID NO: 5: DAEFRHDSGYEVHHQKLVFFAEDVGSNKGAIIGLMVIA TVIVITLVMLKK QYTS (Abeta₁₋₅₇)

### SEQ ID NO: 6: DAEFRHDSGYEVHHQKLVFFAEDVGSNKGAIIGLMVIA TVIVITLVMLKKQYTSIHHGVVEVDAAVTPEERHLSKMQQNGYENPTYKFFEQMGNC OO (CT100) (also known as CT99)

78. As described above, transgenic invertebrate strains can be made which express genes relating to a neurodegenerative diseases, and specifically to amyloidgenic diseases. Examples of such transgenes include, but are not limited to, UAS-Abeta₁₋₄₀ UAS-ssAbeta₁₋₄₀ UAS-Abeta₁₋₄₂, UAS-ssAbeta₁₋₄₂, UAS-CT100, UAS-ssCT100, UAS-A53T α-synuclein, UAS- α-synuclein, UAS-wolframin, and UAS-Flywolf. Abeta₁₋₄₀ and Abeta₁₋₄₀ are expressed throughout the CNS with pan-neuronal drivers (Example 2). These transgenes can comprise a gene coding for a vertebrate amyloid precursor protein (APP), or a fragment, or a derivative or a variant thereof, a gene coding for a vertebrate α-secretase, for example. Each of these sequences is identified from published sequences, is attached (ligated) to sequences containing UAS binding sites within a vector containing p-element insertion sites. These vectors encoding the assembled transgenes are then injected into Drosophila embryos for the generation of transgenic fly strains. Abeta transgenes relate to Alzheimer's disease, Alpha-synuclein transgenes relate to Parkinson's disease, wolframin and Flywolf are related to a model of depression. Specifically, 12 gain of function mutant lines related to Parksinson's Disease have been generated. These are UAS-A53T alpha synuclein lines. These were generated by Genetic Services, Inc. A sequence for human A53T alpha synuclein was cloned into the pUAST vector, purified, checked for sequence and orientation, and sent to Genetic Services for injection. The twelve lines have been balanced and mapped. Chromosomal insertion sites are as follows: one insert was on the X, three were on the Second Chromosome and five were on the third chromosome. Of the 12 lines, most were homozygous viable, including SZ8. It has been misexpressed and examined for embryonic ventral nerve cords for loss of dopaminergic neurons, and stained with Drosophila TH antibodies and with TUNEL staining (Moelcular Probes).

79. Regarding depression, it has been demonstrated that the mood disorders associated with Wolfram's Syndrome are correlated with disruptions of the small cell layers of the cortex and show that the subcellular pathology is similar in flies and humans (Example 6).

80. In one embodiment, the embryo also expresses a gene coding for a protein having
γ-secretase activity. The gene can code for a presenilin, which can be a vertebrate or an invertebrate gene. It can be a human gene, in particular presenilin-1 or presenilin-2, or fragments, derivatives or variants thereof, for instance known human mutated versions thereof that have been implicated in the early-onset, familiar form of Alzheimer's disease. However, it might also be preferred that the animal of the instant invention expresses the endogenous gene coding for presenilin, or a fragment, or a derivative, or a variant thereof. In one embodiment of the invention, a gene coding for a protein having γ-secretase activity, in particular a gene coding for presenilin, is over-expressed or mis-expressed. The expression can be ectopic, which means that said genes are expressed in a tissue or cell or at a defined developmental stage of the invertebrate animal where they are not normally expressed.

81. The UAS-Gal4 system was used to misexpress the A53T alpha synuclein gene in dopaminergic neurons via the dopadecarboxylase gal4 (Ddc-gal4) line. A53T had been identified in Mediteranean families as a strong link to early onset Parksinson's Disease. The A30P point mutated form of alpha synuclein (identified in a German family as being strongly correlated with early onset Parkinson's disease) was misexpressed in dopaminergic neurons via the Ddc-gal4 driver. A specific age-related neurodegeneration, motordefects that mimicked those observed in human PD patients, and the development of Lewy Body like neurofibrillary tangles in dopaminergic neurons was found. It has been found that these drugs ameliorated the symptoms in flies in the same dosage and efficacy ranges as observed in humans.

### (b) Functional Nucleic Acids

82. Another set of molecules that can make up the transgene are functional nucleic acids.

83. Functional nucleic acids are nucleic acid molecules that have a specific function, such as binding a target molecule or catalyzing a specific reaction. Functional nucleic acid molecules can be divided into the following categories, which are not meant to be limiting. For example, functional nucleic acids include antisense molecules, aptamers, ribozymes, triplex forming molecules, and external guide sequences. These functional nucleic acids can be used in the screening methods disclosed herein. The functional nucleic acid molecules can act as affectors, inhibitors, modulators, and stimulators of a specific activity possessed by a target molecule, or the functional nucleic acid molecules can possess a *de novo* activity independent of any other molecules.

84. RNA interference (RNAi) and small interfering RNA (SiRNA) molecules are examples of functional nucleic acids and can be used with the screening methods disclosed herein. It is thought that RNAi involves a two-step mechanism for RNA interference (RNAi): an initiation step and an effector step. For example, in the first step, input double-stranded (ds) RNA is processed into small fragments (siRNA), such as 21-23-nucleotide 'guide sequences'. RNA amplification appears to be able to occur in whole animals. Typically then, the guide RNAs can be incorporated into a protein RNA complex which is cable of degrading RNA, the nuclease complex, which has been called the RNA-induced silencing complex (RISC). This RISC complex acts in the second effector step to destroy mRNAs that are recognized by the guide RNAs through base-pairing interactions. RNAi involves the introduction by any means of double stranded RNA into the cell which triggers events that cause the degradation of a target RNA. RNAi is a form of post-transcriptional gene silencing. Disclosed are RNA hairpins that can act in RNAi.

85. RNAi has been shown to work in a number of cells, including mammalian and invertebrate cells. In certain embodiments the RNA molecules which will be used as targeting sequences within the RISC complex are shorter. For example, less than or equal to 50 or 40 or 30 or 29, 28, 27, 26, 25, 24, 23, ,22, 21, 20, 19, 18, 17, 16, 15, 14, 13, 12, 11, or 10 nucleotides in length. These RNA molecules can also have overhangs on the 3' or 5' ends relative to the target RNA which is to be cleaved. These overhangs can be at least or less than or equal to 1, 2, 3, 4, 5, 6, 7, 8, 9, 10, 15, or 20 nucleotides long.

86. Methods of RNAi and SiRNA are described in detail in Hannon et al. (2002), RNA Interference, Nature 418:244-250; Brummelkamp et al. (2002), A System for Stable Expression of Short Interfering RNAs in Mammalian Cells, Science 296:550-508; Paul et al. (2002), Effective expression of small interfering RNA in human cells, Nature Biotechnology 20: 505-508, which are each incorporated by reference in their entirety for methods of RNAi and SiRNA and for designing and testing various oligos useful therein.

87. Functional nucleic acid molecules can interact with any macromolecule, such as DNA, RNA, polypeptides, or carbohydrate chains. Often functional nucleic acids are designed to interact with other nucleic acids based on sequence homology between the target molecule and the functional nucleic acid molecule. In other situations, the specific recognition between the functional nucleic acid molecule and the target molecule is not based on sequence homology between the functional nucleic acid molecule and the target molecule, but rather is based on the formation of tertiary structure that allows specific recognition to take place.

88. Antisense molecules are designed to interact with a target nucleic acid molecule through either canonical or non-canonical base pairing. The interaction of the antisense molecule and the target molecule is designed to promote the destruction of the target molecule through, for example, RNAseH mediated RNA-DNA hybrid degradation. Alternatively the antisense molecule is designed to interrupt a processing function that normally would take place on the target molecule, such as transcription or replication. Antisense molecules can be designed based on the sequence of the target molecule. Numerous methods for optimization of antisense efficiency by finding the most accessible regions of the target molecule exist. Exemplary methods would be *in vitro* selection experiments and DNA modification studies using DMS and DEPC. It is preferred that antisense molecules bind the target molecule with a dissociation constant (k_{d}) less than or equal to 10⁻⁶, 10⁻⁸, 10⁻¹⁰, or 10⁻¹². A representative sample of methods and techniques which aid in the design and use of antisense molecules can be found in the following non-limiting list of United States patents: 5,135,917, 5,294,533, 5,627,158, 5,641,754, 5,691,317, 5,780,607, 5,786,138, 5,849,903, 5,856,103, 5,919,772, 5,955,590, 5,990,088, 5,994,320, 5,998,602, 6,005,095, 6,007,995, 6,013,522, 6,017,898, 6,018,042, 6,025,198, 6,033,910, 6,040,296, 6,046,004, 6,046,319, and 6,057,437.

89. Aptamers are molecules that interact with a target molecule, preferably in a specific way. Typically aptamers are small nucleic acids ranging from 15-50 bases in length that fold into defined secondary and tertiary structures, such as stem-loops or G-quartets. Aptamers can bind small molecules, such as ATP (United States patent 5,631,146) and theophiline (United States patent 5,580,737), as well as large molecules, such as reverse transcriptase (United States patent 5,786,462) and thrombin (United States patent 5,543,293). Aptamers can bind very tightly with k_{d}s from the target molecule of less than 10⁻¹² M. It is preferred that the aptamers bind the target molecule with a k_{d} less than 10⁻⁶, 10⁻⁸, 10⁻¹⁰, or 10⁻¹².

Aptamers can bind the target molecule with a very high degree of specificity. For example, aptamers have been isolated that have greater than a 10000 fold difference in binding affinities between the target molecule and another molecule that differ at only a single position on the molecule (United States patent 5,543,293). It is preferred that the aptamer have a k_{d} with the target molecule at least 10, 100, 1000, 10,000, or 100,000 fold lower than the k_{d} with a background binding molecule. It is preferred when doing the comparison for a polypeptide for example, that the background molecule be a different polypeptide. Representative examples of how to make and use aptamers to bind a variety of different target molecules can be found in the following non-limiting list of United States patents: 5,476,766, 5,503,978, 5,631,146, 5,731,424, 5,780,228, 5,792,613, 5,795,721, 5,846,713, 5,858,660 , 5,861,254, 5,864,026, 5,869,641, 5,958,691, 6,001,988, 6,011,020, 6,013,443, 6,020,130, 6,028,186, 6,030,776, and 6,051,698.

90. Ribozymes are nucleic acid molecules that are capable of catalyzing a chemical reaction, either intramolecularly or intermolecularly. Ribozymes are thus catalytic nucleic acid. It is preferred that the ribozymes catalyze intermolecular reactions. There are a number of different types of ribozymes that catalyze nuclease or nucleic acid polymerase type reactions which are based on ribozymes found in natural systems, such as hammerhead ribozymes, (for example, but not limited to the following United States patents: 5,334,711, 5,436,330, 5,616,466, 5,633,133, 5,646,020, 5,652,094, 5,712,384, 5,770,715, 5,856,463, 5,861,288, 5,891,683, 5,891,684, 5,985,621, 5,989,908, 5,998,193, 5,998,203, WO 9858058 by Ludwig and Sproat, WO 9858057 by Ludwig and Sproat, and WO 9718312 by Ludwig and Sproat) hairpin ribozymes (for example, but not limited to the following United States patents: 5,631,115, 5,646,031, 5,683,902, 5,712,384, 5,856,188, 5,866,701, 5,869,339, and 6,022,962), and tetrahymena ribozymes (for example, but not limited to the following United States patents: 5,595,873 and 5,652,107). There are also a number of ribozymes that are not found in natural systems, but which have been engineered to catalyze specific reactions de novo (for example, but not limited to the following United States patents: 5,580,967, 5,688,670, 5,807,718, and 5,910,408). Preferred ribozymes cleave RNA or DNA substrates, and more preferably cleave RNA substrates. Ribozymes typically cleave nucleic acid substrates through recognition and binding of the target substrate with subsequent cleavage. This recognition is often based mostly on canonical or non-canonical base pair interactions. This property makes ribozymes particularly good candidates for target specific cleavage of nucleic acids because recognition of the target substrate is based on the target substrates sequence. Representative examples of how to make and use ribozymes to catalyze a variety of different reactions can be found in the following non-limiting list of United States patents: 5,646,042, 5,693,535, 5,731,295, 5,811,300, 5,837,855, 5,869,253, 5,877,021, 5,877,022, 5,972,699, 5,972,704, 5,989,906, and 6,017,756.

91. Triplex forming functional nucleic acid molecules are molecules that can interact with either double-stranded or single-stranded nucleic acid. When triplex molecules interact with a target region, a structure called a triplex is formed, in which there are three strands of DNA forming a complex dependant on both Watson-Crick and Hoogsteen base-pairing. Triplex molecules are preferred because they can bind target regions with high affinity and specificity. It is preferred that the triplex forming molecules bind the target molecule with a k_{d} less than 10⁻⁶, 10⁻⁸, 10⁻¹⁰, or 10⁻¹². Representative examples of how to make and use triplex forming molecules to bind a variety of different target molecules can be found in the following non-limiting list of United States patents: 5,176,996, 5,645,985, 5,650,316, 5,683,874, 5,693,773, 5,834,185, 5,869,246, 5,874,566, and 5,962,426.

92. External guide sequences (EGSs) are molecules that bind a target nucleic acid molecule forming a complex, and this complex is recognized by RNase P, which cleaves the target molecule. EGSs can be designed to specifically target a RNA molecule of choice. RNAse P aids in processing transfer RNA (tRNA) within a cell. Bacterial RNAse P can be recruited to cleave virtually any RNA sequence by using an EGS that causes the target RNA:EGS complex to mimic the natural tRNA substrate. (WO 92/03566 by Yale, and Forster and Altman, Science 238:407-409 (1990)).

93. Similarly, eukaryotic EGS/RNAse P-directed cleavage of RNA can be utilized to cleave desired targets within eukarotic cells. (Yuan et al., Proc. Natl. Acad. Sci. USA 89:8006-8010 (1992); WO 93/22434 by Yale; WO 95/24489 by Yale; Yuan and Altman, EMBO J 14:159-168 (1995), and Carrara et al., Proc. Natl. Acad. Sci. (USA) 92:2627-2631 (1995)). Representative examples of how to make and use EGS molecules to facilitate cleavage of a variety of different target molecules be found in the following non-limiting list of United States patents: 5,168,053, 5,624,824, 5,683,873, 5,728,521, 5,869,248, and 5,877,162.

### (3) Other possible components for transgene constructs

94. A desirable gene expression unit or expression vector for the protein of interest can also be constructed by fusing the protein coding sequence to a desirable signal sequence. The signal sequence functions to direct secretion of the protein from the host cell. Such a signal sequence may be derived from the sequence of tissue plasminogen activator (tPA). Other available signal sequences include, e.g., those derived from Herpes Simplex virus gene HSV-I gD (Lasky et al, Science, 233:209-212 1986).

95. The DNA coding sequence can also be followed by a polyadenylation (poly A) region, such as an SV40 early poly A region. The poly A region which functions in the polyadenylation of RNA transcripts appears to play a role in stabilizing transcription. A similar poly A region can be derived from a variety of genes in which it is naturally present. This region can also be modified to alter its sequence provided that polyadenylation and transcript stabilization functions are not significantly adversely affected.

96. The recombinant DNA molecule can also carry a genetic selection marker, as well as the protein gene functions. The selection marker can be any gene or genes which cause a readily detectable phenotypic change in a transfected host cell. Such phenotypic change can be, for example, drug resistance, such as the gene for hygromycin B resistance (i.e., hygromycin B phosphotransferase).

97. Alternatively, a selection system using the drug methotrexate, and prokaryotic dihydrofolate reductase (DHFR) gene, can be used with invertebrate cells. The endogenous eukaryotic DHFR of the cells is inhibited by methotrexate. Therefore, by transfecting the cells with a plasmid containing the prokaryotic DHFR which is insensitive to methotrexate and selecting with methotrexate, only cells transfected with and expressing the prokaryotic DHFR will survive. Unlike methotrexate, selection of transformed mammalian and bacterial cells, in the Drosophila system, methotrexate can be used to initially high-copy number transfectants. Only cells which have incorporated the protective prokaryotic DHFR gene will survive. Concomitantly, these cells have the gene expression unit of interest.

### (4) Analysis of the transgene

98. Transcription and expression of the heterologous protein coding sequences can be monitored. For example, Southern blot analysis can be used to determine copy number of the gp120 gene. Northern blot analysis provides information regarding the size of the transcribed gene sequence. The level of transcription can also be quantitated. Expression of the selected protein in the recombinant cells can be further verified through Western blot analysis, for example. The transcripts and inserts can also be monitored using the polymerase chain reaction or other amplification procedures used to amplify specific sequences, understood in the art. Furthermore, any nucleic acid detection assay, such as DNA chip technology can also be used to monitor the presence of specific nucleic acids.

### (5) Strains

99. Any Drosophila cell line can be used with the methods described herein. Table 2, for example, provides a list of cell lines that can be used with the methods described herein. Examples of Drosophila cell lines can also be found at The FlyBase Consortium (The FlyBase database of the Drosophila genome projects and community literature. 2003, Nucleic Acids Research 31:172-175. http://flybase.org/.

100. It will be readily apparent to those individuals skilled in the art that any genetically-modified Drosophila strains can be used with the methods disclosed herein. Preferably, the Drosophila strain has an easily monitored phenotype which is detectably altered in response to the modification of genes related to disease pathways. Appropriate disease pathways include, but are not limited to, neurodegenerative disease signaling pathways such as Parkinson's and Alzheimer's; singaling pathways controlled by the Ras proto-oncogene; the WNT tumor suppressor gene; Rb (retinoblastoma tumor suppressor gene); HH (hedgehog development regulator) or the HH vertebrate homolog SHH (sonic hedgehog developmental regulator); activated protein kinase B (PKB/AKT); insulin receptor; insulin receptor substrates (IRS); c-src proto-oncogene; c-Jun proto-oncogene; c-myc proto-oncogene; p53; Janus kinases (JAK/STAT pathway); nitric oxide (NO); calmodulin; cAMP dependent protein kinase (PKA); Ca²⁺ dependent protein kinase (PKC); growth factors such as GH, TGF, PDGF and the like; receptor tyrosine kinases (RTKs); interferons (IFN); lipid metabolites; steroid hormones; phosphatidylinositol; G-protein coupled receptors; c-abl proto-oncogene; TGFβ and Smad gene family members; interleukins; GTPases; and ionophores.

### 8. Insects

101. Arthropods include Crustacea, which are things like prawns, crabs and woodlice; Myriapoda, which are centipedes, millipedes and such; Chelicerata (Arachnida), which are spiders, scorpions and harvestmen etc., and Uniramia (Insecta), which are things like beetles, bees and flies.

Insects are found in the phylum Arthorpoda, Subphylum Insecta (also often called a class), Class Hexapoda, and Subclasses Apterygota, Exopterygota, and Endopterygota. The Apterygota includes the orders Protura, Collembola (Springtails), Thysanura (Silverfish), Diplura (Two Pronged Bristle-tails). The Exopterygota includes the orders Ephemeroptera (Mayflies), Odonata (Dragonflies), Plecoptera (Stoneflies), Grylloblatodea, Orthoptera, Phasmida (Stick-Insects), Dermaptera (Earwigs), Embioptera (Web Spinners), Dictyoptera (Cockroaches and Mantids), Isoptera (Termites), Zoraptera, Psocoptera (Bark and Book Lice), Mallophaga (Biting Lice), Siphunculata (Sucking Lice), Hemiptera (True Bugs) Thysanoptera, The Endopterygota includes the orders Neuropter (Lacewings), Coleoptera (Beetles), Strepsiptera (Stylops), Mecoptera (Scorpionflies), Siphonaptera (Fleas), Diptera (True Flies which are unusual in that they only have one pair of functional wings. The other pair is reduced to a pair of knoblike organs, called halteres, which play a part in stabilizing these insects during flight. True flies include house flies and bluebottles, mosquitoes, horseflies, midges, and antler-headed flies. Also included are Lepidoptera (Butterflies and Moths), Trichoptera (Caddis Flies), and Hymenoptera (Ants Bees and Wasps).

102. An example of Drosophila species that are useful include, but are not limited to, *D. pseudoobscura, D. virilis, D. hydei, D. mauritiana, D. simulans, D. subobscura,* and *D. odysseus.*

### 9. Sorting of embryos

103. Drosophila lethal mutations are maintained over a balancer chromosome. Balancer chromosomes are multiply inverted chromosomes, used to minimize recombination events, that carry lethal mutations so that homozygous balancer embryos die and that also carry dominant markers so that the presence of a balancer chromosome can be readily identified. Embryos produced by parents with the genotype mutation/balancer produce offspring of the following genotypes: homozygous mutant embryos (mutation/mutation), heterozygous embryos (mutation/balancer) and homozygous balancer embryos (balancer/balancer). Only the heterozygous mutant/balancer embryos can develop into adults and so maintain a stable stock. However, for any embryo collection from these parents, only about 25% of the embryos contain the homozygous mutation of interest. Using a GFP marker geneon the balancer chromosome it is possible to identify this minority population of mutant embryos from their balancer containing siblings, in living embryos.

104. In one embodiment, embryos can be sorted using an automated system. An automated sorter can be used to achieve two important goals: 1) heterozygous embryos (which have normal phenotypes in comparison to homozygous siblings) can be separated from homozygous embryos; 2) homozygous embryos can be precisely aliquoted. The principle of automated sorting of living Drosophila embryos can be demonstrated using flow cytometry technology. The Union Biometrica COPAS SELECTsystem™ is an example of such a system. It can separate Drosophila embryos expressing genes of interest from initially large populations of embryos.

105. The COPAS SELECT can sort embryos exhibiting genetic expression by measuring the fluorescence intensity of the protein reporter expressed during early embryo development. For example, a population of embryos containing GFP-tagged balancer chromosomes such as CyO armadillo-GFP, CyOactinGFP, or CyOKruppelGFP. The same drivers direct GFP expression on the third chromosome balancers (TM3 and TM6) can be used. Those embryos not expressing GFP can be selected and sorted from the original population with a high level of accuracy and purity. Embryos can also be isolated that display specific morphological characteristics. For example, an embryo can be sorted out of a population based on its size. The relative size of each embryo can also be analyzed. This information may be used in conjunction with fluorescence intensity, to isolate unique embryos.

106. In one embodiment, Drosophila embryos are diluted using an appropriate solution such as "Liquid Gold". The embryo concentration can then be checked by extracting a known volume of liquid out from the preparation and by counting the amount of embryos present. Multiple aliquots of the solution can be extracted in order to confirm the recommended concentration of embryos. The diluted embryos can then be analyzed. In the COPAS SELECTsystem™, two parameters of optical characteristics, Green Fluorescence Intensity (FLU1) and Extinction (EXT), can be used to initially analyze the population. EXT is a measure of optical density and FLU is a measure of fluorescence including autofluorescence, fluorescent protein expression, or fluorescent markers.

107. All of the embryos analyzed during the experiment can be displayed simultaneously. The desired embryos are then isolated from the whole population. The sort region can be defined using FLU1 (green fluorescence intensity) and FLU2 (red fluorescence intensity) as the two parameters. A collection vial can then be used to gather the embryos of interest. When using these methods, a greater than 95% purity when 15 to 25% of the total population was selected can be observed (COPAS SELECTsystem Application Guide S01, http://www.unionbio.com/applications/ app notes/ d_melan_files/ DrosophilaANS01.pdf).

### 10. Analysis/Readout assay

108. After sorting, Drosophila embryos can be used in the screening methods disclosed herein. Screening optionally takes place in multi-well plates. Multi-well plates are standard in the art and come in a variety of sizes and shapes. For example, the multi-well plate can be 24, 48, or 96 well plates. Such screening assays can be automated or further modified for high throughput analysis.

109. Chemical compounds are then delivered to embryos in the well plates. For high throughput screening, each well can include numerous test components. An assay of phenotypic improvement is then carried out using seed fill algorithms. If a positive reaction is detected in a well, the screening is repeated with one of the test compounds contained in a single well.

110. Seed fill algorithms can be used in the following manner. Embryos develop in the drug, absorb the drug, and are then scored for their reaction to the drug. The reaction can be revealed, for instance, by the expression of Red/Blue/Yellow Fluorescent Protein. A plate reader can use a number of extra features to minimize error in pattern detection, and image data can then be collected, transferred via high speed data storage systems, and then analyzed. The initial analysis can be performed by computer, using a seed fill algorithm, which determines whether a data point should be kept for subsequent study or whether it should be eliminated. Example 3 describes one embodiment of a seed fill algorithm. For example, the plates can be black-welled plates available from a number of suppliers. These are flat-bottomed plates with black walls to minimize fluorescent bleed-through, effectively isolating the contents of each well for optical analysis. Plates are automatically loaded onto the plate reader. Internal standards direct the focal plane to a particular focal plane within the emrbyo. Such standards include a number of specific markers such as vital dyes which are membrane specific. Because the axons of the embryonic CNS lie on the dorsal surface of the CNS, the highest concentration of this dye (in the violet visible light range) directs the focal plane to the dorsal surface of the CNS. Focusing 35 microns below or above the center of this intense purple stain directs the reader to photograph at the level of dopaminergic neurons in the vnc nerve cord, for example. Such specific landmarks can be determined a priori for each disease model of interest. Z-series stacks can be acquired, or single images and these comprise the data sets for each well. These data sets are subjected to analysis by seed fill algorithms.

111. Seed fill algorithms are commonly used algorithms in which the computer performs a pixel by pixel analysis of intensity levels. Since fluorescent proteins never vary in intensity, seed fill algorithms can analyze them with consistency and reproducibility. changes in the number of contiguous pixels meeting the intensity requirement are scored as positive or negative hits, depending on the assay and disease being assayed. For example, for the Alzheimer's model, amelioration of the phenotype can be scored as increased numbers of contiguous pixels. For a Parkinson's model in which distance between intensity loci are measured, decreased readouts can be scored as hits.

### 11. Exemplary Embodiments

112. Specifically, disclosed are methods of screening a candidate compound for its effect on a disease. These methods include administering the compound to an invertebrate animal embryo and assaying the effect of the compound on the embryo. These methods also include comparing phenotypes of the transgenic embryo treated with the compound to a transgenic invertebrate embryo not treated with the compound, wherein a difference in the phenotypes is indicative of the alleviating activity of the candidate compound.

113. These methods also include introducing a nucleic acid into an invertebrate animal, wherein the nucleic acid encodes a peptide associated with a phenotype of the disease, expressing the phenotype in an embryo of the invertebrate animal, aliquoting the embryos of into wells of a plate, delivering a test compound to the well of the plate, and screening for a change in the phenotype associated with the disease, a change indicating a compound with an effect upon the disease.

114. Also disclosed are methods of screening a compound for its effect on an invertebrate embryo. These methods include dechorionating the embryo, incubating the dechorionated embryo with the compound and a cell perforating agent such as DMSO, and assaying the effect of the compound on the embryo. Also disclosed are high through put mechanisms utilizing the above screening methods.

115. Disclosed herein are a methods of manipulating an invertebrate animal embryo, comprising delivering a composition to the embryo. Also disclosed is a method of introducing a compound into an invertebrate embryo, comprising administering to the embryo an effective amount of an agent that facilitates cellular uptake, such as DMSO.

116. Disclosed are methods of screening for a test compound with an effect on a phenotype associated with a neurodegenerative disease. These methods can include, for example, the steps of placing transgenic embryos in a read out assay, delivering a test compound to the read out assay, and scoring the embryos for the ability to extend axons across segment boundaries, said ability indicative of a test compound with an effect on a phenotype associated with a neurodegenerative disease. The transgenic embryos can express a membrane-tagged fluorescent protein, for example.

117. Also disclosed are methods for evaluating the phenotypes of invertebrate animal embryos. Disclosed herein are a methods of manipulating an invertebrate animal embryo, comprising delivering a composition to the embryo. Also disclosed is a method of introducing a compound into an invertebrate embryo, comprising administering to the embryo an effective amount of an agent that facilitates cellular uptake, such as DMSO

118. Also disclosed are methods of making a genetic assay. These methods include introducing a nucleic acid into an invertebrate animal, wherein the nucleic acid encodes a peptide associated with a phenotype of a disease, expressing the phenotype in an embryo of the invertebrate animal, aliquoting the embryos into wells of a plate, and delivering a test compound to the well of the plate.

119. Specifically, disclosed are methods of making a genetic assay for a neurodegenerative disease. Included in these methods are, for example, the steps of introducing a nucleic acid into an invertebrate animal, wherein the nucleic acid encodes a peptide associated with a phenotype of the neurodegenerative disease, introducing a membrane-bound fluorescent protein in to the invertebrate animal, introducing a UAS-Gal4 system into the invertebrate animal, and expressing the phenotype in an embryo of the invertebrate animal. The peptide associated with a phenotype of the neurodegenerative disease can be, for example, an amyloidgenic peptide, such as Abeta₁₋₄₂ or Abeta₁₋₄₀. A signal sequence can also be introduced into the invertebrate animal.

120. Disclosed are methods of scoring a plate. Such methods can include the steps of placing transgenic embryos expressing a phenotype associated with a disease in a read out assay, delivering a compound to the read out assay, and using a seed fill algorithm to score the embryos, wherein the algorithm evaluates contiguous lines of pixels, wherein a compound that ameliorates the phenotype gives a higher score in comparison to a control embryo.

121. Also disclosed are kits for use in screening compounds. The kit can include, for example, transgenic invertebrate animal embryos and a suitable container. Also disclosed is an embryo sorter capable of selecting homozygous embryos, as well as a read out assay useful in a high throughput screen of invertebrate embryos. An example of a read out assay comprises using the following algorithm: 1) images are collected at 512X512 sizes; 2) each of the resulting 262,144 pixels is evaluated on a typical 1-256 grey scale. Because the fluorescent proteins do not vary in intensity, there is no variability from prep to prep or among embryos within a prep. 3) Each pixel is analyzed for its intensity. If it meets the threshold value (e.g. 200), it is assigned a value of 1. If it fails to meet that intensity level, it is assigned a value of 0. 4) Each pixel is evaluated in a pairwise fashion, as follows: Each pixel is surrounded by 8 other pixels. Consider a number pad of the type found on telephones or calculators. If the number 5, surrounded by the numbers/pixels 7,8,9,6,3,2,1, and 4 is found to be adjacent to pixels sufficently bright to meet the cutoff at positions 2 and 8, the algorithm moves in both of those vertical directions until it encounters a pixel that fails to meet that criterion. The logic string determines a directionality. So that if 8 and 2 are found to meet the intensity criteria, then future consideration is given only to pixels in that column and branching in non-linear directions is discounted. However, unless a directionality is specified, all linear clusters will be counted--including horizontal ones; branched clusters are never considered. 5) The longest strings are counted and retained as the data point for that image, and then the computer moves on to the next field of view.

122. For example, the read out for the Alzheimer's Disease phenotype is that axons extend further in the presence of drugs which suppress the ability of the Abeta peptides to stall them. So if the Abeta peptide poisons axonal transport, then molecules that improve axonal transport by for example, upregulating the expression or function of kinesins (the motors that bind cargo to microtubules for axonal transport to the synapse) are be expected to improve the ability of axons to extend in these genetic backgrounds. The abeta peptide is still there, but the drug is directly interfering with its pathological properties. In the case of Parkinson's Disease, in which A53T alpha-synculein misexpression in dopaminergic neurons leads to the loss or shrinkage of dopaminergic neurons, the seed fill algorithm is designed to either determine absolute size of dopaminergic neurons, or the distance between dopaminergic neurons. Drugs that interfere with A53T alpha synuclein toxicity, perhaps by upregulating degradation of even insoluble peptides by the proteasome, or by enhancing the ability of the mitochondria to tolerate the oxidative stress putatively incurred in alpha-synuclein overload would be recovered as positive "hits". Also recovered as hits in a Parkinson's disease screen are molecules that inhibit dopamine synthesis, since the neurotransmitter, dopamine, actually stabilizes the amyloidgenic fibrils derived from mutated forms of alpha-synuclein (like A53T Alpha synuclein); in other words, the reason dopaminergic neurons are susceptible to the accumulation of alpha synuclein plaques can be because dopamine actually reinforces the plaques.

123. Also disclosed are methods of making an invertebrate animal embryo useful for screening compounds. For example, the embryo can be genetically modified. A recombinant DNA molecule or vector containing a heterologous protein gene expression unit can be used to transfect invertebrate cells (United States Patents 4,670,388 and 5,550,043, herein incorporated by reference in their entirety.) A gene expression unit can contain a DNA coding sequence for a selected protein or for a derivative thereof. Such derivatives can be obtained by manipulation of the gene sequence using traditional genetic engineering techniques, e.g., mutagenesis, restriction endonuclease treatment, ligation of other gene sequences including synthetic sequences and the like (T. Maniatis et al, Molecular Cloning, A Laboratory Manual., Cold Spring Harbor Laboratory, Cold Spring Harbor, N.Y. (1982).

### C. Compositions

124. Disclosed are the components to be used to prepare the disclosed compositions as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a particular compound is disclosed and discussed and a number of modifications that can be made to a number of molecules including the amino acids are discussed, specifically contemplated is each and every combination and permutation of the transgene and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods.

### 1. Compositions used in screening

125. The ability to introduce chemical compounds into Drosophila embryos is the basis for screening methods that provide four major advances for high-throughput screens of chemical compounds: it improves the accuracy and speed of template identification; it allows for the simultaneous evaluation of compound efficacy and organism-wide compound toxicity; it allows high throughput screening approaches in genetic model systems in which human diseases can be modeled with unparalleled success (Feany and Bender, 2000); and it is the most cost-effective approach for compound screening to date, requiring on the order of 1/1000^{th} the drug typically required in mammalian cell-based assays. The ease of secondary assays to validate targets is an additional advantage.

126. The composition can be any compound of a suitable size for uptake by the embryo. For example, the composition can follow the guidelines of "Lipinski's Rule of Five." (Lipinski, 1997). Lipinski's Rule of Five is particularly useful when the goals of compound design are (i) to have less than 5 hydrogen donors, (ii) less than 10 hydrogen bond acceptors, (iii) molecular weight of less than 500 Daltons and (iv) the log of the partition coefficient, P (where P = the concentration of the compound in water divided by the concentration of the compound in 1 octanol) is less than 5. The Lipinski Rule of Five is a useful guideline, however, the composition is not limited to these parameters.

127. A wide variety of small molecular weight compounds can be used in the screening methods disclosed herein. Such compounds include, but are not limited to, any compositions which are being tested for drug discovery or development. Such compounds include, but are not limited to, nucleic acids including functional nucleic acids, amino acids including peptides and proteins and fragments thereof, and various other chemical compounds. Compounds can be aqueous- or lipid-soluble. Compounds can be delivered individually to invertebrate animal embryos or may be delivered to as one of a plurality of different chemical compounds. Compounds can be dissolved or suspended within solution, or affixed to a solid-support. Solid supports may include, but are not limited to, insoluble polymer beads or a polymeric matrix coated with one or a plurality of individual compounds, or with combinatorial chemistries. Dosages and volumes which are administered to the Drosophila embryo can be varied so as to optimize dosages for further studies or to rank compounds as to their toxicity and/or potency. Information resulting from variations in conditions can be used to prioritize chemicals for further study, to delineate the relative toxicities of structurally related chemicals, and/or to identify the proper dose range for subsequent toxicity studies (see e.g., Harris, et al., Fundam. Appl. Toxicol. 19:186-196).

128. The carbon-carbon backbone of the compounds can be saturated or unsaturated, cyclic or linear. These aforementioned compounds include, but are not limited to, carbohydrates, polyalcohols (e.g., ethylene glycol and glycerol) and polyphenols (e.g., hydroquinones and tetracylines). Carbohydrate- and polysaccharide-transformed compounds are defined herein so as to include all chemical moieties possessing a saccharide unit or which are transformed from a saccharide. These compounds can also include glycopeptides, glycolipids and other biopolymers (or biomacromolecules) containing saccharides, either in their entirety or as part of the molecular framework. The term carbohydrates merely represent a portion of a much larger family of polyhydroxylated organic compounds. In addition, carbohydrated/polyhydroxylated organic compounds include, but are not limited to: monomeric acyclic compounds (e.g., ethylene glycol, glycerol and 1,2,3-trihydroxy pentane); polymeric acyclic compounds (e.g., di- or tri-ethylene diglycol; monomeric cyclic compounds (e.g., inositol and 1,2,3-trihydroxycyclopentane); polymeric cyclic compounds (e.g., di-inositol); polymeric and monomeric unsaturated compounds (e.g., tetrahydroxy-1,4-quinone) and polyphenols (e.g., tetracyclines) and derivatives, analogs and fragments thereof

129. With respect to the generation of small molecular weight compound libraries, the combination of biochemical diversity is often synergistic with the metabolic diversity obtained from the *in vivo* production of "natural products". Collections of starting compounds, for example peptides, can be administered to cultures of microorganisms. In accord, each microbial strain may potentially create numerous modified peptides or peptide byproducts, thus generating a "metabolite library". Because each of these aforementioned cultures can contain a very complex mixture of metabolites, a highly efficacious method of screening is required (i.e., HTS). An aliquot of the library is incubated with each of the many strains typical of a microorganism fermentation screening program, and the media screened utilizing an HTS-based assay. Furthermore, natural product diversity can be screened by creating a mixture of combinatorially-tagged liposomes; wherein each liposome preferably encapsulates only one member or a simple mixture of a natural product compound library. The libraries which are generated by the methodologies disclosed herein may be screened for any biological activity known within the art. These include, but are not limited to: antimicrobial activity, anti-tumor activity, enzyme inhibiting activity, receptor binding, growth promotion activity, and *in vitro* and *in vivo* tests for biological responses. Compounds may be based on naturally occurring extracellular or intracellular signaling molecules or their derivatives or the like (see, e.g., Alberts, et al., 1989. "Chapter 12: Cell Signaling." 2nd Edition. Garland Publishing, Inc., New York, N.Y., pp. 681-726).

### 2. General Composition parameters or characteristics

### a) Nucleic acids

130. There are a variety of molecules disclosed herein that are nucleic acid based, including for example the neurodegenerative transgenes disclosed herein, as well as those nucleic acids that can be used in the screening methods disclosed herein. The disclosed nucleic acids are made up of for example, nucleotides, nucleotide analogs, or nucleotide substitutes. Non-limiting examples of these and other molecules are discussed herein. It is understood that for example, when a vector is expressed in a cell, that the expressed mRNA will typically be made up of A, C, G, and U. Likewise, it is understood that if, for example, an antisense molecule is introduced into a cell or cell environment through for example exogenous delivery, it is advantagous that the antisense molecule be made up of nucleotide analogs that reduce the degradation of the antisense molecule in the cellular environment.

### (1) Nucleotides and related molecules

131. A nucleotide is a molecule that contains a base moiety, a sugar moiety and a phosphate moiety. Nucleotides can be linked together through their phosphate moieties and sugar moieties creating an internucleoside linkage. The base moiety of a nucleotide can be adenin-9-yl (A), cytosin-1-yl (C), guanin-9-yl (G), uracil-1-yl (U), and thymin-1-yl (T). The sugar moiety of a nucleotide is a ribose or a deoxyribose. The phosphate moiety of a nucleotide is pentavalent phosphate. A non-limiting example of a nucleotide would be 3'-AMP (3'-adenosine monophosphate) or 5'-GMP (5'-guanosine monophosphate).

132. A nucleotide analog is a nucleotide which contains some type of modification to either the base, sugar, or phosphate moieties. Modifications to nucleotides are well known in the art and would include for example, 5-methylcytosine (5-me-C), 5-hydroxymethyl cytosine, xanthine, hypoxanthine, and 2-aminoadenine as well as modifications at the sugar or phosphate moieties.

133. Nucleotide substitutes are molecules having similar functional properties to nucleotides, but which do not contain a phosphate moiety, such as peptide nucleic acid (PNA). Nucleotide substitutes are molecules that will recognize nucleic acids in a Watson-Crick or Hoogsteen manner, but which are linked together through a moiety other than a phosphate moiety. Nucleotide substitutes are able to conform to a double helix type structure when interacting with the appropriate target nucleic acid.

134. It is also possible to link other types of molecules (conjugates) to nucleotides or nucleotide analogs to enhance for example, cellular uptake. Conjugates can be chemically linked to the nucleotide or nucleotide analogs. Such conjugates include but are not limited to lipid moieties such as a cholesterol moiety. (Letsinger et al., Proc. Natl. Acad. Sci. USA, 1989,86, 6553-6556),

135. A Watson-Crick interaction is at least one interaction with the Watson-Crick face of a nucleotide, nucleotide analog, or nucleotide substitute. The Watson-Crick face of a nucleotide, nucleotide analog, or nucleotide substitute includes the C2, N1, and C6 positions of a purine based nucleotide, nucleotide analog, or nucleotide substitute and the C2, N3, C4 positions of a pyrimidine based nucleotide, nucleotide analog, or nucleotide substitute.

136. A Hoogsteen interaction is the interaction that takes place on the Hoogsteen face of a nucleotide or nucleotide analog, which is exposed in the major groove of duplex DNA. The Hoogsteen face includes the N7 position and reactive groups (NH2 or O) at the C6 position of purine nucleotides.

### (2) Sequences

137. There are a variety of sequences related to, for example, Abeta₁₋₄₀ and Abeta₁₋₄₂ as well as any other protein disclosed herein that are disclosed on Genbank, and these sequences and others are herein incorporated by reference in their entireties as well as for individual subsequences contained therein.

138. A variety of sequences are provided herein and these and others can be found in Genbank, at www.pubmed.gov. Those of skill in the art understand how to resolve sequence discrepancies and differences and to adjust the compositions and methods relating to a particular sequence to other related sequences. Primers and/or probes can be designed for any sequence given the information disclosed herein and known in the art.

### (3) Primers and probes

139. Disclosed are compositions including primers and probes, which are capable of interacting with the genes disclosed herein. In certain embodiments the primers are used to support DNA amplification reactions. Typically the primers will be capable of being extended in a sequence specific manner. Extension of a primer in a sequence specific manner includes any methods wherein the sequence and/or composition of the nucleic acid molecule to which the primer is hybridized or otherwise associated directs or influences the composition or sequence of the product produced by the extension of the primer. Extension of the primer in a sequence specific manner therefore includes, but is not limited to, PCR, DNA sequencing, DNA extension, DNA polymerization, RNA transcription, or reverse transcription. Techniques and conditions that amplify the primer in a sequence specific manner are preferred. In certain embodiments the primers are used for the DNA amplification reactions, such as PCR or direct sequencing. It is understood that in certain embodiments the primers can also be extended using non-enzymatic techniques, where for example, the nucleotides or oligonucleotides used to extend the primer are modified such that they will chemically react to extend the primer in a sequence specific manner. Typically the disclosed primers hybridize with the nucleic acid or region of the nucleic acid or they hybridize with the complement of the nucleic acid or complement of a region of the nucleic acid.

### (4) Homology/identity

140. It is understood that one way to define any known variants and derivatives or those that might arise, of the disclosed genes and proteins herein is through defining the variants and derivatives in terms of homology to specific known sequences. For example SEQ ID NO: 7 sets forth a particular sequence of a membrane glycosylphosphatidylinositol-anchored glycoprotein, and SEQ ID NO: see note above sets forth a particular sequence of the protein encoded by SEQ ID NO: 8, an Amyloid A protein. Specifically disclosed are variants of these and other genes and proteins herein disclosed which have at least, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 percent homology to the stated sequence. Those of skill in the art readily understand how to determine the homology of two proteins or nucleic acids, such as genes. For example, the homology can be calculated after aligning the two sequences so that the homology is at its highest level.

141. Another way of calculating homology can be performed by published algorithms. Optimal alignment of sequences for comparison may be conducted by the local homology algorithm of Smith and Waterman Adv. Appl. Math. 2: 482 (1981), by the homology alignment algorithm of Needleman and Wunsch, J. MoL Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

142. The same types of homology can be obtained for nucleic acids by for example the algorithms disclosed in Zuker, M. Science 244:48-52, 1989, Jaeger et al. Proc. Natl. Acad. Sci. USA 86:7706-7710, 1989, Jaeger et al. Methods Enzymol. 183:281-306, 1989 which are herein incorporated by reference for at least material related to nucleic acid alignment.

### b) Peptides

### (1) Protein variants

143. As discussed herein there are numerous variants of amyloidgenic peptides, for example, that are known and herein contemplated. In addition to the known functional variants herein described, for example those related to the Amyloid Precursor Protein such as APPL, CT100, β-CTF, α-CTF, γ-CTF, Abeta₁₋₄₀, Abeta₁₋₄₂, Abetass₁₋₄₀, and Abetass₁₋₄₂, there are derivatives of APP which also function in the disclosed methods and compositions. Protein variants and derivatives are well understood to those of skill in the art and in can involve amino acid sequence modifications. For example, amino acid sequence modifications typically fall into one or more of three classes: substitutional, insertional or deletional variants. Insertions include amino and/or carboxyl terminal fusions as well as intrasequence insertions of single or multiple amino acid residues. Insertions ordinarily will be smaller insertions than those of amino or carboxyl terminal fusions, for example, on the order of one to four residues. Immunogenic fusion protein derivatives, such as those described in the examples, are made by fusing a polypeptide sufficiently large to confer immunogenicity to the target sequence by cross-linking in vitro or by recombinant cell culture transformed with DNA encoding the fusion. Deletions are characterized by the removal of one or more amino acid residues from the protein sequence. Typically, no more than about from 2 to 6 residues are deleted at any one site within the protein molecule. These variants ordinarily are prepared by site specific mutagenesis of nucleotides in the DNA encoding the protein, thereby producing DNA encoding the variant, and thereafter expressing the DNA in recombinant cell culture. Techniques for making substitution mutations at predetermined sites in DNA having a known sequence are well known, for example M13 primer mutagenesis and PCR mutagenesis. Amino acid substitutions are typically of single residues, but can occur at a number of different locations at once; insertions usually will be on the order of about from 1 to 10 amino acid residues; and deletions will range about from 1 to 30 residues. Deletions or insertions preferably are made in adjacent pairs, i.e. a deletion of 2 residues or insertion of 2 residues. Substitutions, deletions, insertions or any combination thereof may be combined to arrive at a final construct. The mutations must not place the sequence out of reading frame and preferably will not create complementary regions that could produce secondary mRNA structure. Substitutional variants are those in which at least one residue has been removed and a different residue inserted in its place. Such substitutions generally are made in accordance with the following Tables 3 and 4 and are referred to as conservative substitutions.

144.

**TABLE 3:Amino Acid Abbreviations**

| **Amino Acid** | **Abbreviations** |
|---|---|
| alanine | AlaA |
| allosoleucine | AIle |
| arginine | ArgR |
| asparagine | AsnN |
| aspartic acid | AspD |
| cysteine | CysC |
| glutamic acid | GluE |
| glutamine | GlnK |
| glycine | GlyG |
| histidine | HisH |
| isolelucine | IleI |
| leucine | LeuL |
| lysine | LysK |
| phenylalanine | PheF |
| proline | ProP |
| pyroglutamic acidp | Glu |
| serine | SerS |
| threonine | ThrT |
| tyrosine | TyrY |
| tryptophan | TrpW |
| valine | ValV |

| TABLE 4:Amino Acid Substitutions |
|---|
| Original Residue Exemplary Conservative Substitutions, others are known in the art. |
| Alaser |
| Arglys, gln |
| Asngln; his |
| Aspglu |
| Cysser |
| Glnasn, lys |
| Gluasp |
| Glypro |
| Hisasn;gln |
| Ileleu; val |
| Leuile; val |
| Lysarg; gln; |
| MetLeu; ile |
| Phemet; leu; tyr |
| Serthr |
| Thrser |
| Trptyr |
| Tyrtrp; phe |
| Valile; leu |

145. Substantial changes in function or immunological identity are made by selecting substitutions that are less conservative than those in Table 4, i.e., selecting residues that differ more significantly in their effect on maintaining (a) the structure of the polypeptide backbone in the area of the substitution, for example as a sheet or helical conformation, (b) the charge or hydrophobicity of the molecule at the target site or (c) the bulk of the side chain. The substitutions which in general are expected to produce the greatest changes in the protein properties will be those in which (a) a hydrophilic residue, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine, in this case, (e) by increasing the number of sites for sulfation and/or glycosylation.

146. For example, the replacement of one amino acid residue with another that is biologically and/or chemically similar is known to those skilled in the art as a conservative substitution. For example, a conservative substitution would be replacing one hydrophobic residue for another, or one polar residue for another. The substitutions include combinations such as, for example, Gly, Ala; Val, Ile, Leu; Asp, Glu; Asn, Gln; Ser, Thr; Lys, Arg; and Phe, Tyr. Such conservatively substituted variations of each explicitly disclosed sequence are included within the mosaic polypeptides provided herein.

147. Substitutional or deletional mutagenesis can be employed to insert sites for N-glycosylation (Asn-X-Thr/Ser) or O-glycosylation (Ser or Thr). Deletions of cysteine or other labile residues also may be desirable. Deletions or substitutions of potential proteolysis sites, e.g. Arg, is accomplished for example by deleting one of the basic residues or substituting one by glutaminyl or histidyl residues.

148. Certain post-translational derivatizations are the result of the action of recombinant host cells on the expressed polypeptide. Glutaminyl and asparaginyl residues are frequently post-translationally deamidated to the corresponding glutamyl and asparyl residues. Alternatively, these residues are deamidated under mildly acidic conditions. Other post-translational modifications include hydroxylation of proline and lysine, phosphorylation of hydroxyl groups of seryl or threonyl residues, methylation of the o-amino groups of lysine, arginine, and histidine side chains (T.E. Creighton, Proteins: Structure and Molecular Properties, W. H. Freeman & Co., San Francisco pp 79-86 [1983]), acetylation of the N-terminal amine and, in some instances, amidation of the C-terminal carboxyl.

149. It is understood that one way to define the variants and derivatives of the disclosed proteins herein is through defining the variants and derivatives in terms of homology/identity to specific known sequences. Specifically disclosed are variants of these and other proteins herein disclosed which have at least, 70% or 75% or 80% or 85% or 90% or 95% homology to the stated sequence. Those of skill in the art readily understand how to determine the homology of two proteins. For example, the homology can be calculated after aligning the two sequences so that the homology is at its highest level.

150. Another way of calculating homology can be performed by published algorithms. Optimal alignment of sequences for comparison may be conducted by the local homology algorithm of Smith and Waterman Adv. Appl. Math. 2: 482 (1981), by the homology alignment algorithm of Needleman and Wunsch, J. MoL Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

151. The same types of homology can be obtained for nucleic acids by for example the algorithms disclosed in Zuker, M. Science 244:48-52, 1989, Jaeger et al. Proc. Natl. Acad. Sci. USA 86:7706-7710, 1989, Jaeger et al. Methods Enzymol. 183:281-306, 1989 which are herein incorporated by reference for at least material related to nucleic acid alignment.

152. It is understood that the description of conservative mutations and homology can be combined together in any combination, such as embodiments that have at least 70% homology to a particular sequence wherein the variants are conservative mutations.

153. As this specification discusses various proteins and protein sequences it is understood that the nucleic acids that can encode those protein sequences are also disclosed. This would include all degenerate sequences related to a specific protein sequence, i.e. all nucleic acids having a sequence that encodes one particular protein sequence as well as all nucleic acids, including degenerate nucleic acids, encoding the disclosed variants and derivatives of the protein sequences. Thus, while each particular nucleic acid sequence may not be written out herein, it is understood that each and every sequence is in fact disclosed and described herein through the disclosed protein sequence. It is understood that for a mutation all of the nucleic acid sequences that encode a particular derivative are also disclosed. It is also understood that while no amino acid sequence indicates what particular DNA sequence encodes that protein within an organism, where particular variants of a disclosed protein are disclosed herein, the known nucleic acid sequence that encodes that protein in the particular location from which that protein arises is also known and herein disclosed and described.

154. It is understood that there are numerous amino acid and peptide analogs which can be incorporated into the disclosed compositions. For example, there are numerous D amino acids or amino acids which have a different functional substituent then the amino acids shown in Table 1 and Table 2. The opposite stereo isomers of naturally occurring peptides are disclosed, as well as the stereo isomers of peptide analogs. These amino acids can readily be incorporated into polypeptide chains by charging tRNA molecules with the amino acid of choice and engineering genetic constructs that utilize, for example, amber codons, to insert the analog amino acid into a peptide chain in a site specific way (Thorson et al., Methods in Molec. Biol. 77:43-73 (1991), Zoller, Current Opinion in Biotechnology, 3:348-354 (1992); Ibba, Biotechnology & Genetic Engineering Reviews 13:197-216 (1995), Cahill et al., TIBS, 14(10):400-403 (1989); Benner, TIB Tech, 12:158-163 (1994); Ibba and Hennecke, Bio/technology, 12:678-682 (1994) all of which are herein incorporated by reference at least for material related to amino acid analogs).

155. Molecules can be produced that resemble peptides, but which are not connected via a natural peptide linkage. For example, linkages for amino acids or amino acid analogs can include CH₂NH--, --CH₂S--, --CH₂--CH₂ --, --CH=CH-- (cis and trans),-COCH₂ --, -CH(OH)CH₂-, and --CHH₂SO-(These and others can be found in Spatola, A. F. in Chemistry and Biochemistry of Amino Acids, Peptides, and Proteins, B. Weinstein, eds., Marcel Dekker, New York, p. 267 (1983); Spatola, A. F., Vega Data (March 1983), Vol. 1, Issue 3, Peptide Backbone Modifications (general review); Morley, Trends Pharm Sci (1980) pp. 463-468; Hudson, D. et al., Int J Pept Prot Res 14:177-185 (1979) (--CH₂NH--, CH₂CH₂--); Spatola et al. Life Sci 38:1243-1249 (1986) (-CH H₂--S); Hann J. Chem. Soc Perkin Trans. 1307-314 (1982) (-CH-CH-, cis and trans); Almquist et al. J. Med. Chem. 23:1392-1398 (1980) (--COCH₂--); Jennings-White et al. Tetrahedron Lett 23:2533 (1982) (--COCH₂--); Szelke et al. European Appln, EP 45665 CA (1982): 97:39405 (1982) (-CH(OH)CH₂--); Holladay et al. Tetrahedron. Lett 24:4401-4404 (1983) (--C(OH)CH₂--); and Hruby Life Sci 31:189-199 (1982) (--CH₂--S--); each of which is incorporated herein by reference. A particularly preferred non-peptide linkage is --CH₂NH--. It is understood that peptide analogs can have more than one atom between the bond atoms, such as b-alanine, g-aminobutyric acid, and the like.

156. Amino acid analogs and analogs and peptide analogs often have enhanced or desirable properties, such as, more economical production, greater chemical stability, enhanced pharmacological properties (half-life, absorption, potency, efficacy, etc.), altered specificity (e.g., a broad-spectrum of biological activities), reduced antigenicity, and others.

157. D-amino acids can be used to generate more stable peptides, because D amino acids are not recognized by peptidases and such. Systematic substitution of one or more amino acids of a consensus sequence with a D-amino acid of the same type (e.g., D-lysine in place of L-lysine) can be used to generate more stable peptides. Cysteine residues can be used to cyclize or attach two or more peptides together. This can be beneficial to constrain peptides into particular conformations. (Rizo and Gierasch Ann. Rev. Biochem. 61:387 (1992), incorporated herein by reference).

### c) Antibodies

### (1) Antibodies Generally

158. The term "antibodies" is used herein in a broad sense and includes both polyclonal and monoclonal antibodies. In addition to intact immunoglobulin molecules, also included in the term "antibodies" are fragments or polymers of those immunoglobulin molecules, and human or humanized versions of immunoglobulin molecules or fragments thereof, as long as they are chosen for their ability to interact with an invertebrate embryo in the screening methods disclosed herein.

159. The term "monoclonal antibody" as used herein refers to an antibody obtained from a substantially homogeneous population of antibodies, i.e., the individual antibodies within the population are identical except for possible naturally occurring mutations that may be present in a small subset of the antibody molecules. The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, as long as they exhibit the desired antagonistic activity (See, U.S. Pat. No. 4,816,567 and Morrison et al., Proc. Natl. Acad. Sci. USA, 81:6851-6855 (1984)).

160. The disclosed monoclonal antibodies can be made using any procedure which produces monoclonal antibodies. For example, disclosed monoclonal antibodies can be prepared using hybridoma methods, such as those described by Kohler and Milstein, Nature, 256:495 (1975). In a hybridoma method, a mouse or other appropriate host animal is typically immunized with an immunizing agent to elicit lymphocytes that produce or are capable of producing antibodies that will specifically bind to the immunizing agent. Alternatively, the lymphocytes may be immunized *in vitro,* e.g., using the HIV Env-CD4-co-receptor complexes described herein.

161. The monoclonal antibodies may also be made by recombinant DNA methods, such as those described in U.S. Pat. No. 4,816,567 (Cabilly et al.). DNA encoding the disclosed monoclonal antibodies can be readily isolated and sequenced using conventional procedures (e.g., by using oligonucleotide probes that are capable of binding specifically to genes encoding the heavy and light chains of murine antibodies). Libraries of antibodies or active antibody fragments can also be generated and screened using phage display techniques, e.g., as described in U.S. Patent No. 5,804,440 to Burton et al. and U.S. Patent No. 6,096,441 to Barbas et al.

162. *In vitro* methods are also suitable for preparing monovalent antibodies. Digestion of antibodies to produce fragments thereof, particularly, Fab fragments, can be accomplished using routine techniques known in the art. For instance, digestion can be performed using papain. Examples of papain digestion are described in WO 94/29348 published Dec. 22, 1994 and U.S. Pat. No. 4,342,566. Papain digestion of antibodies typically produces two identical antigen binding fragments, called Fab fragments, each with a single antigen binding site, and a residual Fc fragment. Pepsin treatment yields a fragment that has two antigen combining sites and is still capable of cross-linking antigen.

163. The fragments, whether attached to other sequences or not, can also include insertions, deletions, substitutions, or other selected modifications of particular regions or specific amino acids residues, provided the activity of the antibody or antibody fragment is not significantly altered or impaired compared to the non-modified antibody or antibody fragment. These modifications can provide for some additional property, such as to remove/add amino acids capable of disulfide bonding, to increase its bio-longevity, to alter its secretory characteristics, etc. In any case, the antibody or antibody fragment must possess a bioactive property, such as specific binding to its cognate antigen. Functional or active regions of the antibody or antibody fragment may be identified by mutagenesis of a specific region of the protein, followed by expression and testing of the expressed polypeptide. Such methods are readily apparent to a skilled practitioner in the art and can include site-specific mutagenesis of the nucleic acid encoding the antibody or antibody fragment. (Zoller, M.J. Curr. Opin. Biotechnol. 3:348-354, 1992).

164. As used herein, the term "antibody" or "antibodies" can also refer to a human antibody and/or a humanized antibody. Many non-human antibodies (e.g., those derived from mice, rats, or rabbits) are naturally antigenic in humans, and thus can give rise to undesirable immune responses when administered to humans. Therefore, the use of human or humanized antibodies in the methods serves to lessen the chance that an antibody administered to a human will evoke an undesirable immune response.

### (2) Humanized antibodies

165. Antibody humanization techniques generally involve the use of recombinant DNA technology to manipulate the DNA sequence encoding one or more polypeptide chains of an antibody molecule. Accordingly, a humanized form of a non-human antibody (or a fragment thereof) is a chimeric antibody or antibody chain (or a fragment thereof, such as an Fv, Fab, Fab', or other antigen-binding portion of an antibody) which contains a portion of an antigen binding site from a non-human (donor) antibody integrated into the framework of a human (recipient) antibody.

166. To generate a humanized antibody, residues from one or more complementarity determining regions (CDRs) of a recipient (human) antibody molecule are replaced by residues from one or more CDRs of a donor (non-human) antibody molecule that is known to have desired antigen binding characteristics (e.g., a certain level of specificity and affinity for the target antigen). In some instances, Fv framework (FR) residues of the human antibody are replaced by corresponding non-human residues. Humanized antibodies may also contain residues which are found neither in the recipient antibody nor in the imported CDR or framework sequences. Generally, a humanized antibody has one or more amino acid residues introduced into it from a source which is non-human. In practice, humanized antibodies are typically human antibodies in which some CDR residues and possibly some FR residues are substituted by residues from analogous sites in rodent antibodies. Humanized antibodies generally contain at least a portion of an antibody constant region (Fc), typically that of a human antibody (Jones et al., Nature, 321:522-525 (1986), Reichmann et al., Nature, 332:323-327 (1988), and Presta, Curr. Opin. Struct. Biol., 2:593-596 (1992)).

167. Methods for humanizing non-human antibodies are well known in the art. For example, humanized antibodies can be generated according to the methods of Winter and co-workers (Jones et al., Nature, 321:522-525 (1986), Riechmann et al., Nature, 332:323-327 (1988), Verhoeyen et al., Science, 239:1534-1536 (1988)), by substituting rodent CDRs or CDR sequences for the corresponding sequences of a human antibody. Methods that can be used to produce humanized antibodies are also described in U.S. Patent No. 4,816,567 (Cabilly et al.), U.S. Patent No. 5,565,332 (Hoogenboom et al.), U.S. Patent No. 5,721,367 (Kay et al.), U.S. Patent No. 5,837,243 (Deo et al.), U.S. Patent No. 5, 939,598 (Kucherlapati et al.), U.S. Patent No. 6,130,364 (Jakobovits et al.), and U.S. Patent No. 6,180,377 (Morgan et al.).

### d) Pharmaceutical carriers

168. The compositions can also be used in the screening methods disclosed herein in conjunction with a pharmaceutically acceptable carrier. Alternatively, pharmaceutical carriers themselves can be tested by the screening methods disclosed herein. By "pharmaceutically acceptable" is meant a material that is not biologically or otherwise undesirable, i.e., the material may be administered to a subject, along with a nucleic acid or vector, without causing any undesirable biological effects or interacting in a deleterious manner with any of the other components of the pharmaceutical composition in which it is contained. The carrier would naturally be selected to minimize any degradation of the active ingredient as would be well known to one of skill in the art. The compositions may, for example, be in solution or suspension (i.e. incorporated into microparticles or liposomes).

169. Suitable carriers and their formulations are described in Remington: The Science and Practice of Pharmacy (19th ed.) ed. A.R. Gennaro, Mack Publishing Company, Easton, PA 1995. For example, an appropriate amount of a pharmaceutically-acceptable salt can be used in the formulation to render the formulation isotonic, and its effect on the invertebrate embryos can also be measured using the screening methods disclosed herein. Examples of the pharmaceutically-acceptable carrier include, but are not limited to, saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of composition being administered.

170. Pharmaceutical carriers are known to those skilled in the art. These most typically would be standard carriers for administration of drugs to humans, including solutions such as sterile water, saline, and buffered solutions at physiological pH. Pharmaceutical compositions may include carriers, thickeners, diluents, buffers, preservatives, surface active agents and the like in addition to the molecule of choice. Pharmaceutical compositions may also include one or more active ingredients such as antimicrobial agents, antiinflammatory agents, anesthetics, and the like.

### 3. Compositions identified by screening and combinatorial methods

171. Disclosed are the compositions that are identified through combinatorial techniques or screening techniques, as disclosed herein, in which the compositions are used in a combinatorial or screening protocol. It is understood that when using the disclosed compositions in combinatorial techniques or screening methods, molecules, such as macromolecular molecules, will be identified that have particular desired properties such as inhibition or stimulation or the transgenic embryo. Thus, the products identified using the combinatorial or screening approaches that involve the disclosed compositions are also considered herein disclosed.

172. It is understood that the disclosed methods for identifying molecules that have a desired effect upon the invertebrate embryo can be performed using high through put means disclosed herein.

173. There are a number of methods for isolating proteins which either have de *novo* activity or a modified activity. For example, phage display libraries have been used to isolate numerous peptides that interact with a specific target. (See for example, United States Patent No. 6,031,071; 5,824,520; 5,596,079; and 5,565,332 which are herein incorporated by reference). These peptides can be used in the invertebrate embryo screening methods disclosed herein.

174. A preferred method for isolating proteins that have a given function, which can be used in the screening methods disclosed herein, is is described by Roberts and Szostak, 1997. This combinatorial chemistry method couples the functional power of proteins and the genetic power of nucleic acids. An RNA molecule is generated in which a puromycin molecule is covalently attached to the 3'-end of the RNA molecule. An *in vitro* translation of this modified RNA molecule causes the correct protein, encoded by the RNA to be translated. In addition, because of the attachment of the puromycin, a peptdyl acceptor which cannot be extended, the growing peptide chain is attached to the puromycin which is attached to the RNA. Thus, the protein molecule is attached to the genetic material that encodes it. Normal *in vitro* selection procedures can now be done to isolate functional peptides. Once the selection procedure for peptide function is complete traditional nucleic acid manipulation procedures are performed to amplify the nucleic acid that codes for the selected functional peptides. After amplification of the genetic material, new RNA is transcribed with puromycin at the 3'-end, new peptide is translated and another functional round of selection is performed. Thus, protein selection can be performed in an iterative manner just like nucleic acid selection techniques. The peptide which is translated is controlled by the sequence of the RNA attached to the puromycin. This sequence can be anything from a random sequence engineered for optimum translation (i.e. no stop codons etc.) or it can be a degenerate sequence of a known RNA molecule to look for improved or altered function of a known peptide. The conditions for nucleic acid amplification and *in vitro* translation are well known to those of ordinary skill in the art and are preferably performed as in Roberts and Szostak (Roberts R.W. and Szostak J.W. Proc. Natl. Acad. Sci. USA, 94(23)12997-302 (1997)). These compositions can then be used in the screening methods disclosed herein.

175. Another preferred method for combinatorial methods designed to isolate peptides for use in the screening methods herein are described in Cohen et al. (Cohen B.A.,et al., Proc. Natl. Acad. Sci. USA 95(24):14272-7 (1998)). This method utilizes and modifies two-hybrid technology. Yeast two-hybrid systems are useful for the detection and analysis of protein:protein interactions. The two-hybrid system, initially described in the yeast *Saccharomyces cerevisiae,* is a powerful molecular genetic technique for identifying new regulatory molecules, specific to the protein of interest (Fields and Song, Nature 340:245-6 (1989)). Cohen et al., modified this technology so that novel interactions between synthetic or engineered peptide sequences could be identified which bind a molecule of choice. The benefit of this type of technology is that the selection is done in an intracellular environment. The method utilizes a library of peptide molecules that attached to an acidic activation domain. A peptide of choice, for example a neurodegenerative associated peptide, is attached to a DNA binding domain of a transcriptional activation protein, such as Gal4. By performing the two-hybrid technique on this type of system, molecules associated with neurodegenerative diseases, for example, can be identified.

176. Using methodology well known to those of skill in the art, in combination with various combinatorial libraries, one can isolate and characterize those small molecules or macromolecules, which bind to or interact with the desired target. The relative binding affinity of these compounds can be compared and optimum compounds identified using competitive binding studies, which are well known to those of skill in the art.

177. Techniques for making combinatorial libraries and screening combinatorial libraries to isolate molecules which are well known to those of skill in the art. These combinatorial libraries can then be used with the invertebrate embryo screening methods disclosed herein. Representative techniques and methods can be found in but are not limited to United States patents 5,084,824, 5,288,514, 5,449,754, 5,506,337, 5,539,083, 5,545,568, 5,556,762, 5,565,324, 5,565,332, 5,573,905, 5,618,825, 5,619,680, 5,627,210, 5,646,285, 5,663,046, 5,670,326, 5,677,195, 5,683,899, 5,688,696, 5,688,997, 5,698,685, 5,712,146, 5,721,099, 5,723,598, 5,741,713, 5,792,431, 5,807,683, 5,807,754, 5,821,130, 5,831,014, 5,834,195, 5,834,318, 5,834,588, 5,840,500, 5,847,150, 5,856,107, 5,856,496, 5,859,190, 5,864,010, 5,874,443, 5,877,214, 5,880,972, 5,886,126, 5,886,127, 5,891,737, 5,916,899, 5,919,955, 5,925,527, 5,939,268, 5,942,387, 5,945,070, 5,948,696, 5,958,702, 5,958,792, 5,962,337, 5,965,719, 5,972,719, 5,976,894, 5,980,704, 5,985,356, 5,999,086, 6,001,579, 6,004,617, 6,008,321, 6,017,768, 6,025,371, 6,030,917, 6,040,193, 6,045,671, 6,045,755, 6,060,596, and 6,061,636.

178. Combinatorial libraries can be made from a wide array of molecules using a number of different synthetic techniques. For example, libraries containing fused 2,4-pyrimidinediones (United States patent 6,025,371) dihydrobenzopyrans (United States Patent 6,017,768and 5,821,130), amide alcohols (United States Patent 5,976,894), hydroxy-amino acid amides (United States Patent 5,972,719) carbohydrates (United States patent 5,965,719), 1,4-benzodiazepin-2,5-diones (United States patent 5,962,337), cyclics (United States patent 5,958,792), biaryl amino acid amides (United States patent 5,948,696), thiophenes (United States patent 5,942,387), tricyclic Tetrahydroquinolines (United States patent 5,925,527), benzofurans (United States patent 5,919,955), isoquinolines (United States patent 5,916,899), hydantoin and thiohydantoin (United States patent 5,859,190), indoles (United States patent 5,856,496), imidazol-pyrido-indole and imidazol-pyrido-benzothiophenes (United States patent 5,856,107) substituted 2-methylene-2, 3-dihydrothiazoles (United States patent 5,847,150), quinolines (United States patent 5,840,500), PNA (United States patent 5,831,014), containing tags (United States patent 5,721,099), polyketides (United States patent 5,712,146), morpholino-subunits (United States patent 5,698,685 and 5,506,337), sulfamides (United States patent 5,618,825), and benzodiazepines (United States patent 5,288,514).

### 4. Computer assisted drug design

179. The compositions for use with the screening methods described herein can be used as targets for any molecular modeling technique to identify either the structure of another composition or to identify potential or actual molecules, such as small molecules, which interact in a desired way with the invertebrate embryos disclosed herein. The nucleic acids, peptides, and related molecules disclosed herein can be used as targets in any molecular modeling program or approach.

180. After a composition has been found to interact in a desired way with an invertebrate embryo, the composition can be used in modeling techniques. Molecules, such as macromolecular molecules, will be identified that have particular desired properties such as inhibition or stimulation or the target molecule's function. The molecules identified and isolated when using the disclosed methods are also disclosed. Thus, the products produced using the molecular modeling approaches that involve the disclosed screening methods, are also considered herein disclosed.

181. Thus, one way to isolate molecules that bind a molecule of choice is through rational design. This is achieved through structural information and computer modeling. Computer modeling technology allows visualization of the three-dimensional atomic structure of a selected molecule and the rational design of new compounds that will interact with the molecule. The three-dimensional construct typically depends on data from x-ray crystallographic analyses or NMR imaging of the selected molecule. The molecular dynamics require force field data. The computer graphics systems enable prediction of how a new compound will link to the target molecule and allow experimental manipulation of the structures of the compound and target molecule to perfect binding specificity. Prediction of what the molecule-compound interaction will be when small changes are made in one or both requires molecular mechanics software and computationally intensive computers, usually coupled with user-friendly, menu-driven interfaces between the molecular design program and the user.

182. Examples of molecular modeling systems are the CHARMm and QUANTA programs, Polygen Corporation, Waltham, MA. CHARMm performs the energy minimization and molecular dynamics functions. QUANTA performs the construction, graphic modeling and analysis of molecular structure. QUANTA allows interactive construction, modification, visualization, and analysis of the behavior of molecules with each other.

183. A number of articles review computer modeling of drugs interactive with specific proteins, such as Rotivinen, et al., 1988 Acta Pharmaceutica Fennica 97, 159-166; Ripka, New Scientist 54-57 (June 16, 1988); McKinaly and Rossmann, 1989 Annu. Rev. Pharmacol. Toxiciol. 29, 111-122; Perry and Davies, QSAR: Quantitative Structure-Activity Relationships in Drug Design pp. 189-193 (Alan R. Liss, Inc. 1989); Lewis and Dean, 1989 Proc. R. Soc. Lond. 236, 125-140 and 141-162; and, with respect to a model enzyme for nucleic acid components, Askew, et al., 1989 J. Am. Chem. Soc. 111, 1082-1090. Other computer programs that screen and graphically depict chemicals are available from companies such as BioDesign, Inc., Pasadena, CA., Allelix, Inc, Mississauga, Ontario, Canada, and Hypercube, Inc., Cambridge, Ontario. Although these are primarily designed for application to drugs specific to particular proteins, they can be adapted to design of molecules specifically interacting with specific regions of DNA or RNA, once that region is identified.

184. Although described above with reference to design and generation of compounds which could alter binding, one could also screen libraries of known compounds, including natural products or synthetic chemicals, and biologically active materials, including proteins, for compounds which alter substrate binding or enzymatic activity.

### 5. Computer readable mediums

185. It is understood that the disclosed nucleic acids and proteins can be represented as a sequence consisting of the nucleotides of amino acids. There are a variety of ways to display these sequences, for example the nucleotide guanosine can be represented by G or g. Likewise the amino acid valine can be represented by Val or V. Those of skill in the art understand how to display and express any nucleic acid or protein sequence in any of the variety of ways that exist, each of which is considered herein disclosed. Specifically contemplated herein is the display of these sequences on computer readable mediums, such as, commercially available floppy disks, tapes, chips, hard drives, compact disks, and video disks, or other computer readable mediums. Also disclosed are the binary code representations of the disclosed sequences. Those of skill in the art understand what computer readable mediums. Thus, computer readable mediums on which the nucleic acids or protein sequences are recorded, stored, or saved.

186. Disclosed are computer readable mediums comprising the sequences and information regarding the sequences set forth herein. Also disclosed are computer readable nediums comprising the sequences and information regarding the sequences set forth herein.

### 6. Kits

187. Disclosed herein are kits that are drawn to reagents that can be used in practicing the methods disclosed herein. The kits can include any reagent or combination of reagent iscussed herein or that would be understood to be required or beneficial in the practice of the isclosed methods. For example, the kits could include transgenic invertebrate animal embryos nd a suitable container. The kit can also include combinatorial libraries of small molecules. The it can also include an embryo sorter. The kit can also include well plates for screening molecules.

### 7. Methods of making the compositions

188. The compositions disclosed herein and the compositions necessary to perform the disclosed methods can be made using any method known to those of skill in the art for that particular reagent or compound unless otherwise specifically noted.

### a) Nucleic acid synthesis

189. For example, the nucleic acids can be made using standard chemical synthesis methods or can be produced using enzymatic methods or any other known method. Such methods can range from standard enzymatic digestion followed by nucleotide fragment isolation (see for example, Sambrook et al., Molecular Cloning: A Laboratory Manual, 2nd Edition (Cold Spring Harbor Laboratory Press, Cold Spring Harbor, N.Y., 1989) Chapters 5, 6) to purely synthetic methods, for example, by the cyanoethyl phosphoramidite method using a Milligen or Beckman System 1Plus DNA synthesizer (for example, Model 8700 automated synthesizer of Milligen-Biosearch, Burlington, MA or ABI Model 380B). Synthetic methods useful for making oligonucleotides are also described by Ikuta et al., Ann. Rev. Biochem. 53:323-356 (1984), (phosphotriester and phosphite-triester methods), and Narang et al., Methods Enzymol., 65:610-620 (1980), (phosphotriester method). Protein nucleic acid molecules can be made using known methods such as those described by Nielsen et al., Bioconjug. Chem. 5:3-7 (1994).

### b) Peptide synthesis

190. One method of producing proteins is to link two or more peptides or polypeptides together by protein chemistry techniques. For example, peptides or polypeptides can be chemically synthesized using currently available laboratory equipment using either Fmoc (9-fluorenylmethyloxycarbonyl) or Boc (*tert* -butyloxycarbonoyl) chemistry. (Applied Biosystems, Inc., Foster City, CA). One skilled in the art can readily appreciate that a peptide or polypeptide corresponding to the disclosed proteins, for example, can be synthesized by standard chemical reactions. For example, a peptide or polypeptide can be synthesized and not cleaved from its synthesis resin whereas the other fragment of a peptide or protein can be synthesized and subsequently cleaved from the resin, thereby exposing a terminal group which is functionally blocked on the other fragment. By peptide condensation reactions, these two fragments can be covalently joined via a peptide bond at their carboxyl and amino termini, respectively, to form an antibody, or fragment thereof. (Grant GA (1992) Synthetic Peptides: A User Guide. W.H. Freeman and Co., N.Y. (1992); Bodansky M and Trost B., Ed. (1993) Principles of Peptide Synthesis. Springer-Verlag Inc., NY (which is herein incorporated by reference at least for material related to peptide synthesis). Alternatively, the peptide or polypeptide is independently synthesized *in vivo* as described herein. Once isolated, these independent peptides or polypeptides may be linked to form a peptide or fragment thereof via similar peptide condensation reactions.

191. For example, enzymatic ligation of cloned or synthetic peptide segments allow relatively short peptide fragments to be joined to produce larger peptide fragments, polypeptides or whole protein domains (Abrahmsen L et al., Biochemistry, 30:4151 (1991)). Alternatively, native chemical ligation of synthetic peptides can be utilized to synthetically construct large peptides or polypeptides from shorter peptide fragments. This method consists of a two step chemical reaction (Dawson et al. Synthesis of Proteins by Native Chemical Ligation. Science, 266:776-779 (1994)). The first step is the chemoselective reaction of an unprotected synthetic peptide--thioester with another unprotected peptide segment containing an amino-terminal Cys residue to give a thioester-linked intermediate as the initial covalent product. Without a change in the reaction conditions, this intermediate undergoes spontaneous, rapid intramolecular reaction to form a native peptide bond at the ligation site (Baggiolini M et al. (1992) FEBS Lett. 307:97-101; Clark-Lewis I et al., J.Biol.Chem., 269:16075 (1994); Clark-Lewis I et al., Biochemistry, 30:3128 (1991); Rajarathnam K et al., Biochemistry 33:6623-30 (1994)).

192. Alternatively, unprotected peptide segments are chemically linked where the bond formed between the peptide segments as a result of the chemical ligation is an unnatural (non-peptide) bond (Schnolzer, M et al. Science, 256:221 (1992)). This technique has been used to synthesize analogs of protein domains as well as large amounts of relatively pure proteins with full biological activity (deLisle Milton RC et al., Techniques in Protein Chemistry IV. Academic Press, New York, pp. 257-267 (1992)).

### c) Processes for making the compositions

193. Disclosed are processes for making the compositions as well as making the intermediates leading to the compositions. There are a variety of methods that can be used for making these compositions, such as synthetic chemical methods and standard molecular biology methods. It is understood that the methods of making these and the other disclosed compositions are specifically disclosed.

194. Disclosed are nucleic acid molecules produced by the process comprising linking in an operative way a nucleic acid comprising the sequence set forth in SEQ ID NO: 1 or 2 for example, and a sequence controlling the expression of the nucleic acid. Such sequences controlling the expression of the nucleic acid are disclosed herein.

195. Also disclosed are nucleic acid molecules produced by the process comprising linking in an operative way a nucleic acid molecule comprising a sequence having 80% identity to a sequence set forth in, for example, SEQ ID NO: 1 or 2, and a sequence controlling the expression of the nucleic acid.

196. Disclosed are nucleic acid molecules produced by the process comprising linking in an operative way a nucleic acid molecule comprising a sequence that hybridizes under stringent hybridization conditions to a sequence set forth in SEQ ID NO: 1 or 2 and a sequence controlling the expression of the nucleic acid.

197. Disclosed are nucleic acid molecules produced by the process comprising linking in an operative way a nucleic acid molecule comprising a sequence encoding a peptide set forth in SEQ ID NO: 7 or 8 and a sequence controlling an expression of the nucleic acid molecule.

198. Disclosed are nucleic acid molecules produced by the process comprising linking in an operative way a nucleic acid molecule comprising a sequence encoding a peptide having 80% identity to a peptide set forth in SEQ ID NO: 7 or 8 and a sequence controlling an expression of the nucleic acid molecule.

199. Disclosed are nucleic acids produced by the process comprising linking in an operative way a nucleic acid molecule comprising a sequence encoding a peptide having 80% identity to a peptide set forth in SEQ ID NO: 7 or 8, wherein any change from the sequence are conservative changes and a sequence controlling an expression of the nucleic acid molecule.

200. Disclosed are cells, including invertebrate embryos, produced by the process of transforming the cell with any of the disclosed nucleic acids. Disclosed are cells produced by the process of transforming the cell with any of the non-naturally occurring disclosed nucleic acids.

201. Disclosed are any of the disclosed peptides produced by the process of expressing any of the disclosed nucleic acids. Disclosed are any of the non-naturally occurring disclosed peptides produced by the process of expressing any of the disclosed nucleic acids. Disclosed are any of the disclosed peptides produced by the process of expressing any of the non-naturally disclosed nucleic acids.

202. Disclosed are animals produced by the process of transfecting a cell within the animal with any of the nucleic acid molecules disclosed herein. Disclosed are animals, including invertebrate embryos, produced by the process of transfecting a cell within the animal any of the nucleic acid molecules disclosed herein, wherein the animal is a mammal. Also disclosed are animals produced by the process of transfecting a cell within the animal any of the nucleic acid molecules disclosed herein, wherein the mammal is mouse, rat, rabbit, cow, sheep, pig, or primate.

203. Also disclose are animals produced by the process of adding to the animal any of the cells disclosed herein.

### D. Examples

204. The following examples are put forth so as to provide those of ordinary skill in the art with a complete disclosure and description of how the compounds, compositions, articles, devices and/or methods claimed herein are made and evaluated, and are intended to be purely exemplary and are not intended to limit the disclosure. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### 1. Example 1: Delivery of Chemical Compounds to Living Drosophila Embryos

205. Drosophila embryos are collected at 30 minutes prior to development. They are dechorionated by soaking in 100% bleach for 2 minutes. After extensive rinsing, embryos are transferred to 96 well plates containing chemical compounds at various concentrations, in 1% DMSO. Embryos are allowed to develop at 18 degrees Centigrade and assayed accordingly.

206. Initial studies examined the toxicity of DMSO in Drosophila embryos. Dose-response curves revealed that embryonic development proceeds normally to eclosion in concentrations of DMSO as high as 5% v/v.

207. Gamma secretase is an enzyme complex that cleaves type I transmembrane proteins. Inhibitors of this complex inhibit the cleavage of gamma secretase substrates, including Notch, a protein essential for normal development at all embryonic stages. A dose response study using gamma secretase inhibitors on developing wild type embryos, generated correspondingly severe Notch phenotypes that were identical to phenotypes reported for Notch genetic hypomorphs. The sensitivity of these assays was extreme, with mild phenotypes detectable at even femtomolar concentrations (Figure 1).

### 2. Example 2: Screen Designed Using the Amyloidgenic Peptide Associated with Alzheimer's Disease.

208. Transgenic Drosophila strains were made in which Abeta₁₋₄₀ and Abeta₁₋₄₂ were expressed throughout the CNS with pan-neuronal drivers. The uncleaved form of these peptides (known as CT100) were used and expressed with identical pan-neuronal drivers.

209. Alzheimer's Disease is characterized by dementia and deposition of amyloid containing plaques throughout the brain. No one type of neuronal sub-population is affected in this neurodegeneration. Furthermore, it was not known whether cells are insulted by an as yet unknown extracellular toxin, killed, and then dump their intracellular stores of undigestable amyloidgenic peptides into the extracellular space, where they accumulate as plaques, or whether the amyloidgenic peptides are themselves toxic intracellularly, accumulate to lethal levels and kill the neurons, leaving the Abeta plaques behind as the remnant ghosts of neurons that once contained them. In an effort to test both possibilities, the following transgenic strains of Drosophila melanogaster were made: UAS-Abeta₁₋₄₀, UAS-Abeta₁₋₄₂, and UAS-CT100.

210. Because these proteins accumulate within neurons, the constructs were duplicated and made with insect cuticle protein signal sequences (ss) as well, so that they would be secreted into the extracellular space. These strains then were: UAS-ₛₛAbeta₁₋₄₀, UAS-ₛₛAbeta₁₋₄₂, and UAS-ₛₛCT100.

211. Taking advantage of the UAS-GAL4 system (Brand and Perrimon, 1993) for the amplification and specific misexpression of reporter genes, pan-neuronal drivers were used to misexpress these peptides in every cell of the embryonic CNS (Figure 3).

212. A model has been proposed by which Alzheimer's Disease arises from impaired axonal transport of vesicles and organelles along microtubules. In this model, APP is packed into vesicles with β̅ any γ̅ secretase, the vesicles are transported via light chain I of kinesin along microtubules to the synapse, where the secretases function to cleave APP, and in the process, liberate vesicles from microtubules (Gunarwardena and Goldstein, 2001; Kamal et al, 2001).

213. When DiI labeling experiments were performed to examine the single cell defects associated with the misexpression of Abeta peptides pan-neuronally, it was observed that the population of neurons most strongly affected were longitudinal projection neurons. These neurons consistently failed to extend beyond segment boundaries in this gain of function background. When staining was done with fascicular antibodies, clear breaks could be visualized at segment boundaries that typically extended for 25% of the Anteroposterior length of the hemisegment.

### 3. Example 3: High Throughput Screen of Chemical Compounds Using Drosophila Melanogaster Embryos.

214. Screenable genetic phenotype in Drosophila melanogaster for human neurodegenerative diseases can be observed by the following method. Drosophila embryos are aliquoted into 96 well plates. Chemical compounds are then delivered to embryos in 96 well plates. An assay of phenotypic improvement is then carried out using seed fill algorithms.

### a) Sorting of embryos

215. Because heterozygous embryos appeared wild type, an embryo sorter from Union Biometrica was used to achieve two important goals: 1) heterozygous embryos (which had normal CNS phenotypes in comparison to homozygous sibs) were separated from homozygous embryos; 2) homozygous embryos can be precisely aliquoted into 96 well plates containing drugs in 1 µM concentrations, in 1% DMSO.

216. Because embryos are sorted using "Liquid Gold", which might generate as much as 1-2 ul volume of liquid per embryo per well, higher 1% DMSO concentration was used, assuming the final concentration would be 50-60% DMSO, concentrations that were known to be as effective in the gamma secretase inhibitor experiment as the 1% DMSO concentrations.

### b) Delivery of compounds

217. It is necessary to demonstrate that compounds can be delivered into Drosophila embryos, long believed to be impossible, because of the presence of the vitelline membrane, a thick chitinous structure that appears to be impervious to most biologically active molecules, including antibodies.

218. First it was shown that DMSO was not deleterious to normal embryonic development. A dose-response curve was performed, and it was demonstrated that normal development occurs in 100% of embryos in concentrations of DMSO as high as 5%.

219. It was then demonstrated that small molecules could be delivered to embryos through an intact vitelline membrane. This was tested using two approaches. Fluorescent size markers available from Molecular Probes Corporation were used, and it was demonstrated that fluorescently tagged molecules could enter into embryos with the cut-off size being 3 Kd. Because of the high level of autofluorescence in Drosophila embryos, the experiment was repeated using commercially available inhibitors of gamma secretase and it was demonstrated that drugs could enter into embryos with great ease, using 1% DMSO in PBS as a vehicle. Also, defects could be detected with great sensitivity in these embryos, with known Notch phenotypes being detectable at femtomolar concentrations.

220. When a transgenic line is generated that has lethal insertion sites, it is necessary to maintain the parental lab stocks with fluorescently tagged balancer chromosomes. (It is important to note that the need for balancer chromosomes will vary from line to line, and is primarily a function of the disruption at the insertion site). The parental lines themselves were stained and the axonal disruptions observed from the Abeta misexpression background were never found.

### c) The read-out assay

221. A seed-fill algorithm can be used as a rapid readout for this screen. Seed fill algorithms work as shown in Figure 7. Each image is divided into approximately 250,000 pixels, and each pixel is assigned a digital value for intensity, on a scale between 1-256. The computer evaluates the intensity of each pixel and then compares pairs of pixels and looks for neighbors that are equally bright. Fluorescent proteins never vary in their intensity, and plate readers can be used with seed fill algorithms. Essentially, the algorithm evaluates contiguous lines of pixels. So if membrane tagged fluorescent axons (pixels) are only 100 pixels long in the untreated controls, then a drug that ameliorates the phenotype might be expected to improve this number by what would be a statistically significant amount (determined, of course, by the variation in the controls). The algorithm can be designed to fit the plate reader software.

### 4. Example 4: Screening of Compounds for an Effect on Parkinson's Disease

222. The Parkinson's Disease model described above is used. A commercially available compound collection is it aliquoted into 96 well plates in 10 nM concentrations (Genesys Corporation, USDA). These collections are blindly seeded with positive and negative controls. Rotenone and other molecules known to be specific dopaminargic toxins serve as negative controls, and the commercially available agonist of Nurr1 (A receptor and transcription factor that directly results in dopaminergic neuron production) is a powerful positive control. The seed fill algorithm is modified to detect distance between dopaminergic neurons as a read-out rather than axonal length.

### 5. Example 5: Determining DMSO Concentrations

223. Broad dilution series were made of DMSO in PBS. Approximately 50 eggs were placed in each well of a 96 well plate containing these solutions. They were placed at 18°C or at 25°C and hatched offspring were counted at the end of embryonic development (36 hours at 18° and 24 hours at 25°). If larvae failed to hatch, particular defects were noted, and percentages that hatched were noted in each dose range. 100% of eggs produced normal larvae at concentrations as high as 5% in PBS, pH 7.2. At 7.5% DMSO the percentage declined dramatically to approximately 50% and by 10% DMSO, the percentage hatching was only ~10%. At 25°, the percentages surviving in solutions above 5% DMSO were always less than those kept at 18°.

### 6. Example 6: Depression and the Wolframin Gene

224. Wolframin is a gene that is strongly linked to depression in humans. Homozygous patients exhibit diabetes, optic atrophy and deafness, severe intellectual impairment and mood disorders and death by age 30. Heterozygous individuals are phenotypically wildtype with the exception of a marked increase in depression; 26 times as many Wolframin heterozygotes seek treatment for depression as compared to wild type siblings. This gene encodes a 9 pass transmembrane protein, which appears to function in subcellular vesicular trafficking.

225. There is one Drosophila ortholog of this gene and it too encodes a 9 pass transmembrane protein alebit with relatively weak homology. There are no extant mutants and the region appears to be chromatin protected in Drosophila. An RNAi approach was used to characterize loss of function phenotypes (Figure 11, panel B). Strong functional conservation has been validated between the orthologs, and a loss of function mutant can be generated by site-directed mutagenesis.

226. 22C10 staining of Flywolf loss of function (RNAi) embryos reveals commissural defects that vary by anteroposterior position. Wild type embryos were injected with dsRNA for Flywolf and were allowed to develop to the end of embryonic development. They were then filleted, fixed and stained with a monoclonal antibody (22C10) that stains pioneer neurons of each of the major tracts of the embryonic ventral nerve cord. Images were collected on a Biorad Microradiance 2000 confocal microscope. Also, the RNAi and DiI labeling protocol was followed to generate the single cell clones (Figure 12), in embryos in which flywolf function had been removed by RNAi. The NB 2-4 clone in loss of function Flywolf backgrounds generates contralaterally projecting motoneurons, but fails to project local interneuronal projections contralaterally. The loss of commissural interneuronal projection occurred in every clone examined.

227. These data are consistent with reports from vertebrate autopsies suggesting that the mood disorders associated with Wolfram's Syndrome are correlated with disruptions of the small cell layers of the cortex and show that the subcellular pathology appears to be similar in flies and humans. Experiments that demonstrate a strong functional conservation between fly and human version of the Wolframin protein have been conducted.

### 7. Example 7: Neuronal Toxicity Assays

228. Using Molecular Probe's TUNEL kit, it was found that pan-neuronal misexpression of Abeta 1-42 generated wide-spread neuronal death in flies. Using an antibody specific for Abeta 1-42, it was found that embryonic ventral nerve cords can generate amyloid peptides, and cell death due to misexpression of Abeta does not occur only in the cells misexpressing Abeta, which is not unexpected as the peptides are secreted.

### E. References

229. Throughout this application, various publications are referenced. The disclosures of these publications in their entireties are hereby incorporated by reference into this application in order to more fully describe the state of the art to which this invention pertains.

230. Campos-Ortega, J.A., and Hartenstein, V., eds., The Embryonic Development of Drosophila melanogaster, 2nd ed.(1996). Springer Verlag Press, Berlin.

231. King, R.C. (1970) Ovarian Development in Drosophila Melanogaster. Academic Press, New York.

232. Drew, J (1999) In Quest of Tomorrow's Medicines. springer Verlag, New York.

233. Mitchison TJ, and J. Sedat (1983). "Localization of antigenic determinants in whole Drosophila embryos" Dev Biol 99(1):261-4

234. Christopher A. Lipinski, Franco Lombardo, Beryl W. Dominy, Paul J. Feeney "Experimental and computational approaches to estimate solubility and permeability in drug discovery and development settings", Adv. Drug Delivery Rev., 1997, 23(1-3), 3-25.

235. Rubin, G. M. and A. C. Spradling. 1982. Genetic transformation of Drosophila with transposable element vectors. Science 218:348-353.

236. Salz, H. K., T. W. Cline and P. Schedl. 1987. Functional changes associated with structural alterations induced by mobilization of a P element inserted in the Sex-lethal gene of Drosophila. Genetics 117:221-231.

237. Searles, L. L., R. S. Jokerst, P. M. Bingham, R. A. Voelker and A. L. Greenleaf. 1982. Molecular cloning of sequences from a Drosophila RNA polymerase II locus by P element transposon tagging. Cell 31:585-592.

238. Sentry, J. W. and K. Kaiser. 1994. Application of inverse PCR to site-selected mutagenesis of Drosophila. Nucleic Acids Res 22:3429-30.

239. Serano, T. L., H. K. Cheung, L. H. Frank and R. S. Cohen. 1994. P element transformation vectors for studying Drosophila melanogaster oogenesis and early embryogenesis. Gene. 138:181-186.

240. Spradling, A. C. 1986. P element-mediated transformation. pp. 175-197 in Drosophila: A Practical Approach, edited by D. B. Roberts. IRL Press, Oxford.

241. The FlyBase Consortium (2003). The FlyBase database of the Drosophila genome projects and community literature. Nucleic Acids Research 31:172-175. http://flybase.org/

242. Spradling, A. C. and G. M. Rubin. 1982. Transposition of cloned P elements into Drosophila germ line chromosomes. Science 218:341-347.

243. Ashburner, M. 1989. Drosophila, A laboratory manual. Cold Spring Harbor Press, Cold Spring Harbor, New York.

244. Cary, L.C. et al. Transposon mutagenesis of baculoviruses: analysis of Trichoplusia ni transposon IFP2 insertions within the FP-locus of nuclear polyhedrosis viruses. Virology 172, 156-69 (1989).

245. Medhora, M.M., Maruyama, K. and Hartl, D.L. (1991) Genetics 128:311.

246. Hirschmann, et al., 1991. Angew. Chem. Int. Ed. Engl. 30:1278-1301

247. Schulz, et al., 1955. Cancer Res. 3(suppl.): 86-100; Schuler, et al., 1982. Terat. Carcin. Mutag. 2:293-301

248. Casso, D., Ramirez-Weber, F., Kornberg, T. GFP-Tagged balancer chromosomes for Drosophila melanogaster. Mechanisms of Development, 91: 451-454, 2000.

249. Furlong, E., Profitt, D., Scott, M. Automated sorting of live transgenic embryos. Nature Biotechnology, 19: 153-156,2001.

250. Lawrence, Peter. (1992) The Making of a Fly, The Genetics of Animal Design. Blackwell Science Ltd, Oxford.

251. Scangos, Nat. Biotechnol. (1997) 15:1220-1221

252. Lastowski-Perry et al, J. Biol. Chem., 260:1527, 1985

253. B. J. Bond et al, Mol. Cell. Biol., 6: 2080, 1986

254. Brand & Perrimon, Development (1993) 118: 401-415

255. Phelps & Brand, Methods (April 1998) 14:367-379

256. Steller & Pirrotta, Mol. Cell. Biol. 6:1640-1649, 1986

257. Christopher A. Lipinski, Franco Lombardo, Beryl W. Dominy, Paul J. Feeney, Adv. Drug Delivery Rev., 1997, 23(1-3), 3-25.

258. Roberts R.W. and Szostak J.W. Proc. Natl. Acad. Sci. USA, 94(23)12997-302 (1997).

259. Brand AH and N Perrimon (1993). "Targeted gene expression as a means of altering cell fates and generating dominant phenotypes." Development 118(2): 401-15.

260. Brand A, et al (1994). "Ectopic expression in Drosophila". Methods Cell Biol 44: 635-54.

261. Schmid A, et al, (1999). "Clonal analysis of Drosophila embryonic neuroblasts: neural cell types, axon projections and muscle targets." Development 126: 4653-89.

262. Feany MB, and WM Bender (2000). "A Drosophila Model of Parkinson's Disease". Nature 404: 394-8.

263. Pendleton RG, et al (2002). "Effects of Pharmacological Agents upon a Transgenic Model of Parkinson's Disease in Drosophila melanogaster." The Journal of Pharmacology and Experimental Therapeutics 300(1): 91-6.

264. Auluck PK et al (2002). "Chaperone Suppression of □-Synuclein Toxicity in a Drosophila Model for Parkinson's Disease". Science 295: 865-8

265. Drews J, (2000). "Drug Discovery: A historical Perspective". Science 287: 1960-4.

266. Gunawardena S, and LSB Goldstein (2001). "Disruption of Axonal Transport and neuronal Viability by Amyloid Precursor Protein Mutations in Drosophila." Neuron 32: 389-401

267. Kamal A, et al (2001). "Kinesin-mediated axonal transport of a membrane compartment containing B-secretase and presenilin-1 requires APP." Nature 414: 643-8.

## Claims

1. A method of making an invertebrate animal embryo useful for screening compounds comprising:
a) introducing a nucleic acid into the invertebrate embryo forming a transgenic invertebrate embryo;
b) dechorionating the transgenic invertebrate embryo; wherein the invertebrate embryo is transgenic for a human gene; wherein the expression of the human gene by the embryo is an invertebrate model for a disease; and
c) exposing the embryo to DMSO.

2. A method of screening a candidate compound for its effect on a disease comprising:
a) dechorionating a transgenic invertebrate animal embryo; wherein the invertebrate embryo is transgenic for a human gene; wherein the expression of the human gene by the embryo is an invertebrate model for the disease ;
b) administering the compound to the invertebrate transgenic animal embryo; and
c) assaying the effect of the compound on the embryo.

3. A method of screening a compound for its effect on an invertebrate embryo comprising :
a) dechorionating the embryo,
b) incubating the dechorionated embryo with the compound and DMSO, and
c) assaying the effect of the compound on the embryo.

4. The method of claim 1 or 2, wherein the effective amount of DMSO is 0.1% to 10%, preferably is 1% to 5%.

5. The method of any one of claims 1 to 4, wherein the invertebrate is from the genus Drosophila.

6. The method of claim 1 or 2, wherein the disease is a neurodegenerative disease.

## Patentansprüche

1. Verfahren zur Herstellung eines Wirbellosen-Embryos, der zum Screening von Verbindungen geeignet ist, umfassend:
a) Einführen einer Nucleinsäure in einen Wirbellosen-Embryo unter Bildung eines transgenen Wirbellosen-Embryos;
b) Dechorionieren des transgenen Wirbeilosen-Embryos; wobei der Wirbellosen-Embryo transgen bezüglich eines humanen Gens ist; wobei die Expression des humanen Gens durch den Embryo ein Wirbellosenmodell für eine Krankheit darstellt; und
c) Einwirkenlassen von DMSO auf den Embryo.

2. Verfahren zum Screening einer zu testenden Verbindung auf ihre Wirkung auf eine Krankheit, umfassend:
a) Dechorionieren eines transgenen Wirbellosen-Embryos; wobei der Wirbellosen-Embryo transgen bezüglich eines humanen Gens ist; wobei die Expression des humanen Gens durch den Embryo ein Wirbellosenmodell für eine Krankheit darstellt;
b) Verabreichen der Verbindung an den transgenen Wirbellosen-Embryo; und
c) Testen der Wirkung der Verbindung auf den Embryo.

3. Verfahren zum Screening einer Verbindung auf ihre Wirkung auf einen Wirbellosen-Embryo, umfassend:
a) Dechorionieren des Embryos;
b) Inkubieren des dechorionierten Embryos mit der Verbindung und DMSO; und
c) Testen der Wirkung der Verbindung auf den Embryo.

4. Verfahren gemäß Anspruch 1 oder 2, wobei die wirksame Menge an DMSO 0,1 bis 10%, vorzugsweise 1 bis 5%, beträgt.

5. Verfahren gemäß einem der Ansprüche 1 bis 4, wobei der Wirbellose zur Gattung *Drosophila* gehört.

6. Verfahren gemäß Anspruch 1 oder 2, wobei die Krankheit eine neurodegenerative Krankheit ist.

## Revendications

1. Procédé de fabrication d'un embryon animal invertébré utile pour la sélection de composés, comprenant:
a) l'introduction d'un acide nucléique dans l'embryon invertébré pour former un embryon invertébré transgénique;
b) la déchorionation de l'embryon invertébré transgénique; où l'embryon invertébré est transgénique pour un gène humain; où l'expression du gène humain par l'embryon est un modèle invertébré d'une maladie; et
c) l'exposition de l'embryon à du DMSO.

2. Procédé de sélection d'un composé candidat pour son effet sur une maladie, comprenant:
a) la déchorionation d'un embryon animal invertébré transgénique; où l'embryon invertébré est transgénique pour un gène humain; où l'expression du gène humain par l'embryon est un modèle invertébré de la maladie;
b) l'administration du composé à l'embryon animal invertébré transgénique; et
c) l'analyse de l'effet du composé sur l'embryon.

3. Procédé de sélection d'un composé pour son effet sur un embryon invertébré, comprenant:
a) la déchorionation de l'embryon,
b) l'incubation de l'embryon déchorioné avec le composé et du DMSO, et
c) l'analyse de l'effet du composé sur l'embryon.

4. Procédé selon la revendication 1 ou 2, dans lequel la quantité efficace de DMSO est de 0, 1 % à 10%, de préférence, de 1 % à 5%.

5. Procédé selon l'une quelconque des revendications 1 à 4, dans lequel l'invertébré est issu du genre Drosophila.

6. Procédé selon la revendication 1 ou 2, dans lequel la maladie est une maladie neurodégénérative.


### SEQUENCE LISTING

<110> University of Utah Research Foundation
<120> METHODS AND COMPOSITIONS RELATED TO DELIVERY OF CHEMICAL COMPOUNDS TO INVERTEBRATE EMBRYOS
<130> 21101.0052P1
<140> Unassigned
   <141> 2005-05-23
<150> 60/573,194
   <151> 2004-05-21
<160> 27
<170> FastSEQ for Windows Version 4.0
<210> 1
   <211> 2479
   <212> DNA
   <213> Artificial Sequence
<220>
   <223> Description of Artificial Sequence; note = synthetic construct
<400> 1
   ccccctcggc cccgcgcgtc gcctgtcctc cgagccagtc gctgacagcc gcggcgccgc     60
   gagcttctcc tctcctcacg accgagagca gtcattatgg cgaaccttgg ctgctggatg    120
   ctggttctct ttgtggccac atggagtgac ctgggcctct gcaagaagcg cccgaagcct    180
   ggaggatgga acactggggg cagccgatac ccggggcagg gcagccctgg aggcaaccgc    240
   tacccacctc agggcggtgg tggctggggg cagcctcatg gtggtggctg ggggcagcct     300
   catggtggtg gctgggggca gccccatggt ggtggctggg gacagcctca tggtggtggc    360
   tggggtcaag gaggtggcac ccacagtcag tggaacaagc cgagtaagcc aaaaaccaac    420
   atgaagcaca tggctggtgc tgcagcagct ggggcagtgg tggggggcct tggcggctac    480
   atgctgggaa gtgccatgag caggcccatc atacatttcg gcagtgacta tgaggaccgt    540
   tactatcgtg aaaacatgca ccgttacccc aaccaagtgt actacaggcc catggatgag    600
   tacagcaacc agaacaactt tgtgcacgac tgcgtcaata tcacaatcaa gcagcacacg    660
   gtcaccacaa ccaccaaggg ggagaacttc accgagaccg acgttaagat gatggagcgc    720
   gtggttgagc agatgtgtat cacccagtac gagagggaat ctcaggccta ttaccagaga    780
   ggatcgagca tggtcctctt ctcctctcca cctgtgatcc tcctgatctc tttcctcatc    840
   ttcctgatag tgggatgagg aaggtcttcc tgttttcacc atctttctaa tctttttcca    900
   gcttgaggga ggcggtatcc acctgcagcc cttttagtgg tggtgtctca ctctttcttc    960
   tctctttgtc ccggataggc taatcaatac ccttggcact gatgggcact ggaaaacata   1020
   gagtagacct gagatgctgg tcaagccccc tttgattgag ttcatcatga gccgttgcta   1080
   atgccaggcc agtaaaagta taacagcaaa taaccattgg ttaatctgga cttatttttg    1140
   gacttagtgc aacaggttga ggctaaaaca aatctcagaa cagtctgaaa tacctttgcc   1200
   tggatacctc tggctccttc agcagctaga gctcagtata ctaatgccct atcttagtag   1260
   agatttcata gctatttaga gatattttcc attttaagaa aacccgacaa catttctgcc   1320
   aggtttgtta ggaggccaca tgatacttat tcaaaaaaat cctagagatt cttagctctt   1380
   gggatgcagg ctcagcccgc tggagcatga gctctgtgtg taccgagaac tggggtgatg   1440
   ttttactttt cacagtatgg gctacacagc agctgttcaa caagagtaaa tattgtcaca   1500
   acactgaacc tctggctaga ggacatattc acagtgaaca taactgtaac atatatgaaa   1560
   ggcttctggg acttgaaatc aaatgtttgg gaatggtgcc cttggaggca acctcccatt   1620
   ttagatgttt aaaggaccct atatgtggca ttcctttctt taaactatag gtaattaagg   1680
   cagctgaaaa gtaaattgcc ttctagacac tgaaggcaaa tctcctttgt ccatttacct   1740
   ggaaaccaga atgattttga catacaggag agctgcagtt gtgaaagcac catcatcata   1800
   gaggatgatg taattaaaaa atggtcagtg tgcaaagaaa agaactgctt gcatttcttt   1860
   atttctgtct cataattgtc aaaaaccaga attaggtcaa gttcatagtt tctgtaattg   1920
   gcttttgaat caaagaatag ggagacaatc taaaaaatat cttaggttgg agatgacaga   1980
   aatatgattg atttgaagtg gaaaaagaaa ttctgttaat gttaattaaa gtaaaattat   2040
   tccctgaatt gtttgatatt gtcacctagc agatatgtat tacttttctg caatgttatt   2100
   attggcttgc actttgtgag tattctatgt aaaaatatat atgtatataa aatatatatt   2160
   gcataggaca gacttaggag ttttgtttag agcagttaac atctgaagtg tctaatgcat   2220
   taacttttgt aaggtactga atacttaata tgtgggaaac ccttttgcgt ggtccttagg    2280
   cttacaatgt gcactgaatc gtttcatgta agaatccaaa gtggacacca ttaacaggtc   2340
   tttgaaatat gcatgtactt tatattttct atatttgtaa ctttgcatgt tcttgttttg   2400
   ttatataaaa aaattgtaaa tgtttaatat ctgactgaaa ttaaacgagc gaagatgagc   2460
   accaaaaaaa aaaaaaaaa                                                2479
<210> 2
   <211> 722
   <212> DNA
   <213> Artificial Sequence
<220>
   <223> Description of Artificial Sequence; note = synthetic construct
<400> 2
   aaggctcagt ataaatagca gccaccgctc cctggcaggc agggacccgc agctcagcta      60
   cagcacagat caggtgagga gcacaccaag gagtgatttt taaaacttac tctgttttct     120
   ctttcccaac aagattatca tttcctttaa aaaaaatagt tatcctgggg catacagcca     180
   taccattctg aaggtgtctt atctcctctg atctagagag caccatgaag cttctcacgg     240
   gcctggtttt ctgctccttg gtcctgggtg tcagcagccg aagcttcttt tcgttccttg     300
   gcgaggcttt tgatggggct cgggacatgt ggagagccta ctctgacatg agagaagcca     360
   attacatcgg ctcagacaaa tacttccatg ctcgggggaa ctatgatgct gccaaaaggg     420
   gacctggggg tgcctgggct gcagaagtga tcagcgatgc cagagagaat atccagagat     480
   tctttggcca tggtgcggag gactcgctgg ctgatcaggc tgccaatgaa tggggcagga     540
   gtggcaaaga ccccaatcac ttccgacctg ctggcctgcc tgagaaatac tgagcttcct     600
   cttcactctg ctctcaggag atctggctgt gaggccctca gggcagggat acaaagcggg     660
   gagagggtac acaatgggta tctaataaat acttaagagg tggaaaaaaa aaaaaaaaaa     720
   aa                                                                    722
<210> 3
   <211> 36
   <212> PRT
   <213> Artificial Sequence
<220>
   <223> Description of Artificial Sequence; note = synthetic construct
<400> 3
   Asp Ala Glu Phe Arg His Asp Ser Gly Tyr Glu Val His His Gln Lys
   1               5                   10                  15
   Leu Val Phe Phe Ala Glu Asp Val Gly Ser Asn Lys Gly Ala Ile Ile
               20                  25                  30
   Gly Leu Met Val
           35
<210> 4
   <211> 38
   <212> PRT
   <213> Artificial Sequence
<220>
   <223> Description of Artificial Sequence; note = synthetic construct
<400> 4
   Asp Ala Glu Phe Arg His Asp Ser Gly Tyr Glu Val His His Gln Lys
   1               5                   10                  15
   Leu Val Phe Phe Ala Glu Asp Val Gly Ser Asn Lys Gly Ala Ile Ile
               20                  25                  30
   Gly Leu Met Val Ile Ala
           35
<210> 5
   <211> 54
   <212> PRT
   <213> Artificial Sequence
<220>
   <223> Description of Artificial Sequence; note = synthetic construct
<400> 5
   Asp Ala Glu Phe Arg His Asp Ser Gly Tyr Glu Val His His Gln Lys
    1               5                  10                  15
   Leu Val Phe Phe Ala Glu Asp Val Gly Ser Asn Lys Gly Ala Ile Ile
               20                  25                  30
   Gly Leu Met Val Ile Ala Thr Val Ile Val Ile Thr Leu Val Met Leu
           35                  40                  45
   Lys Lys Gln Tyr Thr Ser
       50
<210> 6
   <211> 95
   <212> PRT
   <213> Artificial Sequence
<220>
   <223> Description of Artificial Sequence; note = synthetic construct
<400> 6
   Asp Ala Glu Phe Arg His Asp Ser Gly Tyr Glu Val His His Gln Lys
    1               5                  10                  15
   Leu Val Phe Phe Ala Glu Asp Val Gly Ser Asn Lys Gly Ala Ile Ile
               20                  25                  30
   Gly Leu Met Val Ile Ala Thr Val Ile Val Ile Thr Leu Val Met Leu
           35                  40                  45
   Lys Lys Gln Tyr Thr Ser Ile His His Gly Val Val Glu Val Asp Ala
       50                  55                  60
   Ala Val Thr Pro Glu Glu Arg His Leu Ser Lys Met Gln Gln Asn Gly
   65                  70                  75                  80
   Tyr Glu Asn Pro Thr Tyr Lys Phe Phe Glu Gln Met Gly Asn Cys
                   85                  90                  95
<210> 7
   <211> 253
   <212> PRT
   <213> Artificial Sequence
<220>
   <223> Description of Artificial Sequence; note = synthetic construct
<400> 7
<210> 8
   <211> 695
   <212> PRT
   <213> Artificial Sequence
<220>
   <223> Description of Artificial Sequence; note = synthetic construct
<400> 8
   Met Leu Pro Ser Leu Ala Leu Leu Leu Leu Ala Ala Trp Thr Val Arg
    1               5                  10                  15
   Ala Leu Glu Val Pro Thr Asp Gly Asn Ala Gly Leu Leu Ala Glu Pro
               20                  25                  30
   Gln Ile Ala Met Phe Cys Gly Lys Leu Asn Met His Met Asn Val Gln
           35                  40                  45
   Asn Gly Lys Trp Glu Ser Asp Pro Ser Gly Thr Lys Thr Cys Ile Gly
       50                  55                  60
   Thr Lys Glu Gly Ile Leu Gln Tyr Cys Gln Glu Val Tyr Pro Glu Leu
   65                  70                  75                  80
   Gln Ile Thr Asn Val Val Glu Ala Asn Gln Pro Val Thr Ile Gln Asn
                   85                  90                  95
   Trp Cys Lys Arg Gly Arg Lys Gln Cys Lys Thr His Thr His Ile Val
               100                 105                 110
   Ile Pro Tyr Arg Cys Leu Val Gly Glu Phe Val Ser Asp Ala Leu Leu
           115                 120                 125
   Val Pro Asp Lys Cys Lys Phe Leu His Gln Glu Arg Met Asp Val Cys
   130 135 140
   Glu Thr His Leu His Trp His Thr Val Ala Lys Glu Thr Cys Ser Glu
   145 150 155 160
   Lys Ser Thr Asn Leu His Asp Tyr Gly Met Leu Leu Pro Cys Gly Ile
   165 170 175
   Asp Lys Phe Arg Gly Val Glu Phe Val Cys Cys Pro Leu Ala Glu Glu
   180 185 190
   Ser Asp Ser Val Asp Ser Ala Asp Ala Glu Glu Asp Asp Ser Asp Val
   195 200 205
   Trp Trp Gly Gly Ala Asp Thr Asp Tyr Ala Asp Gly Gly Glu Asp Lys
   210 215 220
   Val Val Glu Val Ala Glu Glu Glu Glu Val Ala Asp Val Glu Glu Glu
   225 230 235 240
   Glu Ala Asp Asp Asp Glu Asp Val Glu Asp Gly Asp Glu Val Glu Glu
   245 250 255
   Glu Ala Glu Glu Pro Tyr Glu Glu Ala Thr Glu Arg Thr Thr Ser Thr
   260 265 270
   Ala Thr Thr Thr Thr Thr Thr Thr Glu Ser Val Glu Glu Val Val Arg
   275 280 285
   Val Pro Thr Thr Ala Ala Ser Thr Pro Asp Ala Val Asp Lys Tyr Leu
   290 295 300
   Glu Thr Pro Gly Asp Glu Asn Glu His Ala His Phe Gln Lys Ala Lys
   305 310 315 320
   Glu Arg Leu Glu Ala Lys His Arg Glu Arg Met Ser Gln Val Met Arg
   325 330 335
   Glu Trp Glu Glu Ala Glu Arg Gln Ala Lys Asn Leu Pro Lys Ala Asp
   340 345 350
   Lys Lys Ala Val Ile Gln His Phe Gln Glu Lys Val Glu Ser Leu Glu
   355 360 365
   Gln Glu Ala Ala Asn Glu Arg Gln Gln Leu Val Glu Thr His Met Ala
   370 375 380
   Arg Val Glu Ala Met Leu Asn Asp Arg Arg Arg Leu Ala Leu Glu Asn
   385 390 395 400
   Tyr Ile Thr Ala Leu Gln Ala Val Pro Pro Arg Pro His His Val Phe
   405 410 415
   Asn Met Leu Lys Lys Tyr Val Arg Ala Glu Gln Lys Asp Arg Gln His
   420 425 430
   Thr Leu Lys His Phe Glu His Val Arg Met Val Asp Pro Lys Lys Ala
   435 440 445
   Ala Gln Ile Arg Ser Gln Val Met Thr His Leu Arg Val Ile Tyr Glu
   450 455 460
   Arg Met Asn Gln Ser Leu Ser Leu Leu Tyr Asn Val Pro Ala Val Ala
   465 470 475 480
   Glu Glu Ile Gln Asp Glu Val Asp Glu Leu Leu Gln Lys Glu Gln Asn
   485 490 495
   Tyr Ser Asp Asp Val Leu Ala Asn Met Ile Ser Glu Pro Arg Ile Ser
   500 505 510
   Tyr Gly Asn Asp Ala Leu Met Pro Ser Leu Thr Glu Thr Lys Thr Thr
   515 520 525
   Val Glu Leu Leu Pro Val Asn Gly Glu Phe Ser Leu Asp Asp Leu Gln
   530 535 540
   Pro Trp His Pro Phe Gly Val Asp Ser Val Pro Ala Asn Thr Glu Asn
   545 550 555 560
   Glu Val Glu Pro Val Asp Ala Arg Pro Ala Ala Asp Arg Gly Leu Thr
   565 570 575
   Thr Arg Pro Gly Ser Gly Leu Thr Asn Ile Lys Thr Glu Glu Ile Ser
   580 585 590
   Glu Val Lys Met Asp Ala Glu Phe Gly His Asp Ser Gly Phe Glu Val
   595 600 605
   Arg His Gln Lys Leu Val Phe Phe Ala Glu Asp Val Gly Ser Asn Lys
       610                 615                 620
   Gly Ala Ile Ile Gly Leu Met Val Gly Gly Val Val Ile Ala Thr Val
   625                 630                 635                 640
   Ile Val Ile Thr Leu Val Met Leu Lys Lys Lys Gln Tyr Thr Ser Ile
                   645                 650                 655
   His His Gly Val Val Glu Val Asp Ala Ala Val Thr Pro Glu Glu Arg
               660                 665                 670
   His Leu Ser Lys Met Gln Gln Asn Gly Tyr Glu Asn Pro Thr Tyr Lys
           675                 680                 685
   Phe Phe Glu Gln Met Gln Asn
       690                 695
<210> 9
   <211> 360
   <212> PRT
   <213> Artificial Sequence
<220>
   <223> Description of Artificial Sequence; note = synthetic construct
<400> 9
   Met Glu Glu Arg Glu Arg Gly Ala Arg Ser Ala Gly Ala Gly Ser Pro
    1               5                  10                  15
   Ala Arg Pro Pro Ser Pro Arg Leu Asp Val Ser Ser Asp Ser Phe Asp
               20                  25                  30
   Pro Leu Leu Ala Leu Tyr Ala Pro Arg Leu Pro Pro Ile Pro Tyr Pro
           35                  40                  45
   Asn Ala Pro Cys Phe Asn Asn Val Ala Glu Tyr Glu Ser Phe Leu Arg
       50                  55                  60
   Thr Gly Val Arg Gly Gly Gly Arg Gly Arg Gly Arg Ala Arg Gly Ala
   65                  70                  75                  80
   Ala Ala Gly Ser Gly Val Pro Ala Ala Pro Gly Pro Ser Gly Arg Thr
                   85                  90                  95
   Arg Arg Arg Pro Asp Ala Pro Ala Pro Asp Pro Glu Arg Ile Gln Arg
               100                 105                 110
   Leu Arg Arg Leu Met Val Ala Lys Glu Glu Gly Asp Gly Ala Ala Gly
           115                 120                 125
   Ala Gly Arg Arg Gly Pro Gly Arg Ser Arg Lys Ala Pro Arg Asn Val
       130                 135                 140
   Leu Thr Arg Met Pro Leu His Glu Gly Ser Pro Leu Gly Glu Leu His
   145                 150                 155                 160
   Arg Cys Ile Arg Glu Gly Val Lys Val Asn Val His Ile Arg Thr Phe
                   165                 170                 175
   Lys Gly Leu Arg Gly Val Cys Thr Gly Phe Leu Val Ala Phe Asp Lys
               180                 185                 190
   Phe Trp Asn Met Ala Leu Thr Asp Val Asp Glu Thr Tyr Arg Lys Pro
           195                 200                 205
   Val Leu Gly Lys Ala Tyr Glu Arg Asp Ser Ser Leu Thr Leu Thr Arg
       210                 215                 220
   Leu Phe Asp Arg Leu Lys Leu Gln Asp Ser Ser Lys Lys Glu Ala Asp
   225                 230                 235                 240
   Ser Lys Ser Ala Val Glu Asp Ser Thr Leu Ser Arg Tyr Ser Gln Thr
                   245                 250                 255
   Ser Thr Trp Lys Leu Ala Ser Val Trp Gly Arg Ala Asp Thr Gly Arg
               260                 265                 270
   Gly Ser His Lys Arg Ser Arg Ser Val Pro Ser Ser Leu Gln Ala Ser
           275                 280                 285
   Ala Arg Glu Glu Ser Arg Ser Glu Leu Ser Gly Arg Thr Thr Arg Thr
   290 295 300
   Asp Gly Ser Ser Val Gly Gly Thr Phe Ser Arg Ala Thr Thr Leu Ser
   305 310 315 320
   Arg Gly Gln Ser Arg Lys Lys Lys Arg Lys Pro Lys Val Asp Tyr Gln
   325 330 335
   Gln Val Phe Thr Arg His Ile Asn Gln Ile Phe Ile Arg Gly Glu Asn
   340 345 350
   Val Leu Leu Val His Leu Ala Gln
   355 360
<210> 10
   <211> 226
   <212> PRT
   <213> Artificial Sequence
<220>
   <223> Description of Artificial Sequence; note = synthetic construct
<400> 10
   Met Ser Asp Glu Thr Asn Ala Ala Val Trp Asp Asp Ser Leu Leu Val
   1 5 10 15
   Lys Thr Tyr Asp Glu Ser Val Gly Leu Ala Arg Glu Ala Leu Ala Arg
   20 25 30
   Arg Leu Ala Asp Ser Thr Asn Lys Arg Glu Glu Glu Asn Ala Ala Ala
   35 40 45
   Ala Glu Glu Glu Ala Gly Glu Ile Ser Ala Thr Gly Gly Ala Thr Ser
   50 55 60
   Pro Glu Pro Val Ser Phe Lys Val Gly Asp Tyr Ala Arg Ala Thr Tyr
   65 70 75 80
   Val Asp Gly Val Asp Tyr Glu Gly Ala Val Val Ser Ile Asn Glu Glu
   85 90 95
   Lys Gly Thr Cys Val Leu Arg Tyr Leu Gly Tyr Glu Asn Glu Gln Glu
   100 105 110
   Val Leu Leu Val Asp Leu Leu Pro Ser Trp Gly Lys Arg Val Arg Arg
   115 120 125
   Glu Gln Phe Leu Ile Ala Lys Lys Asp Glu Asp Glu Gln Leu Ser Arg
   130 135 140
   Pro Lys Ala Ser Ala Gly Ser His Ser Lys Thr Pro Lys Ser Ser Arg
   145 150 155 160
   Arg Ser Arg Ile Ser Gly Gly Leu Val Met Pro Pro Met Pro Pro Val
   165 170 175
   Pro Pro Met Ile Val Gly Gln Gly Asp Gly Ala Glu Gln Asp Phe Val
   180 185 190
   Ala Met Leu Thr Ala Trp Tyr Met Ser Gly Tyr Tyr Thr Gly Leu Tyr
   195 200 205
   Gln Gly Lys Lys Glu Ala Ser Thr Thr Ser Gly Lys Lys Lys Thr Pro
   210 215 220
   Lys Lys
   225
<210> 11
   <211> 282
   <212> PRT
   <213> Artificial Sequence
<220>
   <223> Description of Artificial Sequence; note = synthetic construct
<400> 11
   Met Ala Met Ser Ser Gly Gly Ser Gly Gly Gly Val Pro Glu Gln Glu
    1              5                   10                  15
   Asp Ser Val Leu Phe Arg Arg Gly Thr Gly Gln Ser Asp Asp Ser Asp
               20                  25                  30
   Ile Trp Asp Asp Thr Ala Leu Ile Lys Ala Tyr Asp Lys Ala Val Ala
           35                  40                  45
   Ser Phe Lys His Ala Leu Lys Asn Gly Asp Ile Cys Glu Thr Ser Gly
       50                  55                  60
   Lys Pro Lys Thr Thr Pro Lys Arg Lys Pro Ala Lys Lys Asn Lys Ser
   65                  70                  75                  80
   Gln Lys Lys Asn Thr Ala Ala Ser Leu Gln Gln Trp Lys Val Gly Asp
                   85                  90                  95
   Lys Cys Ser Ala Ile Trp Ser Glu Asp Gly Cys Ile Tyr Pro Ala Thr
               100                 105                 110
   Ile Ala Ser Ile Asp Phe Lys Arg Glu Thr Cys Val Val Val Tyr Thr
           115                 120                 125
   Gly Tyr Gly Asn Arg Glu Glu Gln Asn Leu Ser Asp Leu Leu Ser Pro
       130                 135                 140
   Ile Cys Glu Val Ala Asn Asn Ile Glu Gln Asn Ala Gln Glu Asn Glu
   145                 150                 155                 160
   Asn Glu Ser Gln Val Ser Thr Asp Glu Ser Glu Asn Ser Arg Ser Pro
                   165                 170                 175
   Gly Asn Lys Ser Asp Asn Ile Lys Pro Lys Ser Ala Pro Trp Asn Ser
               180                 185                 190
   Phe Leu Pro Pro Pro Pro Pro Met Pro Gly Pro Arg Leu Gly Pro Gly
           195                 200                 205
   Lys Pro Gly Leu Lys Phe Asn Gly Pro Pro Pro Pro Pro Pro Pro Pro
       210                 215                 220
   Pro Pro His Leu Leu Ser Cys Trp Leu Pro Pro Phe Pro Ser Gly Pro
   225                 230                 235                 240
   Pro Ile Ile Pro Pro Pro Pro Pro Ile Cys Pro Asp Ser Leu Asp Asp
                   245                 250                 255
   Ala Asp Ala Leu Gly Ser Met Leu Ile Ser Trp Tyr Met Ser Gly Tyr
               260                 265                 270
   His Thr Gly Tyr Tyr Met Glu Met Leu Ala
           275                 280
<210> 12
   <211> 262
   <212> PRT
   <213> Artificial Sequence
<220>
   <223> Description of Artificial Sequence; note = synthetic construct
<400> 12
   Met Ala Met Ser Ser Gly Gly Ser Gly Gly Gly Val Pro Glu Gln Glu
    1              5                   10                  15
   Asp Ser Val Leu Phe Arg Arg Gly Thr Gly Gln Ser Asp Asp Ser Asp
               20                  25                  30
   Ile Trp Asp Asp Thr Ala Leu Ile Lys Ala Tyr Asp Lys Ala Val Ala
           35                  40                  45
   Ser Phe Lys His Ala Leu Lys Asn Gly Asp Ile Cys Glu Thr Ser Gly
       50                  55                  60
   Lys Pro Lys Thr Thr Pro Lys Arg Lys Pro Ala Lys Lys Asn Lys Ser
   65                  70                  75                  80
   Gln Lys Lys Asn Thr Ala Ala Ser Leu Gln Gln Trp Lys Val Gly Asp
                   85                  90                  95
   Lys Cys Ser Ala Ile Trp Ser Glu Asp Gly Cys Ile Tyr Pro Ala Thr
               100                 105                 110
   Ile Ala Ser Ile Asp Phe Lys Arg Glu Thr Cys Val Val Val Tyr Thr
           115                 120                 125
   Gly Tyr Gly Asn Arg Glu Glu Gln Asn Leu Ser Asp Leu Leu Ser Pro
       130                 135                 140
   Ile Cys Glu Val Ala Asn Asn Ile Glu Gln Asn Ala Gln Glu Asn Glu
   145                 150                 155                 160
   Asn Glu Ser Gln Val Ser Thr Asp Glu Ser Glu Asn Ser Arg Ser Pro
                   165                 170                 175
   Gly Asn Lys Ser Asp Asn Ile Lys Pro Lys Ser Ala Pro Trp Asn Ser
               180                 185                 190
   Phe Leu Pro Pro Pro Pro Pro Met Pro Gly Pro Arg Leu Gly Pro Gly
           195                 200                 205
   Lys Ile Ile Pro Pro Pro Pro Pro Ile Cys Pro Asp Ser Leu Asp Asp
       210                 215                 220
   Ala Asp Ala Leu Gly Ser Met Leu Ile Ser Trp Tyr Met Ser Gly Tyr
   225                 230                 235                 240
   His Thr Gly Tyr Tyr Met Gly Phe Arg Gln Asn Gln Lys Glu Gly Arg
                   245                 250                 255
   Cys Ser His Ser Leu Asn
               260
<210> 13
   <211> 250
   <212> PRT
   <213> Artificial Sequence
<220>
   <223> Description of Artificial Sequence; note = synthetic construct
<400> 13
   Met Ala Met Ser Ser Gly Gly Ser Gly Gly Gly Val Pro Glu Gln Glu
   1 5 10 15
   Asp Ser Val Leu Phe Arg Arg Gly Thr Gly Gln Ser Asp Asp Ser Asp
   20 25 30
   Ile Trp Asp Asp Thr Ala Leu Ile Lys Ala Tyr Asp Lys Ala Val Ala
   35 40 45
   Ser Phe Lys His Ala Leu Lys Asn Gly Asp Ile Cys Glu Thr Ser Gly
   50 55 60
   Lys Pro Lys Thr Thr Pro Lys Arg Lys Pro Ala Lys Lys Asn Lys Ser
   65 70 75 80
   Gln Lys Lys Asn Thr Ala Ala Ser Leu Gln Gln Trp Lys Val Gly Asp
   85 90 95
   Lys Cys Ser Ala Ile Trp Ser Glu Asp Gly Cys Ile Tyr Pro Ala Thr
   100 105 110
   Ile Ala Ser Ile Asp Phe Lys Arg Glu Thr Cys Val Val Val Tyr Thr
   115 120 125
   Gly Tyr Gly Asn Arg Glu Glu Gln Asn Leu Ser Asp Leu Leu Ser Pro
   130 135 140
   Ile Cys Glu Val Ala Asn Asn Ile Glu Gln Asn Ala Gln Glu Asn Glu
   145 150 155 160
   Asn Glu Ser Gln Val Ser Thr Asp Glu Ser Glu Asn Ser Arg Ser Pro
   165 170 175
   Gly Asn Lys Ser Asp Asn Ile Lys Pro Lys Ser Ala Pro Trp Asn Ser
   180 185 190
   Phe Leu Pro Pro Pro Pro Pro Met Pro Gly Pro Arg Leu Gly Pro Gly
   195 200 205
   Lys Ile Ile Pro Pro Pro Pro Pro Ile Cys Pro Asp Ser Leu Asp Asp
   210 215 220
   Ala Asp Ala Leu Gly Ser Met Leu Ile Ser Trp Tyr Met Ser Gly Tyr
   225 230 235 240
   His Thr Gly Tyr Tyr Met Glu Met Leu Ala
   245 250
<210> 14
   <211> 294
   <212> PRT
   <213> Artificial Sequence
<220>
   <223> Description of Artificial Sequence; note = synthetic construct
<400> 14
   Met Ala Met Ser Ser Gly Gly Ser Gly Gly Gly Val Pro Glu Gln Glu
    1              5                   10                  15
   Asp Ser Val Leu Phe Arg Arg Gly Thr Gly Gln Ser Asp Asp Ser Asp
               20                  25                  30
   Ile Trp Asp Asp Thr Ala Leu Ile Lys Ala Tyr Asp Lys Ala Val Ala
           35                  40                  45
   Ser Phe Lys His Ala Leu Lys Asn Gly Asp Ile Cys Glu Thr Ser Gly
       50                  55                  60
   Lys Pro Lys Thr Thr Pro Lys Arg Lys Pro Ala Lys Lys Asn Lys Ser
   65                  70                  75                  80
   Gln Lys Lys Asn Thr Ala Ala Ser Leu Gln Gln Trp Lys Val Gly Asp
                   85                  90                  95
   Lys Cys Ser Ala Ile Trp Ser Glu Asp Gly Cys Ile Tyr Pro Ala Thr
               100                 105                 110
   Ile Ala Ser Ile Asp Phe Lys Arg Glu Thr Cys Val Val Val Tyr Thr
           115                 120                 125
   Gly Tyr Gly Asn Arg Glu Glu Gln Asn Leu Ser Asp Leu Leu Ser Pro
       130                 135                 140
   Ile Cys Glu Val Ala Asn Asn Ile Glu Gln Asn Ala Gln Glu Asn Glu
   145                 150                 155                 160
   Asn Glu Ser Gln Val Ser Thr Asp Glu Ser Glu Asn Ser Arg Ser Pro
                   165                 170                 175
   Gly Asn Lys Ser Asp Asn Ile Lys Pro Lys Ser Ala Pro Trp Asn Ser
               180                 185                 190
   Phe Leu Pro Pro Pro Pro Pro Met Pro Gly Pro Arg Leu Gly Pro Gly
           195                 200                 205
   Lys Pro Gly Leu Lys Phe Asn Gly Pro Pro Pro Pro Pro Pro Pro Pro
       210                 215                 220
   Pro Pro His Leu Leu Ser Cys Trp Leu Pro Pro Phe Pro Ser Gly Pro
   225                 230                 235                 240
   Pro Ile Ile Pro Pro Pro Pro Pro Ile Cys Pro Asp Ser Leu Asp Asp
                   245                 250                 255
   Ala Asp Ala Leu Gly Ser Met Leu Ile Ser Trp Tyr Met Ser Gly Tyr
               260                 265                 270
   His Thr Gly Tyr Tyr Met Gly Phe Arg Gln Asn Gln Lys Glu Gly Arg
           275                 280                 285
   Cys Ser His Ser Leu Asn
       290
<210> 15
   <211> 140
   <212> PRT
   <213> Artificial Sequence
<220>
   <223> Description of Artificial Sequence; note = synthetic construct
<400> 15
<210> 16
   <211> 3139
   <212> PRT
   <213> Artificial Sequence
<220>
   <223> Description of Artificial Sequence; note = synthetic construct
<400> 16
   Met Ala Thr Leu Glu Lys Leu Met Lys Ala Phe Glu Ser Leu Lys Ser
    1               5                  10                  15
   Phe Gln Gln Gln Gln Gln Gln Gln Gln Gln Gln Gln Gln Gln Gln Gln
               20                  25                  30
   Gln Gln Gln Gln Gln Gln Gln Gln Pro Pro Pro Pro Pro Pro Pro Pro
           35                  40                  45
   Pro Pro Pro Gln Leu Pro Gln Pro Pro Pro Gln Ala Gln Pro Leu Leu
       50                  55                  60
   Pro Gln Pro Gln Pro Pro Pro Pro Pro Pro Pro Pro Pro Pro Gly Pro
   65                  70                  75                  80
   Ala Val Ala Glu Glu Pro Leu His Arg Pro Lys Lys Glu Leu Ser Ala
                   85                  90                  95
   Thr Lys Lys Asp Arg Val Asn His Cys Leu Thr Ile Cys Glu Asn Ile
               100                 105                 110
   Val Ala Gln Ser Val Arg Asn Ser Pro Glu Phe Gln Lys Leu Leu Gly
           115                 120                 125
   Ile Ala Met Glu Leu Phe Leu Leu Cys Ser Asp Asp Ala Glu Ser Asp
       130                 135                 140
   Val Arg Met Val Ala Asp Glu Cys Leu Asn Lys Val Ile Lys Ala Leu
   145                 150                 155                 160
   Met Asp Ser Asn Leu Pro Arg Leu Gln Leu Glu Leu Tyr Lys Glu Ile
                   165                 170                 175
   Lys Lys Asn Gly Ala Pro Arg Ser Leu Arg Ala Ala Leu Trp Arg Phe
               180                 185                 190
   Ala Glu Leu Ala His Leu Val Arg Pro Gln Lys Cys Arg Pro Tyr Leu
           195                 200                 205
   Val Asn Leu Leu Pro Cys Leu Thr Arg Thr Ser Lys Arg Pro Glu Glu
       210                 215                 220
   Ser Val Gln Glu Thr Leu Ala Ala Ala Val Pro Lys Ile Met Ala Ser
   225 230 235 240
   Phe Gly Asn Phe Ala Asn Asp Asn Glu Ile Lys Val Leu Leu Lys Ala
   245 250 255
   Phe Ile Ala Asn Leu Lys Ser Ser Ser Pro Thr Ile Arg Arg Thr Ala
   260 265 270
   Ala Gly Ser Ala Val Ser Ile Cys Gln His Ser Arg Arg Thr Gln Tyr
   275 280 285
   Phe Tyr Ser Trp Leu Leu Asn Val Leu Leu Gly Leu Leu Val Pro Val
   290 295 300
   Glu Asp Glu His Ser Thr Leu Leu Ile Leu Gly Val Leu Leu Thr Leu
   305 310 315 320
   Arg Tyr Leu Val Pro Leu Leu Gln Gln Gln Val Lys Asp Thr Ser Leu
   325 330 335
   Lys Gly Ser Phe Gly Val Thr Arg Lys Glu Met Glu Val Ser Pro Ser
   340 345 350
   Ala Glu Gln Leu Val Gln Val Tyr Glu Leu Thr Leu His His Thr Gln
   355 360 365
   His Gln Asp His Asn Val Val Thr Gly Ala Leu Glu Leu Leu Gln Gln
   370 375 380
   Leu Phe Arg Thr Pro Pro Pro Glu Leu Leu Gln Thr Leu Thr Ala Val
   385 390 395 400
   Gly Gly Ile Gly Gln Leu Thr Ala Ala Lys Glu Glu Ser Gly Gly Arg
   405 410 415
   Ser Arg Ser Gly Ser Ile Val Glu Leu Ile Ala Gly Gly Gly Ser Ser
   420 425 430
   Cys Ser Pro Val Leu Ser Arg Lys Gln Lys Gly Lys Val Leu Leu Gly
   435 440 445
   Glu Glu Glu Ala Leu Glu Asp Asp Ser Glu Ser Arg Ser Asp Val Ser
   450 455 460
   Ser Ser Ala Leu Thr Ala Ser Val Lys Asp Glu Ile Ser Gly Glu Leu
   465 470 475 480
   Ala Ala Ser Ser Gly Val Ser Thr Pro Gly Ser Ala Gly His Asp Ile
   485 490 495
   Ile Thr Glu Gln Pro Arg Ser Gln His Thr Leu Gln Ala Asp Ser Val
   500 505 510
   Asp Leu Ala Ser Cys Asp Leu Thr Ser Ser Ala Thr Asp Gly Asp Glu
   515 520 525
   Glu Asp Ile Leu Ser His Ser Ser Ser Gln Val Ser Ala Val Pro Ser
   530 535 540
   Asp Pro Ala Met Asp Leu Asn Asp Gly Thr Gln Ala Ser Ser Pro Ile
   545 550 555 560
   Ser Asp Ser Ser Gln Thr Thr Thr Glu Gly Pro Asp Ser Ala Val Thr
   565 570 575
   Pro Ser Asp Ser Ser Glu Ile Val Leu Asp Gly Thr Asp Asn Gln Tyr
   580 585 590
   Leu Gly Leu Gln Ile Gly Gln Pro Gln Asp Glu Asp Glu Glu Ala Thr
   595 600 605
   Gly Ile Leu Pro Asp Glu Ala Ser Glu Ala Phe Arg Asn Ser Ser Met
   610 615 620
   Ala Leu Gln Gln Ala His Leu Leu Lys Asn Met Ser His Cys Arg Gln
   625 630 635 640
   Pro Ser Asp Ser Ser Val Asp Lys Phe Val Leu Arg Asp Glu Ala Thr
   645 650 655
   Glu Pro Gly Asp Gln Glu Asn Lys Pro Cys Arg Ile Lys Gly Asp Ile
   660 665 670
   Gly Gln Ser Thr Asp Asp Ser Ala Pro Leu Val His Cys Val Arg Leu
   675 680 685
   Leu Ser Ala Ser Phe Leu Leu Thr Gly Gly Lys Asn Val Leu Val Pro
   690 695 700
   Asp Arg Asp Val Arg Val Ser Val Lys Ala Leu Ala Leu Ser Cys Val
   705 710 715 720
   Gly Ala Ala Val Ala Leu His Pro Glu Ser Phe Phe Ser Lys Leu Tyr
   725 730 735
   Lys Val Pro Leu Asp Thr Thr Glu Tyr Pro Glu Glu Gln Tyr Val Ser
   740 745 750
   Asp Ile Leu Asn Tyr Ile Asp His Gly Asp Pro Gln Val Arg Gly Ala
   755 760 765
   Thr Ala Ile Leu Cys Gly Thr Leu Ile Cys Ser Ile Leu Ser Arg Ser
   770 775 780
   Arg Phe His Val Gly Asp Trp Met Gly Thr Ile Arg Thr Leu Thr Gly
   785 790 795 800
   Asn Thr Phe Ser Leu Ala Asp Cys Ile Pro Leu Leu Arg Lys Thr Leu
   805 810 815
   Lys Asp Glu Ser Ser Val Thr Cys Lys Leu Ala Cys Thr Ala Val Arg
   820 825 830
   Asn Cys Val Met Ser Leu Cys Ser Ser Ser Tyr Ser Glu Leu Gly Leu
   835 840 845
   Gln Leu Ile Ile Asp Val Leu Thr Leu Arg Asn Ser Ser Tyr Trp Leu
   850 855 860
   Val Arg Thr Glu Leu Leu Glu Thr Leu Ala Glu Ile Asp Phe Arg Leu
   865 870 875 880
   Val Ser Phe Leu Glu Ala Lys Ala Glu Asn Leu His Arg Gly Ala His
   885 890 895
   His Tyr Thr Gly Leu Leu Lys Leu Gln Glu Arg Val Leu Asn Asn Val
   900 905 910
   Val Ile His Leu Leu Gly Asp Glu Asp Pro Arg Val Arg His Val Ala
   915 920 925
   Ala Ala Ser Leu Ile Arg Leu Val Pro Lys Leu Phe Tyr Lys Cys Asp
   930 935 940
   Gln Gly Gln Ala Asp Pro Val Val Ala Val Ala Arg Asp Gln Ser Ser
   945 950 955 960
   Val Tyr Leu Lys Leu Leu Met His Glu Thr Gln Pro Pro Ser His Phe
   965 970 975
   Ser Val Ser Thr Ile Thr Arg Ile Tyr Arg Gly Tyr Asn Leu Leu Pro
   980 985 990
   Ser Ile Thr Asp Val Thr Met Glu Asn Asn Leu Ser Arg Val Ile Ala
   995 1000 1005
   Ala Val Ser His Glu Leu Ile Thr Ser Thr Thr Arg Ala Leu Thr Phe
   1010 1015 1020
   Gly Cys Cys Glu Ala Leu Cys Leu Leu Ser Thr Ala Phe Pro Val Cys
   1025 1030 1035 1040
   Ile Trp Ser Leu Gly Trp His Cys Gly Val Pro Pro Leu Ser Ala Ser
   1045 1050 1055
   Asp Glu Ser Arg Lys Ser Cys Thr Val Gly Met Ala Thr Met Ile Leu
   1060 1065 1070
   Thr Leu Leu Ser Ser Ala Trp Phe Pro Leu Asp Leu Ser Ala His Gln
   1075 1080 1085
   Asp Ala Leu Ile Leu Ala Gly Asn Leu Leu Ala Ala Ser Ala Pro Lys
   1090 1095 1100
   Ser Leu Arg Ser Ser Trp Ala Ser Glu Glu Glu Ala Asn Pro Ala Ala
   1105 1110 1115 1120
   Thr Lys Gln Glu Glu Val Trp Pro Ala Leu Gly Asp Arg Ala Leu Val
   1125 1130 1135
   Pro Met Val Glu Gln Leu Phe Ser His Leu Leu Lys Val Ile Asn Ile
   1140 1145 1150
   Cys Ala His Val Leu Asp Asp Val Ala Pro Gly Pro Ala Ile Lys Ala
   1155 1160 1165
   Ala Leu Pro Ser Leu Thr Asn Pro Pro Ser Leu Ser Pro Ile Arg Arg
   1170 1175 1180
   Lys Gly Lys Glu Lys Glu Pro Gly Glu Gln Ala Ser Val Pro Leu Ser
   1185 1190 1195 1200
   Pro Lys Lys Gly Ser Glu Ala Ser Ala Ala Ser Arg Gln Ser Asp Thr
   1205 1210 1215
   Ser Gly Pro Val Thr Thr Ser Lys Ser Ser Ser Leu Gly Ser Phe Tyr
   1220 1225 1230
   His Leu Pro Ser Tyr Leu Lys Leu His Asp Val Leu Lys Ala Thr His
   1235 1240 1245
   Ala Asn Tyr Lys Val Thr Leu Asp Leu Gln Asn Ser Thr Glu Lys Phe
   1250 1255 1260
   Gly Gly Phe Leu Arg Ser Ala Leu Asp Val Leu Ser Gln Ile Leu Glu
   1265 1270 1275 1280
   Leu Ala Thr Leu Gln Asp Ile Gly Lys Cys Val Glu Glu Ile Leu Gly
   1285 1290 1295
   Tyr Leu Lys Ser Cys Phe Ser Arg Glu Pro Met Met Ala Thr Val Cys
   1300 1305 1310
   Val Gln Gln Leu Leu Lys Thr Leu Phe Gly Thr Asn Leu Ala Ser Gln
   1315 1320 1325
   Phe Asp Gly Leu Ser Ser Asn Pro Ser Lys Ser Gln Gly Arg Ala Gln
   1330 1335 1340
   Arg Leu Gly Ser Ser Ser Val Arg Pro Gly Leu Tyr His Tyr Cys Phe
   1345 1350 1355 1360
   Met Ala Pro Tyr Thr His Phe Thr Gln Ala Leu Ala Asp Ala Ser Leu
   1365 1370 1375
   Arg Asn Met Val Gln Ala Glu Gln Glu Asn Asp Thr Ser Gly Trp Phe
   1380 1385 1390
   Asp Val Leu Gln Lys Val Ser Thr Gln Leu Lys Thr Asn Leu Thr Ser
   1395 1400 1405
   Val Thr Lys Asn Arg Ala Asp Lys Asn Ala Ile His Asn His Ile Arg
   1410 1415 1420
   Leu Phe Glu Pro Leu Val Ile Lys Ala Leu Lys Gln Tyr Thr Thr Thr
   1425 1430 1435 1440
   Thr Cys Val Gln Leu Gln Lys Gln Val Leu Asp Leu Leu Ala Gln Leu
   1445 1450 1455
   Val Gln Leu Arg Val Asn Tyr Cys Leu Leu Asp Ser Asp Gln Val Phe
   1460 1465 1470
   Ile Gly Phe Val Leu Lys Gln Phe Glu Tyr Ile Glu Val Gly Gln Phe
   1475 1480 1485
   Arg Glu Ser Glu Ala Ile Ile Pro Asn Ile Phe Phe Phe Leu Val Leu
   1490 1495 1500
   Leu Ser Tyr Glu Arg Tyr His Ser Lys Gln Ile Ile Gly Ile Pro Lys
   1505 1510 1515 1520
   Ile Ile Gln Leu Cys Asp Gly Ile Met Ala Ser Gly Arg Lys Ala Val
   1525 1530 1535
   Thr His Ala Ile Pro Ala Leu Gln Pro Ile Val His Asp Leu Phe Val
   1540 1545 1550
   Leu Arg Gly Thr Asn Lys Ala Asp Ala Gly Lys Glu Leu Glu Thr Gln
   1555 1560 1565
   Lys Glu Val Val Val Ser Met Leu Leu Arg Leu Ile Gln Tyr His Gln
   1570 1575 1580
   Val Leu Glu Met Phe Ile Leu Val Leu Gln Gln Cys His Lys Glu Asn
   1585 1590 1595 1600
   Glu Asp Lys Trp Lys Arg Leu Ser Arg Gln Ile Ala Asp Ile Ile Leu
   1605 1610 1615
   Pro Met Leu Ala Lys Gln Gln Met His Ile Asp Ser His Glu Ala Leu
   1620 1625 1630
   Gly Val Leu Asn Thr Leu Phe Glu Ile Leu Ala Pro Ser Ser Leu Arg
   1635 1640 1645
   Pro Val Asp Met Leu Leu Arg Ser Met Phe Val Thr Pro Asn Thr Met
   1650 1655 1660
   Ala Ser Val Ser Thr Val Gln Leu Trp Ile Ser Gly Ile Leu Ala Ile
   1665 1670 1675 1680
   Leu Arg Val Leu Ile Ser Gln Ser Thr Glu Asp Ile Val Leu Ser Arg
   1685 1690 1695
   Ile Gln Glu Leu Ser Phe Ser Pro Tyr Leu Ile Ser Cys Thr Val Ile
   1700 1705 1710
   Asn Arg Leu Arg Asp Gly Asp Ser Thr Ser Thr Leu Glu Glu His Ser
   1715 1720 1725
   Glu Gly Lys Gln Ile Lys Asn Leu Pro Glu Glu Thr Phe Ser Arg Phe
   1730 1735 1740
   Leu Leu Gln Leu Val Gly Ile Leu Leu Glu Asp Ile Val Lys Gln Leu
   1745 1750 1755 1760
   Lys Val Met Ser Gln Gln His Thr Phe Tyr Cys Gln Glu Leu Gly Thr
   1765 1770 1775
   Leu Leu Met Cys Leu Ile His Ile Phe Lys Ser Gly Met Phe Arg Arg
   1780 1785 1790
   Ile Thr Ala Ala Ala Thr Arg Leu Phe Arg Ser Asp Gly Cys Gly Gly
   1795 1800 1805
   Ser Phe Tyr Thr Leu Asp Ser Leu Asn Leu Arg Ala Arg Ser Met Ile
   1810 1815 1820
   Thr Thr His Pro Ala Leu Val Leu Leu Trp Cys Gln Ile Leu Leu Leu
   1825 1830 1835 1840
   Val Asn His Thr Asp Tyr Arg Trp Trp Ala Glu Val Gln Gln Thr Pro
   1845 1850 1855
   Lys Arg His Ser Leu Ser Ser Thr Lys Leu Leu Ser Pro Gln Met Ser
   1860 1865 1870
   Gly Glu Glu Glu Asp Ser Asp Leu Ala Ala Lys Leu Gly Met Cys Asn
   1875 1880 1885
   Arg Glu Ile Val Arg Arg Gly Ala Leu Ile Leu Phe Cys Asp Tyr Val
   1890 1895 1900
   Cys Gln Asn Leu His Asp Ser Glu His Leu Thr Trp Leu Ile Val Asn
   1905 1910 1915 1920
   His Ile Gln Asp Leu Ile Ser Leu Ser His Glu Pro Pro Val Gln Asp
   1925 1930 1935
   Phe Ile Ser Ala Val His Arg Asn Ser Ala Ala Ser Gly Leu Phe Ile
   1940 1945 1950
   Gln Ala Ile Gln Ser Arg Cys Glu Asn Leu Ser Thr Pro Thr Met Leu
   1955 1960 1965
   Lys Lys Thr Leu Gln Cys Leu Glu Gly Ile His Leu Ser Gln Ser Gly
   1970 1975 1980
   Ala Val Leu Thr Leu Tyr Val Asp Arg Leu Leu Cys Thr Pro Phe Arg
   1985 1990 1995 2000
   Val Leu Ala Arg Met Val Asp Ile Leu Ala Cys Arg Arg Val Glu Met
   2005 2010 2015
   Leu Leu Ala Ala Asn Gln Ser Ser Met Ala Gln Leu Pro Met Glu Glu
   2020 2025 2030
   Leu Asn Arg Ile Gln Glu Tyr Leu Gln Ser Ser Gly Leu Ala Gln Arg
   2035 2040 2045
   His Gln Arg Leu Tyr Ser Leu Leu Asp Arg Phe Arg Leu Ser Thr Met
   2050 2055 2060
   Gln Asp Ser Leu Ser Pro Ser Pro Pro Val Ser Ser His Pro Leu Asp
   2065 2070 2075 2080
   Gly Asp Gly His Val Ser Leu Glu Thr Val Ser Pro Asp Lys Asp Trp
   2085 2090 2095
   Tyr Val His Leu Val Lys Ser Gln Cys Trp Thr Arg Ser Asp Ser Ala
   2100 2105 2110
   Leu Leu Glu Gly Ala Glu Leu Val Asn Arg Ile Pro Ala Glu Asp Met
   2115 2120 2125
   Asn Ala Phe Met Met Asn Ser Glu Phe Asn Leu Ser Leu Leu Ala Pro
   2130 2135 2140
   Cys Leu Ser Leu Gly Met Ser Glu Ile Ser Gly Gly Gln Lys Ser Ala
   2145 2150 2155 2160
   Leu Phe Glu Ala Ala Arg Glu Val Thr Leu Ala Arg Val Ser Gly Thr
   2165 2170 2175
   Val Gln Gln Leu Pro Ala Val His His Val Phe Gln Pro Glu Leu Pro
   2180 2185 2190
   Ala Glu Pro Ala Ala Tyr Trp Ser Lys Leu Asn Asp Leu Phe Gly Asp
   2195 2200 2205
   Ala Ala Leu Tyr Gln Ser Leu Pro Thr Leu Ala Arg Ala Leu Ala Gln
   2210 2215 2220
   Tyr Leu Val Val Val Ser Lys Leu Pro Ser His Leu His Leu Pro Pro
   2225 2230 2235 2240
   Glu Lys Glu Lys Asp Ile Val Lys Phe Val Val Ala Thr Leu Glu Ala
   2245 2250 2255
   Leu Ser Trp His Leu Ile His Glu Gln Ile Pro Leu Ser Leu Asp Leu
   2260 2265 2270
   Gln Ala Gly Leu Asp Cys Cys Cys Leu Ala Leu Gln Leu Pro Gly Leu
   2275 2280 2285
   Trp Ser Val Val Ser Ser Thr Glu Phe Val Thr His Ala Cys Ser Leu
   2290 2295 2300
   Ile Tyr Cys Val His Phe Ile Leu Glu Ala Val Ala Val Gln Pro Gly
   2305 2310 2315 2320
   Glu Gln Leu Leu Ser Pro Glu Arg Arg Thr Asn Thr Pro Lys Ala Ile
   2325 2330 2335
   Ser Glu Glu Glu Glu Glu Val Asp Pro Asn Thr Gln Asn Pro Lys Tyr
   2340 2345 2350
   Ile Thr Ala Ala Cys Glu Met Val Ala Glu Met Val Glu Ser Leu Gln
   2355 2360 2365
   Ser Val Leu Ala Leu Gly His Lys Arg Asn Ser Gly Val Pro Ala Phe
   2370 2375 2380
   Leu Thr Pro Leu Leu Arg Asn Ile Ile Ile Ser Leu Ala Arg Leu Pro
   2385 2390 2395 2400
   Leu Val Asn Ser Tyr Thr Arg Val Pro Pro Leu Val Trp Lys Leu Gly
   2405 2410 2415
   Trp Ser Pro Lys Pro Gly Gly Asp Phe Gly Thr Ala Phe Pro Glu Ile
   2420 2425 2430
   Pro Val Glu Phe Leu Gln Glu Lys Glu Val Phe Lys Glu Phe Ile Tyr
   2435 2440 2445
   Arg Ile Asn Thr Leu Gly Trp Thr Ser Arg Thr Gln Phe Glu Glu Thr
   2450 2455 2460
   Trp Ala Thr Leu Leu Gly Val Leu Val Thr Gln Pro Leu Val Met Glu
   2465 2470 2475 2480
   Gln Glu Glu Ser Pro Pro Glu Glu Asp Thr Glu Arg Thr Gln Ile Asn
   2485 2490 2495
   Val Leu Ala Val Gln Ala Ile Thr Ser Leu Val Leu Ser Ala Met Thr
   2500 2505 2510
   Val Pro Val Ala Gly Asn Pro Ala Val Ser Cys Leu Glu Gln Gln Pro
   2515 2520 2525
   Arg Asn Lys Pro Leu Lys Ala Leu Asp Thr Arg Phe Gly Arg Lys Leu
   2530 2535 2540
   Ser Ile Ile Arg Gly Ile Val Glu Gln Glu Ile Gln Ala Met Val Ser
   2545 2550 2555 2560
   Lys Arg Glu Asn Ile Ala Thr His His Leu Tyr Gln Ala Trp Asp Pro
   2565 2570 2575
   Val Pro Ser Leu Ser Pro Ala Thr Thr Gly Ala Leu Ile Ser His Glu
   2580 2585 2590
   Lys Leu Leu Leu Gln Ile Asn Pro Glu Arg Glu Leu Gly Ser Met Ser
   2595 2600 2605
   Tyr Lys Leu Gly Gln Val Ser Ile His Ser Val Trp Leu Gly Asn Ser
   2610 2615 2620
   Ile Thr Pro Leu Arg Glu Glu Glu Trp Asp Glu Glu Glu Glu Glu Glu
   2625 2630 2635 2640
   Ala Asp Ala Pro Ala Pro Ser Ser Pro Pro Thr Ser Pro Val Asn Ser
   2645 2650 2655
   Arg Lys His Arg Ala Gly Val Asp Ile His Ser Cys Ser Gln Phe Leu
   2660 2665 2670
   Leu Glu Leu Tyr Ser Arg Trp Ile Leu Pro Ser Ser Ser Ala Arg Arg
   2675 2680 2685
   Thr Pro Ala Ile Leu Ile Ser Glu Val Val Arg Ser Leu Leu Val Val
   2690 2695 2700
   Ser Asp Leu Phe Thr Glu Arg Asn Gln Phe Glu Leu Met Tyr Val Thr
   2705 2710 2715 2720
   Leu Thr Glu Leu Arg Arg Val His Pro Ser Glu Asp Glu Ile Leu Ala
   2725 2730 2735
   Gln Tyr Leu Val Pro Ala Thr Cys Lys Ala Ala Ala Val Leu Gly Met
   2740 2745 2750
   Asp Lys Ala Val Ala Glu Pro Val Ser Arg Leu Leu Glu Ser Thr Leu
   2755 2760 2765
   Arg Ser Ser His Leu Pro Ser Arg Val Gly Ala Leu His Gly Val Leu
   2770 2775 2780
   Tyr Val Leu Glu Cys Asp Leu Leu Asp Asp Thr Ala Lys Gln Leu Ile
   2785 2790 2795 2800
   Pro Val Ile Ser Asp Tyr Leu Leu Ser Asn Leu Lys Gly Ile Ala His
   2805 2810 2815
   Cys Val Asn Ile His Ser Gln Gln His Val Leu Val Met Cys Ala Thr
   2820 2825 2830
   Ala Phe Tyr Leu Ile Glu Asn Tyr Pro Leu Asp Val Gly Pro Glu Phe
   2835 2840 2845
   Ser Ala Ser Ile Ile Gln Met Cys Gly Val Met Leu Ser Gly Ser Glu
   2850 2855 2860
   Glu Ser Thr Pro Ser Ile Ile Tyr His Cys Ala Leu Arg Gly Leu Glu
   2865 2870 2875 2880
   Arg Leu Leu Leu Ser Glu Gln Leu Ser Arg Leu Asp Ala Glu Ser Leu
   2885 2890 2895
   Val Lys Leu Ser Val Asp Arg Val Asn Val His Ser Pro His Arg Ala
   2900 2905 2910
   Met Ala Ala Leu Gly Leu Met Leu Thr Cys Met Tyr Thr Gly Lys Glu
   2915 2920 2925
   Lys Val Ser Pro Gly Arg Thr Ser Asp Pro Asn Pro Ala Ala Pro Asp
   2930 2935 2940
   Ser Glu Ser Val Ile Val Ala Met Glu Arg Val Ser Val Leu Phe Asp
   2945 2950 2955 2960
   Arg Ile Arg Lys Gly Phe Pro Cys Glu Ala Arg Val Val Ala Arg Ile
   2965 2970 2975
   Leu Pro Gln Phe Leu Asp Asp Phe Phe Pro Pro Gln Asp Ile Met Asn
   2980 2985 2990
   Lys Val Ile Gly Glu Phe Leu Ser Asn Gln Gln Pro Tyr Pro Gln Phe
   2995 3000 3005
   Met Ala Thr Val Val Tyr Lys Val Phe Gln Thr Leu His Ser Thr Gly
   3010 3015 3020
   Gln Ser Ser Met Val Arg Asp Trp Val Met Leu Ser Leu Ser Asn Phe
   3025 3030 3035 3040
   Thr Gln Arg Ala Pro Val Ala Met Ala Thr Trp Ser Leu Ser Cys Phe
   3045 3050 3055
   Phe Val Ser Ala Ser Thr Ser Pro Trp Val Ala Ala Ile Leu Pro His
   3060 3065 3070
   Val Ile Ser Arg Met Gly Lys Leu Glu Gln Val Asp Val Asn Leu Phe
   3075 3080 3085
   Cys Leu Val Ala Thr Asp Phe Tyr Arg His Gln Ile Glu Glu Glu Leu
   3090 3095 3100
   Asp Arg Arg Ala Phe Gln Ser Val Leu Glu Val Val Ala Ala Pro Gly
   3105                3110                3115                3120
   Ser Pro Tyr His Arg Leu Leu Thr Cys Leu Arg Asn Val His Lys Val
                   3125                3130                3135
   Thr Thr Cys
<210> 17
   <211> 512
   <212> DNA
   <213> Artificial Sequence
<220>
   <223> Description of Artificial Sequence; note = synthetic construct
<400> 17
   agcaatgttt tcctgtgttg tttatgctta aaaagcgatt ttaggcgtta ttaaggacgc      60
   gaaaatggac aaatccaggt ccagtgcgta cgacaagttc gttggcttcg tggaacaact     120
   gcgaaatacg gaatgcagcc aaaagcagaa gatcacctgc tttcagcaga ttgccgagtg     180
   catcatgtcg ccctcgctgg cgggccacat caactatgcc gcccactgcg gcacggccac     240
   gaatgttctg ctgctgttct gcgaggatgt ggattccgtg gttcggatga gcgccgagga     300
   aaacctaaat aaaatactga ggtcactcga gaaaacccgg gtcagtcgca ttcttatgga     360
   tctgtatggt gaaattaagc gtaatggtaa ccagagatcc ctgaggattt gcctaaacct     420
   tttctcctac tatgctccac aaataaagga gaaacacatc aagtggtatg ccgtgagatt     480
   gctccaatgt atgaccacca tttcccagcg ca                                   512
<210> 18
   <211> 154
   <212> PRT
   <213> Artificial Sequence
<220>
   <223> Description of Artificial Sequence; note = synthetic construct
<400> 18
<210> 19
   <211> 714
   <212> DNA
   <213> Artificial Sequence
<220>
   <223> Description of Artificial Sequence; note = synthetic construct
<400> 19
   ctacggtcac accatagaag atacctggaa agttctcaac ttttttcgtt ttgataaatt      60
   gattaattca ttcgaaatgg tggttaaagc tgtctgcgta attaacggcg atgccaaggg     120
   cacggttttc ttcgaacagg agagcagcgg tacgcccgtg aaggtctccg gtgaggtgtg     180
   cggcctggcc aagggtctgc acggattcca cgtgcacgag ttcggtgaca acaccaatgg     240
   ctgcatgtcg tccggaccgc acttcaatcc gtatggcaag gagcatggcg ctcccgtcga     300
   cgagaatcgt cacctgggcg atctgggcaa cattgaggcc accggcgact gccccaccaa     360
   ggtcaacatc accgactcca agattacgct cttcggcgcc gacagcatca tcggacgcac     420
   cgttgtcgtg cacgccgatg ccgatgatct tggccagggt ggacacgagc tgagcaagtc     480
   aacgggcaac gctggtgccc gcatcgggtg cggcgttatt ggcattgcca aggtctaagc     540
   gataatctat tccgatgtcg gccactgtgc tgatctactc tatttagcac tacccactgg     600
   agatatacaa acgatataca tacttctaaa cataaataca tagcctgtgg tctgttagtt     660
   gatacgcaac ctttgaggtt caataaattg gtgttttgaa attgccccat aaac           714
<210> 20
   <211> 352
   <212> PRT
   <213> Artificial Sequence
<220>
   <223> Description of Artificial Sequence; note = synthetic construct
<400> 20
   Met Ala Glu Pro Arg Gln Glu Phe Glu Val Met Glu Asp His Ala Gly
    1               5                  10                  15
   Thr Tyr Gly Leu Gly Asp Arg Lys Asp Gln Gly Gly Tyr Thr Met His
               20                  25                  30
   Gln Asp Gln Glu Gly Asp Thr Asp Ala Gly Leu Lys Ala Glu Glu Ala
           35                  40                  45
   Gly Ile Gly Asp Thr Pro Ser Leu Glu Asp Glu Ala Ala Gly His Val
       50                  55                  60
   Thr Gln Ala Arg Met Val Ser Lys Ser Lys Asp Gly Thr Gly Ser Asp
   65                  70                  75                  80
   Asp Lys Lys Ala Lys Gly Ala Asp Gly Lys Thr Lys Ile Ala Thr Pro
                   85                  90                  95
   Arg Gly Ala Ala Pro Pro Gly Gln Lys Gly Gln Ala Asn Ala Thr Arg
               100                 105                 110
   Ile Pro Ala Lys Thr Pro Pro Ala Pro Lys Thr Pro Pro Ser Ser Gly
           115                 120                 125
   Glu Pro Pro Lys Ser Gly Asp Arg Ser Gly Tyr Ser Ser Pro Gly Ser
       130                 135                 140
   Pro Gly Thr Pro Gly Ser Arg Ser Arg Thr Pro Ser Leu Pro Thr Pro
   145                 150                 155                 160
   Pro Thr Arg Glu Pro Lys Lys Val Ala Val Val Arg Thr Pro Pro Lys
                   165                 170                 175
   Ser Pro Ser Ser Ala Lys Ser Arg Leu Gln Thr Ala Pro Val Pro Met
               180                 185                 190
   Pro Asp Leu Lys Asn Val Lys Ser Lys Ile Gly Ser Thr Glu Asn Leu
           195                 200                 205
   Lys His Gln Pro Gly Gly Gly Lys Val Gln Ile Val Tyr Lys Pro Val
       210                 215                 220
   Asp Leu Ser Lys Val Thr Ser Lys Cys Gly Ser Leu Gly Asn Ile His
   225                 230                 235                 240
   His Lys Pro Gly Gly Gly Gln Val Glu Val Lys Ser Glu Lys Leu Asp
                   245                 250                 255
   Phe Lys Asp Arg Val Gln Ser Lys Ile Gly Ser Leu Asp Asn Ile Thr
               260                 265                 270
   His Val Pro Gly Gly Gly Asn Lys Lys Ile Glu Thr His Lys Leu Thr
           275                 280                 285
   Phe Arg Glu Asn Ala Lys Ala Lys Thr Asp His Gly Ala Glu Ile Val
       290                 295                 300
   Tyr Lys Ser Pro Val Val Ser Gly Asp Thr Ser Pro Arg His Leu Ser
   305                 310                 315                 320
   Asn Val Ser Ser Thr Gly Ser Ile Asp Met Val Asp Ser Pro Gln Leu
                   325                 330                 335
   Ala Thr Leu Ala Asp Glu Val Ser Ala Ser Leu Ala Lys Gln Gly Leu
               340                 345                 350
<210> 21
   <211> 360
   <212> PRT
   <213> Artificial Sequence
<220>
   <223> Description of Artificial Sequence; note = synthetic construct
<400> 21
   Met Ala Asp Val Leu Glu Lys Ser Ser Leu Leu Asp Ala Val Pro Pro
    1               5                  10                  15
   Leu Gly Asp Pro His Pro Pro Leu Pro His Gln Gln Leu Gln Gln Glu
               20                  25                  30
   Ala Ala Ala Ala Ala Ala Ala Asn Ala Ala Pro Pro Ala Pro Pro Gln
           35                  40                  45
   Gln Gln Gln Pro Pro Pro His Gln Leu Gln Gln Gln Gln Pro Gln Gln
       50                  55                  60
   Gln Gln Leu Gln Gln Lys Pro Ala Asn Ala Arg Ala Asn Gln Asp Gln
   65                  70                  75                  80
   Lys Glu Gly Asp Asn Asp Ser Gly Val Asp Glu Ser Thr Gln Glu Lys
                   85                  90                  95
   Asp Arg Asn Gly Pro Asn Ser Pro Ser Ser Pro Val Lys Thr Pro Thr
               100                 105                 110
   Ser Thr Ser Ser Lys Pro Asp Lys Ser Gly Thr Ser Arg Pro Pro Ser
           115                 120                 125
   Ala Thr Pro Ser Asn Lys Ser Ala Pro Lys Ser Arg Ser Ala Ser Lys
       130                 135                 140
   Asn Arg Leu Leu Leu Lys Thr Pro Glu Pro Glu Pro Val Lys Lys Val
   145                 150                 155                 160
   Pro Met Asn Lys Val Gln Val Gly His Ala Pro Ser Pro Asn Leu Lys
                   165                 170                 175
   Ala Val Arg Ser Lys Ile Gly Ser Leu Asp Asn Ala Thr Tyr Lys Pro
               180                 185                 190
   Gly Gly Gly His Val Lys Ile Glu Ser Lys Lys Ile Asp Ile Lys Ala
           195                 200                 205
   Ala Pro Arg Ile Glu Ala Lys Asn Asp Lys Tyr Met Pro Lys Gly Gly
       210                 215                 220
   Glu Lys Lys Ile Val Thr Thr Lys Leu Gln Trp Asn Ala Lys Ser Lys
   225                 230                 235                 240
   Ile Gly Ser Leu Glu Asn Ala Ala His Lys Pro Gly Gly Gly Asp Lys
                   245                 250                 255
   Lys Ile Glu Thr Leu Lys Met Asp Phe Lys Asp Lys Ala Lys Pro Lys
               260                 265                 270
   Val Gly Ser Thr Ala Asn Val Lys His Gln Pro Gly Gly Gly Asp Ile
           275                 280                 285
   Lys Ile Gln Thr Gln Lys Leu Glu Ile Lys Ala Gln Ser Lys Val Gly
       290                 295                 300
   Ser Leu Asp Asn Val Lys His Lys Pro Gly Gly Gly Glu Lys Lys Ile
   305                 310                 315                 320
   Phe Asp Asp Lys Asp Tyr Leu Lys Asn Val Glu His Ser Val Ala Leu
                   325                 330                 335
   Thr Thr Pro Pro Thr Gln Ser Pro Leu Pro Ser Met Thr Ala Ser Gly
               340                 345                 350
   Ala Asp Glu Asn Leu Asn Gln Gln
           355                 360
<210> 22
   <211> 585
   <212> PRT
   <213> Artificial Sequence
<220>
   <223> Description of Artificial Sequence; note = synthetic construct
<400> 22
<210> 23
   <211> 721
   <212> PRT
   <213> Artificial Sequence
<220>
   <223> Description of Artificial Sequence; note = synthetic construct
<400> 23
   Met Thr Ile Thr Tyr Thr Gly Glu Val Ala Thr Cys Arg Gly Phe Gly
    1               5                  10                  15
   Cys Phe Leu Lys Leu Leu Leu Arg Trp Arg Gly Ser Ile Tyr Lys Leu
               20                  25                  30
   Val Trp Leu Asp Leu Leu Ala Phe Leu Thr Ile Tyr Tyr Ala Ile Asn
           35                  40                  45
   Met Val Tyr Arg Phe Gly Leu Asn Pro Ala Gln Lys Glu Thr Phe Glu
       50                  55                  60
   Ala Ile Val Gln Tyr Cys Asp Ser Tyr Arg Glu Leu Ile Pro Leu Ser
   65                  70                  75                  80
   Phe Val Leu Gly Phe Tyr Val Ser Ile Val Met Thr Arg Trp Trp Asn
                   85                  90                  95
   Gln Tyr Thr Ser Ile Pro Trp Pro Asp Pro Ile Ala Val Phe Val Ser
               100                 105                 110
   Ser Asn Val His Gly Gln Asp Glu Arg Gly Arg Met Met Arg Arg Thr
           115                 120                 125
   Ile Met Arg Tyr Val Cys Leu Cys Leu Thr Met Val Leu Ala Asn Val
       130                 135                 140
   Ser Pro Arg Val Lys Lys Arg Phe Pro Gly Leu Asn Asn Leu Val Glu
   145                 150                 155                 160
   Ala Gly Leu Leu Asn Asp Asn Glu Lys Thr Ile Ile Glu Thr Met Asn
                   165                 170                 175
   Lys Ala Phe Pro Arg Pro Ser Lys His Trp Leu Pro Ile Val Trp Ala
               180                 185                 190
   Ala Ser Ile Ile Thr Arg Ala Arg Lys Glu Gly Arg Ile Arg Asp Asp
           195                 200                 205
   Phe Ala Val Lys Thr Ile Ile Asp Glu Leu Asn Lys Phe Arg Gly Gln
       210                 215                 220
   Cys Gly Leu Leu Ile Ser Tyr Asp Thr Ile Ser Val Pro Leu Val Tyr
   225                 230                 235                 240
   Thr Gln Val Val Thr Leu Ala Val Tyr Ser Tyr Phe Leu Thr Cys Cys
                   245                 250                 255
   Met Gly Gln Gln Trp Thr Asp Gly Lys Val Val Gly Asn Thr Thr Tyr
               260                 265                 270
   Leu Asn Lys Val Asp Leu Tyr Phe Pro Val Phe Thr Thr Leu Gln Phe
           275                 280                 285
   Phe Phe Tyr Met Gly Trp Leu Lys Val Ala Glu Ser Leu Ile Asn Pro
       290                 295                 300
   Phe Gly Glu Asp Asp Asp Asp Phe Glu Val Asn Trp Met Val Asp Arg
   305                 310                 315                 320
   Asn Leu Gln Val Ser Tyr Leu Ile Val Asp Glu Met His His Asp His
                   325                 330                 335
   Pro Glu Leu Leu Lys Asp Gln Tyr Trp Asp Glu Val Phe Pro Asn Glu
               340                 345                 350
   Leu Pro Tyr Thr Ile Ala Ala Glu Arg Phe Arg Glu Asn His Pro Glu
           355                 360                 365
   Pro Ser Thr Ala Lys Ile Glu Val Pro Lys Asn Ala Ala Met Pro Ser
       370                 375                 380
   Thr Met Ser Ser Val Arg Ile Asp Glu Met Ala Asp Asp Ala Ser Gly
   385                 390                 395                 400
   Ile His Phe Ser Ala Gly Asn Gly Lys Met Arg Leu Asp Ser Ser Pro
                   405                 410                 415
   Ser Leu Val Ser Val Ser Gly Thr Leu Ser Arg Val Asn Thr Val Ala
               420                 425                 430
   Ser Ala Leu Lys Arg Phe Leu Ser Arg Asp Asp Ser Arg Pro Gly Ser
           435                 440                 445
   Ala Thr Pro Ser Gln Asp Gln Pro Tyr Lys Phe Pro Ala Ser Ala Ser
       450                 455                 460
   Ser Ala Ser Leu Ser Gly Ala Val Val Gly Ser Ala Thr Ser Ala Gly
   465                 470                 475                 480
   Lys Pro Ala Gly Ser Leu Arg Ile Thr Gln Gln Val Ile Glu Glu Val
                   485                 490                 495
   Asp Glu Gln Ala Thr Ile Thr Ser Met Arg Ala Asn Asp Pro Arg Pro
               500                 505                 510
   Asn Val Met Asp Ile Phe Ala Gln Thr Ser Ser Gly Ala Gly Thr Ser
           515                 520                 525
   Gly Pro Leu Gln Pro Pro Pro Ala His Ser Glu Pro Val Asp Ile Pro
       530                 535                 540
   Ser Arg Pro Pro Ser Tyr Asn Arg Ala Gln Ser Gln Tyr Glu Pro Asn
   545                 550                 555                 560
   Leu Phe Pro Pro Gly Gly Val Asp Ala Leu Leu Ser Thr Ser Ala Pro
                   565                 570                 575
   Ala Gly Gly Ser Pro Leu Leu Leu Ser Asn Ala Ala Thr Ala Pro Ser
               580                 585                 590
   Ser Pro Val Gly Glu Ser Ser Lys Ser Leu Tyr Asp Pro Gln Lys Gly
           595                 600                 605
   Ala Ser Arg Glu Thr Val Glu Ser Met Asp Leu Arg Ser Ser Thr Asp
       610                 615                 620
   Leu Leu Gly Asp Ala Ala Val Gln Pro Glu Asp Glu Gly Asp Asp Phe
   625                 630                 635                 640
   Asp Lys Leu Lys Ala Glu Arg Glu Lys Glu Lys Leu Met Arg Gln Gln
                   645                 650                 655
   Lys Asn Leu Ala Arg Thr Ile Ser Thr Ala Pro Gly Met Glu Ala Thr
               660                 665                 670
   Ala Val Pro Met Val Pro Met Val Pro Val Asn Val Ala Val Gln Gln
           675                 680                 685
   Ala Gln Leu Gln Pro Val Ala Ser Ser Ala Asp Leu Leu Ala Gly Gly
       690                 695                 700
   Asp Gln Phe Ser Asn Ser Thr Met Lys Ser Glu Asp Ala Ile Asn Gly
   705                 710                 715                 720
   Ser
<210> 24
   <211> 890
   <212> PRT
   <213> Artificial Sequence
<220>
   <223> Description of Artificial Sequence; note = synthetic construct
<400> 24
<210> 25
   <211> 813
   <212> PRT
   <213> Artificial Sequence
<220>
   <223> Description of Artificial Sequence; note = synthetic construct
<400> 25
   Met Gln Tyr Asp Leu Ala Lys Glu Leu Leu Asp Asn Ser Ile Val Glu
    1               5                  10                  15
   Pro Asn Leu Ala Lys Gly Asn Gln Ser Gln Lys Ala Val Asn Trp Leu
               20                  25                  30
   Val Ser Ala Ala His Asn Gly His Glu Asp Ala Val Lys Leu Leu Arg
           35                  40                  45
   Gln Cys Tyr Asn Asp Gly Ser Gly Ile Thr Ala Glu Asn Thr Asp Glu
       50                  55                  60
   Val Arg Arg Cys Leu Ala Met Thr Pro Gly Glu Arg Ala Ala Arg Lys
   65                  70                  75                  80
   Ala Ala Arg Glu Leu Phe Ala Cys Leu Ser Asn Gly Asn Glu His Ile
                   85                  90                  95
   Thr Pro Lys Gln Leu Glu Arg Lys Met Arg Arg Ile Tyr Asn Leu Gln
               100                 105                 110
   Arg Lys Arg Arg Arg Arg Asp Asp Asp Arg Ser Ser Ser Ser Ser Glu
           115                 120                 125
   Gly Glu Gln Glu Pro Glu Cys Glu Pro Leu Glu Asp Val Pro Thr Ile
       130                 135                 140
   Asp Leu Ala Asn Val Glu Arg Arg Arg Leu Ile Thr Glu Ala His Leu
   145                 150                 155                 160
   Val Ser Ala Ala Ser Asn Tyr Ser Ala Gly Gln Met Pro Ser Val Asn
                   165                 170                 175
   Asp Ala Leu Thr Leu Ser Val Pro Asp Pro Arg Ser Leu Asp His Val
               180                 185                 190
   Pro Cys Phe Tyr Arg Met Ile Phe His Pro Leu Ile Phe Phe Thr Leu
           195                 200                 205
   Phe Tyr His Arg Leu Leu Asn Leu Ile Val Ser Ile Pro Asn Val Ile
       210                 215                 220
   Pro Leu Ser Val Arg Cys Ser Val Leu Val Ala Ile Ser Trp Trp Ser
   225                 230                 235                 240
   Ser Arg His Met Leu Pro Leu Val Ser Tyr Tyr Leu Ser Leu Gly Ile
                   245                 250                 255
   Met Ile Trp Ala Thr Cys Lys Met Leu Lys Thr Lys Gln Gln Phe Val
               260                 265                 270
   Asp Phe Arg Ile Trp Ser Gly Leu Phe Leu Ser Tyr Gly Asp Gln Asn
           275                 280                 285
   Ile Glu Ala Asp Ile Ala Glu His Arg Phe Leu Arg Asn Asn Met Lys
       290                 295                 300
   Pro Tyr Leu Tyr Phe Phe Cys Ala Phe Ile Cys Asn Leu Ile Val Tyr
   305                 310                 315                 320
   Pro Leu Val Thr Asp Ala Trp Leu Pro His Ser Glu Leu Thr Ile Ile
                   325                 330                 335
   Ser Gly Ala Leu Thr Phe Ile Thr Met Cys Val Ser Met Tyr Ala Ser
               340                 345                 350
   Ser His Gln Leu Pro Asp Trp Leu Val Ile Val Ser Phe Ala Val Asn
           355                 360                 365
   Val Leu Ala Lys Tyr Pro Tyr Glu Met Asp Glu Val Val Ser Thr Arg
       370                 375                 380
   Trp Arg Phe Leu Asp Leu Arg Val Pro Thr Phe Ser Ser Phe Val Ile
   385                 390                 395                 400
   Gly Asn Gly Ile Glu Phe Cys Leu Asn Cys Arg Thr Ala Leu Tyr Leu
                   405                 410                 415
   Phe Ile Pro Val Leu Leu Ile Met Met Ala Lys Arg Ser Arg Trp His
               420                 425                 430
   Gly Val Tyr Thr Phe Leu Ile Pro His Cys Val Thr Leu Ser Trp Leu
           435                 440                 445
   Gln Val Cys Ile Ala Thr Ser Gln Ser Ser Thr Val Phe Gly Val Met
       450                 455                 460
   Arg Ala Ala Leu Gly Leu Ala Gly Ile Val Leu Phe Leu Pro Leu Phe
   465                 470                 475                 480
   Gly Ile Val Ala Leu Leu Val Pro Val Phe Val Ala Ile Asp Ser Leu
                   485                 490                 495
   Gly Leu Ala Ser Glu Gln Leu Arg Trp Gly Ser Thr Ala Leu Ala Cys
               500                 505                 510
   Gly Leu Val Val Val Leu Ser Cys Ile Leu Ala Leu Asn Arg Ala Thr
           515                 520                 525
   Gln Lys Tyr Ile Thr Met Leu Gln Leu Ile Thr Ala Ile Thr Thr Ala
       530                 535                 540
   Cys Leu Leu Val Leu Pro Tyr Met Thr Ser Ser Phe Lys Asp Thr Pro
   545                 550                 555                 560
   Arg Phe Asn Ala Met Pro Arg Ala Gly Leu His Ser Leu Ser Glu Thr
                   565                 570                 575
   Asn Thr Leu Pro Trp Asp Arg Phe His Ala Leu Cys Ala Gln Pro Val
               580                 585                 590
   His Glu Gln Pro Asn Lys Ile Lys Ala Gln Leu Arg Cys Ser Leu Leu
           595                 600                 605
   Asn Gly Met Pro Val Ile Trp Glu Gly Ser Val Thr Lys Val Glu Ile
       610                 615                 620
   Ser Arg Val Ser Asn Phe Leu Glu Asp Thr Ile Ala Asn Tyr Leu Pro
   625                 630                 635                 640
   Val Trp Leu Gly Arg Met Leu Arg Cys Leu His Gly Glu Asn Ile Ser
                   645                 650                 655
   Gln His Phe Lys Cys Asp Pro Lys Leu Asp Ala Gln Cys Glu Glu Trp
               660                 665                 670
   Arg Ser Val Phe Lys Thr Phe Asn Ala Gln Ser Gly Ser Cys Thr Leu
           675                 680                 685
   Gln Arg Trp Asn Arg Tyr Glu Tyr Glu Leu Leu Val Lys Val Gly Thr
       690                 695                 700
   Lys Arg Ser Gly Arg Leu Leu Gly Arg Ser Thr Thr Thr Asp Val Ile
   705                 710                 715                 720
   Leu Arg Ala His His Asp Phe Gly Asn Phe Thr Arg Leu Leu Ser Glu
                   725                 730                 735
   Gly Asp Val Val Leu Phe Tyr Gly Ile Leu His Asn Ser Arg Leu Leu
   740 745 750
   Ala Asp Asn Val Gln Val Lys Leu Lys Thr Ile Glu Cys Val Glu Cys
   755 760 765
   Arg Ser Arg Asp Leu Gly Thr Ala Ser Ile Glu Arg Val Val Ala Ala
   770 775 780
   Ser Pro Met Asp Ala Arg Leu Gln Asp Leu Met Arg Gly Ile Lys Tyr
   785 790 795 800
   Leu Leu Asn Ala Leu Leu Asn Pro Leu Ile Thr Phe Lys
   805 810
<210> 26
   <211> 351
   <212> PRT
   <213> Artificial Sequence
<220>
   <223> Description of Artificial Sequence; note = synthetic construct
<400> 26
   Met Leu Glu Thr Leu Arg Glu Arg Leu Leu Ser Val Gln Gln Asp Phe
    1               5                  10                  15
   Thr Ser Gly Leu Lys Thr Leu Ser Asp Lys Ser Arg Glu Ala Lys Val
               20                  25                  30
   Lys Ser Lys Pro Arg Thr Val Pro Phe Leu Pro Lys Tyr Ser Ala Gly
           35                  40                  45
   Leu Glu Leu Leu Ser Arg Tyr Glu Asp Thr Trp Ala Ala Leu His Arg
       50                  55                  60
   Arg Ala Lys Asp Cys Ala Ser Ala Gly Glu Leu Val Asp Ser Glu Val
   65                  70                  75                  80
   Val Met Leu Ser Ala His Trp Glu Lys Lys Lys Thr Ser Leu Val Glu
                   85                  90                  95
   Leu Gln Glu Gln Leu Gln Gln Leu Pro Ala Leu Ile Ala Asp Leu Glu
               100                 105                 110
   Ser Met Thr Ala Asn Leu Thr His Leu Glu Ala Ser Phe Glu Glu Val
           115                 120                 125
   Glu Asn Asn Leu Leu His Leu Glu Asp Leu Cys Gly Gln Cys Glu Leu
       130                 135                 140
   Glu Arg Cys Lys His Met Gln Ser Gln Gln Leu Glu Asn Tyr Lys Lys
   145                 150                 155                 160
   Asn Lys Arg Lys Glu Leu Glu Thr Phe Lys Ala Glu Leu Asp Ala Glu
                   165                 170                 175
   His Ala Gln Lys Val Leu Glu Met Glu His Thr Gln Gln Met Lys Leu
               180                 185                 190
   Lys Glu Arg Gln Lys Phe Phe Glu Glu Ala Phe Gln Gln Asp Met Glu
           195                 200                 205
   Gln Tyr Leu Ser Thr Gly Tyr Leu Gln Ile Ala Glu Arg Arg Glu Pro
       210                 215                 220
   Ile Gly Ser Met Ser Ser Met Glu Val Asn Val Asp Met Leu Glu Gln
   225                 230                 235                 240
   Met Asp Leu Met Asp Ile Ser Asp Gln Glu Ala Leu Asp Val Phe Leu
                   245                 250                 255
   Asn Ser Gly Gly Glu Glu Asn Thr Val Leu Ser Pro Ala Leu Gly Pro
               260                 265                 270
   Glu Ser Ser Thr Cys Gln Asn Glu Ile Thr Leu Gln Val Pro Asn Pro
           275                 280                 285
   Ser Glu Leu Arg Ala Lys Pro Pro Ser Ser Ser Ser Thr Cys Thr Asp
       290                 295                 300
   Ser Ala Thr Arg Asp Ile Ser Glu Gly Gly Glu Ser Pro Val Val Gln
   305                 310                 315                 320
   Ser Asp Glu Glu Glu Val Gln Val Asp Thr Ala Leu Ala Thr Ser His
                   325                 330                 335
   Thr Asp Arg Glu Ala Thr Pro Asp Gly Gly Glu Asp Ser Asp Ser
               340                 345                 350
<210> 27
   <211> 288
   <212> PRT
   <213> Artificial Sequence
<220>
   <223> Description of Artificial Sequence; note = synthetic construct
<400> 27
   Met Phe Gly Asn Leu Lys Lys Lys Leu Ser Ser Ala Ile Gln Glu Gly
    1               5                  10                  15
   Leu Val Ile Ser Glu Asn Leu Gln Gln Gln Tyr Arg Gln Arg Val Ser
               20                  25                  30
   Ser Gly Asn Ser Gly Ser Ser Gln Ala Ser Gly Ile Thr Thr Pro Ile
           35                  40                  45
   Ser Pro Leu Gly Leu Asn Glu Ser Leu Ser Ser Ser Arg Ser Ser Ser
       50                  55                  60
   Leu Ser Leu Ser Ala Pro Phe Gln Leu Thr Asp Gly Val Pro Ser His
   65                  70                  75                  80
   Leu Asn Val Ala Ala Gly Cys Ser Leu Leu Ala Lys Tyr Glu Asp Asp
                   85                  90                  95
   Trp Gln Gln Ile His Gly Ala Asn Glu Lys Asn Ala Glu Lys Ala Ala
               100                 105                 110
   Gln Ile Ala Asn Gln Ile Ser Gly Ile Gln Asp Gln Ala Ser His Gln
           115                 120                 125
   His Arg Ile Met Ser Glu Leu Asn Ser Ser Leu Ala Gly Ile Pro Thr
       130                 135                 140
   Leu Ile Ala Gln Leu Gln Asn Ser Ser Gln Val Leu Asn Ser Leu Glu
   145                 150                 155                 160
   Glu Met Gly Lys Gln Leu Glu Ile Glu Leu Glu Lys Leu Glu Asp Leu
                   165                 170                 175
   Arg Glu Glu Cys Glu Leu Gln Glu Phe Ile Leu Glu Gln Gln Phe Gln
               180                 185                 190
   Leu Ser Arg His Lys Gln Lys Lys Leu Asn Glu Leu Glu Gln Tyr Arg
           195                 200                 205
   Gln Gln Ile Ala Gln Lys His Gln Ser Lys Ile Lys Asp Gln Glu Gln
       210                 215                 220
   Thr Leu Leu Lys Leu Gln Arg Glu Arg Gln Ala Val Phe Asp Asp Ala
   225                 230                 235                 240
   Phe Arg Glu Asp Met Glu Glu Tyr Lys Gln Arg Gly Gln Leu Thr Lys
                   245                 250                 255
   Ile Gln Thr Thr Ser Asn Lys Leu Ala Leu Glu Glu Val Val Leu Glu
               260                 265                 270
   Ala Asn Glu Val Glu Thr Lys Asp Ala Leu Glu Gln Phe Leu Asn Gly
           275                 280                 285